(19) Europäisches Patentamt
European Patent Office
Office européen des brevets

(11) EP 0 712 396 B1

(12) **EUROPÄISCHE PATENTSCHRIFT**

(45) Veröffentlichungstag und Bekanntmachung des Hinweises auf die Patenterteilung:
**04.11.1998 Patentblatt 1998/45**

(21) Anmeldenummer: **94924825.6**

(22) Anmeldetag: **29.07.1994**

(51) Int Cl.[6]: **C07D 273/01**, C07D 413/00, C07D 417/00, A01N 43/72, A01N 43/82, A01N 43/88

(86) Internationale Anmeldenummer:
**PCT/EP94/02533**

(87) Internationale Veröffentlichungsnummer:
**WO 95/04728 (16.02.1995 Gazette 1995/08)**

(54) **SUBSTITUIERTE AZADIOXACYCLOALKENE UND IHRE VERWENDUNG ALS FUNGIZIDE**
SUBSTITUTED AZADIOXACYCLOALKENES AND THEIR USE AS FUNGICIDES
AZADIOXACYCLOALCENES SUBSTITUES ET LEUR UTILISATION COMME FONGICIDES

(84) Benannte Vertragsstaaten:
**AT BE CH DE DK ES FR GB GR IE IT LI NL PT SE**

(30) Priorität: **11.08.1993 DE 4326908**
**10.03.1994 DE 4408005**

(43) Veröffentlichungstag der Anmeldung:
**22.05.1996 Patentblatt 1996/21**

(73) Patentinhaber: **BAYER AG**
**51368 Leverkusen (DE)**

(72) Erfinder:
- **KRÜGER, Bernd-Wieland**
  **D-51467 Bergisch Gladbach (DE)**
- **ASSMANN, Lutz**
  **D-23701 Eutin (DE)**
- **GAYER, Herbert**
  **D-40789 Monheim (DE)**
- **GERDES, Peter**
  **D-52080 Aachen (DE)**
- **HEINEMANN, Ulrich**
  **D-42799 Leichlingen (DE)**
- **KUHNT, Dietmar**
  **D-51373 Leverkusen (DE)**
- **PHILIPP, Ulrich**
  **D-51065 Köln (DE)**
- **SEITZ, Thomas**
  **D-40764 Langenfeld (DE)**
- **STETTER, Jörg**
  **D-42115 Wuppertal (DE)**
- **TIEMANN, Ralf**
  **D-51375 Leverkusen (DE)**
- **DEHNE, Heinz-Wilhelm**
  **D-53125 Bonn (DE)**
- **DUTZMANN, Stefan**
  **D-40721 Hilden (DE)**
- **HÄNSSLER, Gerd**
  **D-51381 Leverkusen (DE)**

(56) Entgegenhaltungen:
**EP-A- 0 056 161** **EP-A- 0 242 081**
**EP-A- 0 528 681**

- **CHEMICAL ABSTRACTS, vol. 112, no. 11, 12. März 1990, Columbus, Ohio, US; abstract no. 98566t, Seite 757 ; in der Anmeldung erwähnt**
- **CHEMICAL ABSTRACTS, vol. 113, no. 1, 2. Juli 1990, Columbus, Ohio, US; abstract no. 6381y, Seite 622 ; in der Anmeldung erwähnt**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 712 396 B1

## Beschreibung

Die Erfindung betrifft neue substituierte Azadioxacycloalkene, ein Verfahren zu ihrer Herstellung und ihre Verwendung als Fungizide.

Es ist bekannt, daß bestimmte substituierte 5,6-Dihydro-1,4,2-dioxazine fungizide Eigenschaften aufweisen (vgl. JP-A 01221371 - zitiert in Chem. Abstracts 112: 98566t; JP 02001484 - zitiert in Chem. Abstracts 113: 6381y).

Diese Verbindungen haben jedoch keine besondere Bedeutung erlangt.

Es wurden nun die neuen substituierten Azadioxacycloalkene der allgemeinen Formel (I) gefunden,

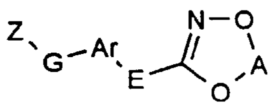

(I)

in welcher

A für gegebenenfalls substituiertes Alkandiyl (Alkylen) steht,

Ar für jeweils gegebenenfalls substituiertes Arylen oder Heteroarylen steht,

E für eine 1-Alken-1,1-diyl-Gruppierung steht, die in 2-Position einen Rest $R^1$ enthält, oder für eine 2-Aza-1-alken-1,1-diyl-Gruppierung steht, die in 2-Position einen Rest $R^2$ enthält, oder für eine 3-Oxa- oder 3-Thia-1-propen-2,3-diyl-Gruppierung steht, die in 1-Position einen Rest $R^1$ enthält, oder für eine 3-Aza-1-propen-2,3-diyl-Gruppierung steht, die in 3-Position einen Rest R und in 1-Position einen Rest $R^1$ enthält, oder für eine 1-Aza-1-propen-2,3-diyl-Gruppierung steht, die in 1-Position einen Rest $R^2$ enthält, oder für eine 3-Oxa- oder 3-Thia-1-aza-propen-2,3-diyl-Gruppierung steht, die in 1-Position einen Rest $R^2$ enthält, oder für eine 1,3-Diaza-1-propen-2,3-diyl-Gruppierung steht, die in 3-Position einen Rest R und in 1-Position einen Rest $R^2$ enthält, oder für eine gegebenenfalls substituierte Imino-Gruppierung ("Azamethylen", N-$R^3$) steht,
wobei

R für Alkyl steht,

$R^1$ für Wasserstoff, Halogen, Cyano oder für jeweils gegebenenfalls substituiertes Alkyl, Alkoxy, Alkylthio, Alkylamino oder Dialkylamino steht,

$R^2$ für Wasserstoff, Amino, Cyano oder für jeweils gegebenenfalls substituiertes Alkyl, Alkoxy, Alkylamino oder Dialkylamino steht, und

$R^3$ für Wasserstoff, Cyano oder für jeweils gegebenenfalls substituiertes Alkyl, Alkenyl, Alkinyl, Cycloalkyl oder Cycloalkylalkyl steht,

G für eine Einfachbindung, für Sauerstoff, für jeweils gegebenenfalls durch Halogen, Hydroxy, Alkyl, Halogenalkyl oder Cycloalkyl substituiertes Alkandiyl, Alkendiyl, Oxaalkendiyl, Alkindiyl oder eine der nachstehenden Gruppierungen steht
-Q-CQ-, -CQ-Q-, -$CH_2$-Q-; -Q-$CH_2$-, -CQ-Q-$CH_2$-, -$CH_2$-Q-CQ-, -Q-CQ-$CH_2$-, -Q-CQ-Q-$CH_2$-, -N=N-, -$S(O)_n$-, -$CH_2$-$S(O)_n$-, -CQ-, -$S(O)_n$-$CH_2$-, -C($R^4$)=N-O-, -C($R^4$)=N-O-$CH_2$-, -N($R^5$)-, -CQ-N($R^5$)-, -N($R^5$)-CQ-, -Q-CQ-N($R^5$)-, -N=C($R^4$)-Q-$CH_2$-, -$CH_2$-O-N=C($R^4$)-, -N($R^5$)-CQ-Q-, -CQ-N($R^5$)-CQ-Q-, -N($R^5$)-CQ-Q-$CH_2$-, -CQ-$CH_2$- oder -N=N-C($R^4$)=N-O-,
wobei

n für die Zahlen 0, 1 oder 2 steht,

Q für Sauerstoff oder Schwefel steht,

$R^4$ für Wasserstoff, Cyano oder jeweils gegebenenfalls substituiertes Alkyl, Alkoxy, Alkylthio, Alkylamino, Dialkylamino oder Cycloalkyl steht, und

$R^5$ für Wasserstoff, Hydroxy, Cyano oder jeweils gegebenenfalls substituiertes Alkyl, Alkoxy oder Cycloalkyl steht, und

Z für jeweils gegebenenfalls substituiertes Alkyl, Alkenyl, Alkinyl, Cycloalkyl, Aryl oder Heterocyclyl steht.

Weiter wurde gefunden, daß man die neuen substituierten Azadioxacycloalkene der allgemeinen Formel (I) erhält, wenn man

(a) Carbonsäurederivate der allgemeinen Formel (II)

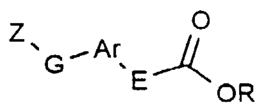

(II)

in welcher

Ar, E, G und Z die oben angegebene Bedeutung haben, und

R für Alkyl steht,
in einer ersten Stufe mit Hydroxylamin oder mit einem Hydrogenhalogenid hiervon, gegebenenfalls in Gegenwart eines Säureakzeptors und gegebenenfalls in Gegenwart eines Verdünnungsmittels umsetzt und in situ, d.h. ohne Zwischenisolierung des Produktes der ersten Stufe, in einer zweiten Stufe mit disubstituierten Alkanen der allgemeinen Formel (III)

X-A-X (III)

in welcher

A die oben angegebene Bedeutung hat und

X für Halogen, Alkylsulfonyloxy oder Arylsulfonyloxy steht,

gegebenenfalls in Gegenwart eines Säureakzeptors und gegebenenfalls in Gegenwart eines Verdünnungsmittels umsetzt, oder wenn man

(b) für den Fall, daß in der Formel (I) G für Sauerstoff oder die Gruppierung $-CH_2-O-$ steht und A, Ar, E und Z die oben angegebene Bedeutung haben,
Hydroxyarylverbindungen der allgemeinen Formel (IV),

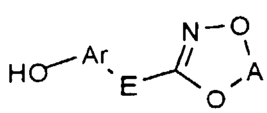

(IV)

in welcher

A, Ar und E die oben angegebene Bedeutung haben,

mit Verbindungen der allgemeinen Formel (V),

$$Z\text{-}(CH_2)_m\text{-}X \quad (V)$$

in welcher

X und Z die oben angegebene Bedeutung haben und

m für die Zahlen 0 oder 1 steht,

gegebenenfalls in Gegenwart eines Säureakzeptors und gegebenenfalls in Gegenwart eines Verdünnungsmittels umsetzt und gegebenenfalls anschließend an der Gruppierung Z nach üblichen Methoden Substitutionsreaktionen durchführt, oder wenn man

(c) für den Fall, daß in der Formel (I) G für die Gruppierung -Q-$CH_2$- steht und A, Ar, E und Z die oben angegebene Bedeutung haben,
Halogenmethylverbindungen der allgemeinen Formel (VI)

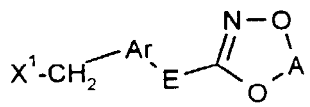

(VI)

in welcher

A, Ar und E die oben angegebene Bedeutung haben und

$X^1$ für Halogen steht,

mit Verbindungen der allgemeinen Formel (VII)

$$Z\text{-}Q\text{-}H \quad (VII)$$

in welcher

Q und Z die oben angegebene Bedeutung haben,

gegebenenfalls in Gegenwart eines Säureakzeptors und gegebenenfalls in Gegenwart eines Verdünnungsmittels umsetzt, oder wenn man

(d) Hydroxyalkoxyamide der allgemeinen Formel (VIII)

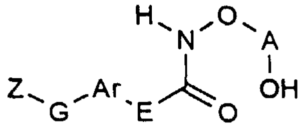

(VIII)

in welcher

A, Ar, E, G und Z die oben angegebenen Bedeutung haben,

mit einem Wasser-entziehenden Mittel, gegebenenfalls in Gegenwart eines Verdünnungsmittels cyclisiert.

Schließlich wurde gefunden, daß die neuen substituierten Azadioxacycloalkene der allgemeinen Formel (I) sehr

starke fungizide Wirksamkeit zeigen.

Die erfindungsgemäßen Verbindungen können gegebenenfalls als Mischungen verschiedener möglicher isomerer Formen, insbesondere von E- und Z-Isomeren, vorliegen. Es werden sowohl die E- als auch die Z-Isomeren wie auch beliebige Mischungen dieser Isomeren beansprucht.

Gegenstand der Erfindung sind vorzugsweise Verbindungen der Formel (I), in welcher

A für gegebenenfalls durch Halogen oder durch Alkyl oder Halogenalkyl mit jeweils 1 bis 4 Kohlenstoffatomen substituiertes Alkandiyl mit 1 bis 3 Kohlenstoffatomen steht,

Ar für jeweils gegebenenfalls substituiertes Phenylen oder Naphthylen oder für Heteroarylen mit 5 oder 6 Ringgliedern, von denen mindestens eines für Sauerstoff, Schwefel oder Stickstoff steht und gegebenenfalls ein oder zwei weitere für Stickstoff stehen, steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl, Alkenyloxy oder Alkinyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,

E für eine der nachstehenden Gruppierungen steht

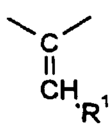 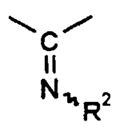 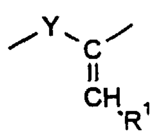 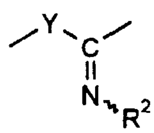 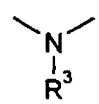

worin

Y für Sauerstoff, Schwefel, Methylen ($CH_2$) oder Alkylimino (N-R) steht,

R für Alkyl mit 1 bis 6 Kohlenstoffatomen steht,

$R^1$ für Wasserstoff, Halogen, Cyano oder für jeweils gegebenenfalls durch Halogen, Cyano oder $C_1$-$C_4$-Alkoxy substituiertes Alkyl, Alkoxy, Alkylthio, Alkylamino oder Dialkylamino mit jeweils 1 bis 6 Kohlenstoffatomen in den Alkylresten steht,

$R^2$ für Wasserstoff, Amino, Cyano oder für jeweils gegebenenfalls durch Halogen, Cyano oder $C_1$-$C_4$-Alkoxy substituiertes Alkyl, Alkoxy, Alkylamino oder Dialkylamino mit jeweils 1 bis 6 Kohlenstoffatomen in den Alkylresten steht, und

$R^3$ für Wasserstoff, Cyano oder für jeweils gegebenenfalls durch Halogen, Cyano oder $C_1$-$C_4$-Alkoxy substituiertes Alkyl, Alkenyl oder Alkinyl mit jeweils bis zu 6 Kohlenstoffatomen oder für jeweils gegebenenfalls durch Halogen, Cyano, Carboxy, $C_1$-$C_4$-Alkyl oder $C_1$-$C_4$-Alkoxy-carbonyl substituiertes Cycloalkyl oder Cycloalkylalkyl mit 3 bis 6 Kohlenstoffatomen in den Cycloalkylteilen und gegebenenfalls 1 bis 4 Kohlenstoffatomen im Alkylteil steht,

G für eine Einfachbindung, für Sauerstoff, für jeweils gegebenenfalls durch Halogen, Hydroxy, $C_1$-$C_4$-Alkyl, $C_1$-$C_4$-

Halogenalkyl oder $C_3$-$C_6$-Cycloalkyl substituiertes Alkandiyl, Alkendiyl, Oxaalkendiyl, Alkindiyl mit jeweils bis zu 4 Kohlenstoffatomen oder eine der nachstehenden Gruppierungen steht
-Q-CQ-, -CQ-Q-, $-CH_2$-Q-; -Q-$CH_2$-, -CQ-Q-$CH_2$-, -$CH_2$-Q-CQ-, -Q-CQ-$CH_2$-, -Q-CQ-Q-$CH_2$-, -N=N-, -S(O)$_n$-, -$CH_2$-S(O)$_n$-, -CQ-, -S(O)$_n$-$CH_2$-, -C($R^4$)=N-O-, -C($R^4$)=N-O-$CH_2$-, -N($R^5$)-, -CQ-N($R^5$)-, -N($R^5$)-CQ-, -Q-CQ-N ($R^5$)-, -N=C($R^4$)-Q-CH2-, -$CH_2$-O-N=C($R^4$)-, -N($R^5$)-CQ-Q-, -CQ-N($R^5$)-CQ-Q-, -N($R^5$)-CQ-Q-$CH_2$-, -CQ-$CH_2$- oder -N=N-C($R^4$)=N-=-, wobei

n für die Zahlen 0, 1 oder 2 steht,

Q für Sauerstoff oder Schwefel steht,

$R^4$ für Wasserstoff, Cyano, für gegebenenfalls durch Halogen, Cyano oder $C_1$-$C_4$-Alkoxy substituiertes Alkyl, Alkoxy, Alkylthio, Alkylamino oder Dialkylamino mit jeweils 1 bis 6 Kohlenstoffatomen in den Alkylgruppen oder für jeweils gegebenenfalls durch Halogen, Cyano, Carboxy, $C_1$-$C_4$-Alkyl oder $C_1$-$C_4$-Alkoxy-carbonyl substituiertes Cycloalkyl mit 3 bis 6 Kohlenstoffatomen steht, und

$R^5$ für Wasserstoff, Hydroxy, Cyano oder für gegebenenfalls durch Halogen, Cyano oder $C_1$-$C_4$-Alkoxy substituiertes Alkyl mit 1 bis 6 Kohlenstoffatomen oder für gegebenenfalls durch Halogen, Cyano, Carboxy, $C_1$-$C_4$-Alkyl oder $C_1$-$C_4$-Alkoxy-carbonyl substituiertes Cycloalkyl mit 3 bis 6 Kohlenstoffatomen steht, und

Z für gegebenenfalls durch Halogen, Cyano, Hydroxy, Amino, $C_1$-$C_4$-Alkoxy, $C_1$-$C_4$-Alkylthio, $C_1$-$C_4$-Alkylsulfinyl oder $C_1$-$C_4$-Alkylsulfonyl (welche jeweils gegebenenfalls durch Halogen substituiert sind) substituiertes Alkyl mit 1 bis 8 Kohlenstoffatomen, für jeweils gegebenenfalls durch Halogen substituiertes Alkenyl oder Alkinyl mit jeweils bis zu 8 Kohlenstoffatomen, für jeweils gegebenenfalls durch Halogen, Cyano, Carboxy, Phenyl (welches gegebenenfalls durch Halogen, Cyano, $C_1$-$C_4$-Alkyl, $C_1$-$C_4$-Halogenalkyl, $C_1$-$C_4$-Alkoxy oder $C_1$-$C_4$-Halogenalkoxy substituiert ist), $C_1$-$C_4$-Alkyl oder $C_1$-$C_4$-Alkoxy-carbonyl substiuiertes Cycloalkyl mit 3 bis 6 Kohlenstoffatomen, für jeweils gegebenenfalls substituiertes Phenyl, Naphthyl oder (gegebenenfalls benzannelliertes) Heterocyclyl mit 5 oder 6 Ringgliedern, von denen mindestens eines für Sauerstoff, Schwefel oder Stickstoff und gegebenenfalls ein oder zwei weitere für Stickstoff stehen, steht, wobei die möglichen Substituenten vorzugsweise aus der nachstehenden Aufzählung ausgewählt sind: Sauerstoff (als Ersatz für zwei geminale Wasserstoffatome), Halogen, Cyano, Nitro, Amino, Hydroxy, Formyl, Carboxy, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl oder Alkenyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen, Cycloalkyl mit 3 bis 6 Kohlenstoffatomen, Heterocyclyl oder Heterocyclyl-methyl mit jeweils 3 bis 7 Ringgliedern, von denen jeweils 1 bis 3 gleiche oder verschiedene Heteroatome sind - insbesondere Stickstoff, Sauerstoff und/oder Schwefel -, sowie jeweils gegebenenfalls im Phenylteil einfach oder mehrfach, gleich oder verschieden durch Halogen, Cyano, Nitro, Carboxy, Carbamoyl und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen und/oder geradkettiges oder verzweigtes Alkoxy mit 1 bis 4 Kohlenstoffatomen und/oder geradkettiges oder verzweigtes Halogenalkoxy mit 1 bis 4 Kohlenstoffatomen und 1 bis 9 gleichen oder verschiedenen Halogenatomen und/oder Alkylcarbonyl oder Alkoxycarbonyl mit jeweils bis zu 5 Kohlenstoffatomen substituiertes Phenyl, Phenoxy, Benzyl, Benzyloxy, Phenylethyl oder Phenylethyloxy.

In den Definitionen sind die gesättigten oder ungesättigten Kohlenwasserstoffketten, wie Alkyl, Alkandiyl, Alkenyl oder Alkinyl, auch in Verknüpfung mit Heteroatomen, wie in Alkoxy, Alkylthio oder Alkylamino, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Die Erfindung betrifft insbesondere Verbindungen der Formel (I), in welcher

A für jeweils gegebenenfalls durch Fluor, Chlor, Methyl, Ethyl oder Trifluormethyl substituiertes Methylen oder Dimethylen (Ethan-1,2-diyl) steht

Ar für jeweils gegebenenfalls substituiertes ortho-, meta- oder para-Phenylen, für Furandiyl, Thiophendiyl, Pyrroldiyl, Pyrazoldiyl, Triazoldiyl, Oxazoldiyl, Isoxazoldiyl, Thiazoldiyl, Isothiazoldiyl, Oxadiazoldiyl, Thiadiazoldiyl, Pyridindiyl, Pyrimidindiyl, Pyridazindiyl, Pyrazindiyl, 1,3,4-Triazindiyl oder 1,2,3-Triazindiyl steht, wobei die möglichen Substituenten insbesondere aus der nachstehenden Aufzählung ausgewählt sind:
Fluor, Chlor, Cyano, Methyl, Ethyl, Trifluormethyl, Methoxy, Ethoxy, Methylthio, Methylsulfinyl oder Methylsulfonyl,

E für eine der nachstehenden Gruppierungen steht

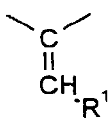 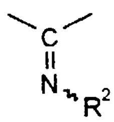 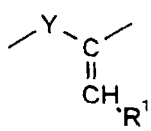 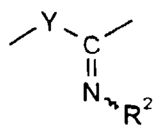 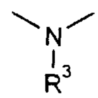

worin

Y für Sauerstoff, Schwefel, Methylen ($CH_2$) oder Alkylimino (N-R) steht,

R für Methyl, Ethyl, n- oder i-Propyl, n-, i- oder s-Butyl steht,

$R^1$ für Wasserstoff, Fluor, Chlor, Brom, Cyano oder für jeweils gegebenenfalls durch Fluor, Chlor, Cyano, Methoxy oder Ethoxy substituiertes Methyl, Ethyl, Propyl, Methoxy, Ethoxy, Methylthio, Ethylthio, Methylamino, Ethylamino oder Dimethylamino steht,

$R^2$ für Wasserstoff, Amino, Cyano oder für jeweils gegebenenfalls durch Fluor, Chlor, Cyano, Methoxy oder Ethoxy substituiertes Methyl, Ethyl, Methoxy, Ethoxy, Methylamino, Ethylamino oder Dimethylamino steht, und

$R^3$ für Wasserstoff, Cyano oder für jeweils gegebenenfalls durch Fluor, Cyano, Methoxy oder Ethoxy substituiertes Methyl, Ethyl n- oder i-Propyl, n-, i- oder s-Butyl, für Allyl oder Propargyl oder für jeweils gegebenenfalls durch Fluor, Chlor, Cyano, Carboxy, Methyl, Ethyl, n-oder i-Propyl, Methoxy-carbonyl oder Ethoxy-carbonyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht,

G für eine Einfachbindung, für Sauerstoff, für jeweils gegebenenfalls durch Fluor, Chlor, Hydroxy, Methyl, Ethyl, n- oder i-Propyl, Trifluormethyl, Cyclopropyl, Cyclobutyl, Cyclopentyl oder Cyclohexyl substituiertes Methylen, Dimethylen (Ethan-1,2-diyl), Ethen-1,2-diyl, Ethin-1,2-diyl oder eine der nachstehenden Gruppierungen
-Q-CQ-, -CQ-Q-, $-CH_2-Q-$, $-Q-CH_2-$, $-CQ-Q-CH_2-$, $-CH_2-Q-CQ-$, $-Q-CQ-CH_2-$, $-Q-CQ-Q-CH_2-$, -N=N-, $-S(O)_n-$, $-CH_2-S(O)_n-$, -CQ-, $-S(O)_n-CH_2-$, $-C(R^4)=N-O-$, $-C(R^4)=N-O-CH_2-$, $-N(R^5)-$, $-CQ-N(R^5)-$, $-N(R^5)-CQ-$, $-Q-CQ-N(R^5)-$, $-N=C(R^4)-Q-CH2-$, $-CH_2-O-N=C(R^4)-$, $-N(R^5)-CQ-Q-$, $-CQ-N(R^5)-CQ-Q-$ oder $-N(R^5)-CQ-Q-CH_2-$ steht, wobei

n für die Zahlen 0, 1 oder 2 steht,

Q für Sauerstoff oder Schwefel steht,

$R^4$ für Wasserstoff, Cyano, für gegebenenfalls durch Fluor, Chlor, Cyano, Methoxy oder Ethoxy substituiertes Methyl, Ethyl, n- oder i-Propyl, n-, i- oder s-Butyl, Methoxy, Ethoxy, Propoxy, Butoxy, Methylthio, Ethylthio, Propylthio, Butylthio, Methylamino, Ethylamino, Propylamino, Dimethylamino oder Diethylamino oder für jeweils gegebenenfalls durch Fluor, Chlor, Cyano, Carboxy, Methyl, Ethyl, n- oder i-Propyl, Methoxycarbonyl oder Ethoxy-carbonyl substituiertes Cyclopropyl, Cyclobutyl, Cyclopentyl oder Cyclohexyl steht, und

$R^5$ für Wasserstoff, Hydroxy, Cyano oder für jeweils gegebenenfalls durch Fluor, Chlor, Cyano, Methoxy oder Ethoxy substituiertes Methyl, Ethyl, n- oder i-Propyl, n-, i-, s- oder t-Butyl oder für gegebenenfalls durch Fluor, Chlor, Cyano, Carboxy, Methyl, Ethyl, n- oder i-Propyl, Methoxy-carbonyl oder Ethoxy-carbonyl substituiertes Cyclopropyl, Cyclobutyl, Cyclopentyl oder Cyclohexyl steht, und

Z für gegebenenfalls durch Fluor, Chlor, Brom, Cyano, Hydroxy, Amino, Methoxy, Ethoxy, Methylthio, Ethylthio, Methylsulfinyl, Ethyl sulfinyl, Methylsulfonyl, Ethylsulfonyl (welche jeweils gegebenenfalls durch Fluor und/oder Chlor substituiert sind) substituiertes Methyl, Ethyl, n- oder i-Propyl, n-, i-, s- oder t-Butyl, für jeweils gegebenenfalls durch Fluor, Chlor oder Brom substituiertes Allyl, Crotonyl, 1-Methyl-allyl, Propargyl oder 1-Methyl-propargyl , für jeweils gegebenenfalls durch Fluor, Chlor, Brom, Cyano, Carboxy, Phenyl (welches gegebenenfalls durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiert ist), Methyl, Ethyl, n- oder i-Propyl, Methoxy-carbonyl oder Ethoxy-carbonyl substiuiertes Cyclopropyl, Cyclobutyl, Cyclopentyl oder Cyclohexyl , für jeweils gegebenenfalls substituiertes Phenyl, Naphthyl, Furyl, Tetrahydrofuryl, Benzofuryl, Tetrahydropyranyl, Thienyl, Benzothienyl, Pyrrolyl, Dihydropyrrolyl, Tetrahydropyrrolyl, Benzopyrrolyl, Benzodihydropyrrolyl, Oxazolyl, Benzoxazolyl, Isoxazolyl, Thiazolyl, Benzthiazolyl, Isothiazolyl, Imidazolyl, Benzimidazolyl, Oxadiazolyl, Thiadiazolyl, Pyridinyl, Pyrimidinyl, Pyridazinyl, Pyrazinyl, 1,2,3-Triazinyl, 1,2,4-Triazinyl oder 1,3,5-Triazinyl steht, wobei die möglichen Substituenten vorzugsweise aus der nachstehenden Aufzählung ausgewählt sind:
Sauerstoff (als Ersatz für zwei geminale Wasserstoffatome), Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl; jeweils gegebenenfalls einfach oder mehrfach, gleich oder verschieden durch Fluor, Chlor, Methyl, Ethyl, n- oder i-Propyl substituiertes Trimethylen (Propan-1,3-diyl), Methylendioxy oder Ethylendioxy, Cyclopropyl, Cyclobutyl, Cyclopentyl oder Cyclohexyl, sowie jeweils gegebenenfalls im Phenylteil einfach oder mehrfach, gleich oder verschieden durch Fluor, Chlor, Brom, Cyano, Nitro, Carboxy, Carbamoyl, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Acetyl, Methoxycarbonyl oder Ethoxycarbonyl substituiertes Phenyl, Phenoxy, Benzyl oder Benzyloxy.

Eine besonders bevorzugte Gruppe erfindungsgemäßer Verbindungen sind diejenigen Verbindungen der Formel (I), in welcher

A für Dimethylen (Ethan-1,2-diyl) steht,

Ar für ortho-Phenylen, Pyridin-2,3-diyl oder Thiophen-2,3-diyl steht,

E für eine der nachstehenden Gruppierungen steht

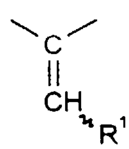

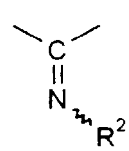

worin

$R^1$ und $R^2$ jeweils für Methoxy stehen,

G für Sauerstoff, Methylen oder eine der nachstehenden Gruppierungen $-CH_2-O-$, $-O-CH_2-$, $-S(O)_n-$, $-CH_2-S(O)_n-$, $-S(O)_n-CH_2-$, $-C(R^4)=N-O-$, $-O-N=C(R^4)-$, $-C(R^4)=N-O-CH_2-$, $-N(R^5)-$ oder $-CH_2-O-N=C(R^4)-$ steht, wobei

n für die Zahlen 0, 1 oder 2 steht,

$R^4$ für Wasserstoff, Methyl oder Ethyl steht und

$R^5$ für Wasserstoff, Methyl oder Ethyl steht, und

Z für jeweils gegebenenfalls substituiertes Phenyl, Pyridinyl, Pyrimidinyl, Pyridazinyl, Pyrazinyl, 1,2,3-Triazinyl, 1,2,4-Triazinyl oder 1,3,5-Triazinyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluormethylthio, Trifluormethylthio, Trifluormethyl sulfinyl oder Trifluormethylsulfonyl, Methoxy-carbonyl, Ethoxycarbonyl, Methoximinomethyl, Ethoximinomethyl, Methoximinoethyl, Ethoximinoethyl, jeweils gegebenenfalls einfach oder mehrfach, gleich oder verschieden durch Fluor, Chlor, Methyl oder Ethyl substituiertes Methylendioxy oder Ethylendioxy, sowie jeweils gegebenenfalls im Phenylteil einfach oder mehrfach, gleich oder verschieden durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, Phenoxy, Benzyl oder Benzyloxy.

Die oben aufgeführten allgemeinen oder in Vorzugsbereichen angegebenen Restedefinitionen gelten sowohl für die Endprodukte der Formel (I) als auch entsprechend für die jeweils zu Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Verwendet man beispielsweise α-Methoximino-α-(2-phenoxy-phenyl)-essigsäure-methylester, Hydroxylamin-Hydrochlorid und 1,2-Dibrom-ethan als Ausgangsstoffe, so kann der Reaktionsablauf beim erfindungsgemäßen Herstellungsverfahren (a) durch das folgende Formelschema skizziert werden:

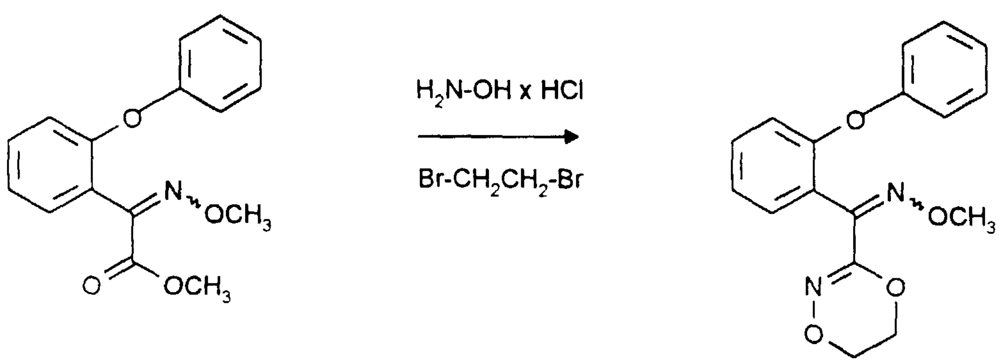


Verwendetmanbeispielsweise 3-[α-Methoximino-α-(2-hydroxy-phenyl)-methyl]-5,6-dihydro-1,4,2-dioxazin und Benzylchlorid als Ausgangsstoffe, so kann der Reaktionsablauf beim erfindungsgemäßen Herstellungsverfahren (b) durch das folgende Formelschema skizziert werden:

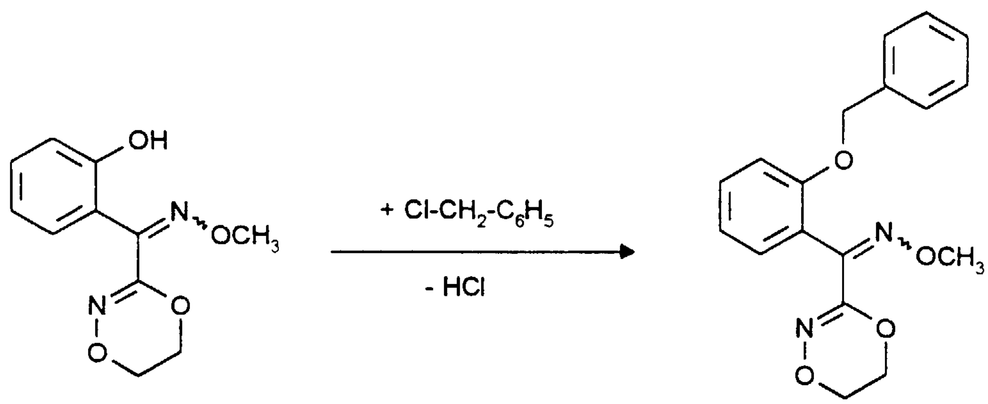

Verwendetmanbeispielsweise 3-[α-Methoximino-α-(2-chlormethyl-phenyl)-methyl]-5,6-dihydro-1,4,2-dioxazin und 2-Methyl-phenol als Ausgangsstoffe, so kann der Reaktionsablauf beim erfindungsgemäßen Verfahren (c) durch das folgende Formelschema skizziert werden:

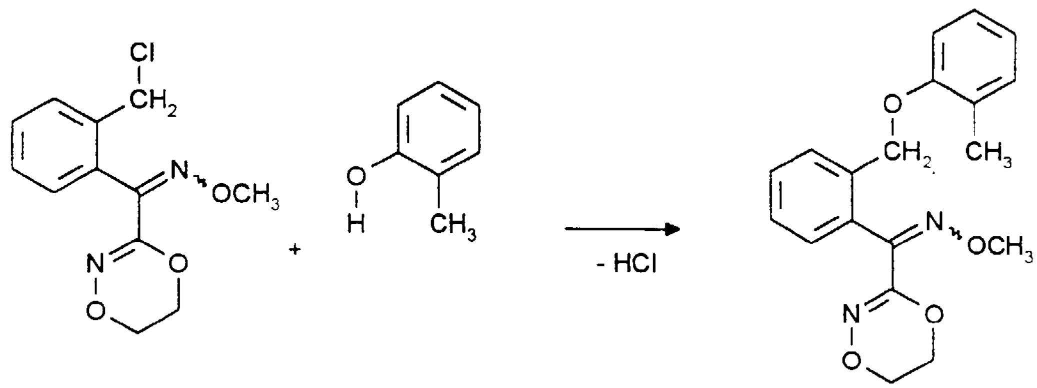


Verwendet man beispielsweise N-(2-Hydroxy-ethoxy)-α-methoximino-α-(2-phenoxyphenyl)-acetamid als Ausgangsverbindung so kann der Reaktionsablauf beim erfindungsgemäßen Verfahren durch das folgende Formelschema skizziert werden:

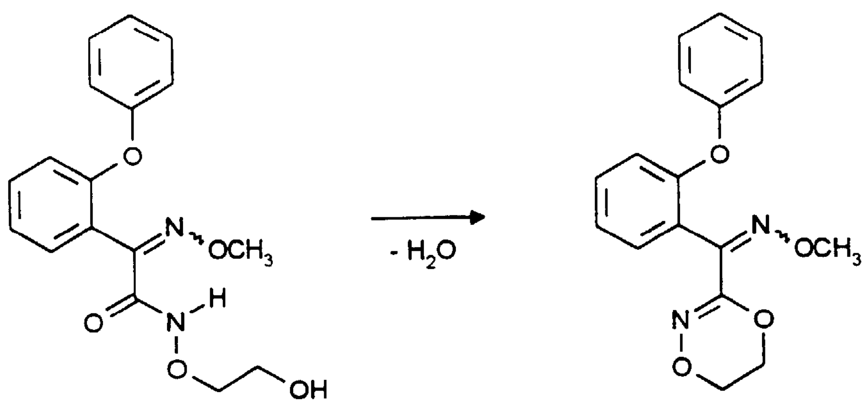


Die zur Durchführung des erfindungsgemäßen Verfahrens (a) als Ausgangsstoffe benötigten Carbonsäurederivate sind durch die Formel (II) allgemein definiert. In dieser Formel (II) haben Ar, E, G und Z vorzugsweise bzw. insbesondere diejenige Bedeutungen, die bereits im Zusammenhang mit der Beschreibung der erfindungsgemäßen Verbindungen der Formel (I) als bevorzugt bzw. als insbesondere bevorzugt für Ar, E, G und Z angegeben wurden; R steht vorzugsweise für Alkyl mit 1 bis 4 Kohlenstoffatomen, insbesondere für Methyl oder Ethyl.

Die Ausgangsstoffe der Formel (II) sind bekannt und/oder können nach an sich bekannten Verfahren hergestellt werden (vgl. EP-A 178826, EP-A 242081, EP-A 382375, EP-A 493711).

Die beim erfindungsgemäßen Verfahren (a) weiter als Ausgangsstoffe zu verwendenden disubstituierten Alkane sind durch die Formel (III) allgemein definiert. In der Formel (III) hat A vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für A angegeben wurde; X steht vorzugsweise für Chlor, Brom, Methylsulfonyloxy, Phenylsulfonyloxy oder Tolylsulfonyloxy.

Die Ausgangsstoffe der Formel (III) sind bekannte organische Synthesechemikalien. Die beim erfindungsgemäßen Verfahren (b) zur Herstellung der Verbindungen der allgemeinen Formel (I) als Ausgangsstoffe zu verwendenden Hydroxyarylverbindungen sind durch die Formel (IV) allgemein definiert. In der Formel (IV) haben A, Ar und E vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für A, Ar und E angegeben wurden.

Die Ausgangsstoffe der Formel (IV) sind noch nicht aus der Literatur bekannt; sie sind als neue Stoffe Gegenstand der vorliegenden Anmeldung.

Man erhält die neuen Hydroxyarylverbindungen der Formel (IV), wenn man Tetrahydropyranyloxyverbindungen der allgemeinen Formel (IX)

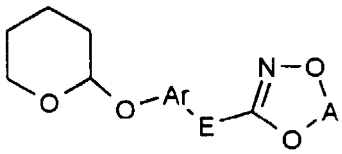

(IX)

in welcher

A, Ar und E die oben angegebene Bedeutung haben,

mit einer Säure, wie z.B. Salzsäure, Schwefelsäure, Methansulfonsäure, Benzolsulfonsäure, p-Toluolsulfon-säure oder einem sauren Ionenaustauscher, gegebenenfalls in Gegenwart eines Verdünnungsmittels, wie z.B. Wasser, Methanol, Ethanol oder Essigsäureethylester bei Temperaturen zwischen 0°C und 100°C umsetzt (vgl. die Herstellungsbeispiele).

Die Tetrahydropyranyloxyverbindungen der Formel (IX) sind noch nicht aus der Literatur bekannt; sie sind als neue Stoffe Gegenstand der vorliegenden Anmeldung.

Man erhält die neuen Tetrahydropyranyloxyverbindungen der Formel (IX), wenn man Ester der allgemeinen Formel (X)

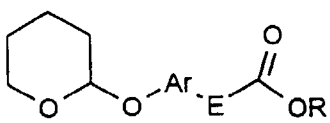

(X)

in welcher

Ar, E und R die oben angegebene Bedeutung haben,

mit Hydroxylamin - oder gegebenenfalls mit dessen Hydrochlorid - gegebenenfalls in Gegenwart eines Säureakzeptors, wie z.B. Kaliumhydroxid, und gegebenenfalls in Gegenwart von Verdünnungsmitteln, wie z.B. Methanol und Wasser, umsetzt und das hierbei gebildete Intermediat in situ weiter mit Dihalogenalkanen der allgemeinen Formel (III) - oben - gegebenenfalls in Gegenwart eines Säureakzeptors, wie z.B. Kaliumcarbonat, bei Temperaturen zwischen 0°C und 100°C umsetzt (vgl. die Beschreibung des erfindungsgemäßen Verfahrens (a) und die Herstellungsbeispiele).

Die Ester der Formel (X) sind noch nicht aus der Literatur bekannt.

Man erhält die neuen Ester der Formel (X), wenn man Tetrahydropyranyloxy-phenylessigsäure-ester der allgemeinen Formel (XI)

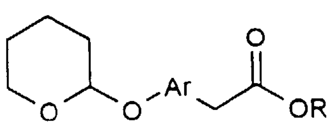

(XI)

in welcher

Ar und R die oben angegebene Bedeutung haben,

nach üblichen Methoden derivatisiert (vgl. die Herstellungsbeispiele).

Die Tetrahydropyranyloxy-phenylessigsäure-ester der Formel (XI) sind noch nicht aus der Literatur bekannt.

Man erhält die neuen Tetrahydropyranyloxy-phenylessigsäure-ester der Formel (XI), wenn man Hydroxyphenylessigsäureester der allgemeinen Formel (XII)

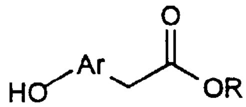

(XII)

in welcher

Ar und R die oben angegebene Bedeutung haben,

mit Dihydropyran, gegebenenfalls in Gegenwart eines Katalysators, wie z.B. p-Toluolsulfonsäure, und gegebenenfalls in Gegenwart eines Verdünnungsmittels, wie z.B. Tetrahydrofuran, bei Temperaturen zwischen 0°C und 100°C umsetzt (vgl. die Herstellungsbeispiele).

Die Ausgangsstoffe der Formel (XII) sind bekannte Synthesechemikalien.

Die beim erfindungsgemäßen Verfahren (b) zur Herstellung der Verbindungen der allgemeinen Formel (I) weiter als Ausgangsstoffe zu verwendenden Verbindungen sind durch die Formel (V) allgemein definiert. In der Formel (V) hat Z vorzugsweise bzw. insbesondere diejenige Bedeutunge, die bereits oben im Zusammenhang mit der Beschreibung der Verbindungen der Formel (I) als bevorzugt bzw. als insbesondere bevorzugt für Z angegeben wurde; X steht vorzugsweise für Chlor, Brom, Methylsulfonyloxy, Phenylsulfonyloxy oder Tolylsulfonyloxy.

Die Ausgangsstoffe der Formel (V) sind bekannte Synthesechemikalien.

Die beim erfindungsgemäßen Verfahren (c) zur Herstellung der Verbindungen der allgemeinen Formel (I) als Ausgangsstoffe zu verwendenden Halogenmethylverbindungen sind durch die Formel (VI) allgemein definiert. In der Formel (VI) haben A, Ar und E vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für A, Ar und E angegeben wurden; $X^1$ steht vorzugsweise für Fluor, Chlor, Brom oder Iod, insbesondere für Chlor oder Brom.

Die Ausgangsstoffe der Formel (VI) sind noch nicht aus der Literatur bekannt; sie sind als neue Stoffe Gegenstand der vorliegenden Anmeldung

Man erhält die neuen Halogenmethylverbindungen der Formel (VI), wenn man Methylverbindungen der allgemeinen Formel (XIII)

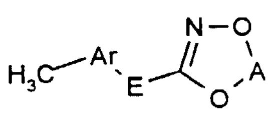

(XIII)

in welcher

A, Ar und E die oben angegebene Bedeutung haben,

mit einem Halogenierungsmittel, wie z.B. N-Brom- oder N-Chlor-succinimid, gegebenenfalls in Gegenwart eines Katalysators, wie z.B. Azoisobutyronitril, und gegebenenfalls in Gegenwart eines Verdünnungsmittels, wie z.B. Tetrachlormethan, bei Temperaturen zwischen 0°C und 150°C umsetzt (vgl. die Herstellungsbeispiele).

Die als Vorprodukte benötigten Methylverbindungen der Formel (XIII) sind noch nicht aus der Literatur bekannt; sie sind als neue Stoffe Gegenstand der vorliegenden Anmeldung.

Man erhält die neuen Methylverbindungen der Formel (XIII), wenn man Ester der allgemeinen Formel (XIV)

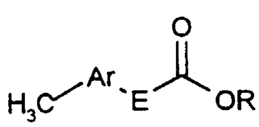

(XIV)

in welcher

Ar, E und R die oben angegebene Bedeutung haben,

mit Hydroxylamin oder Hydroxylamin-Hydrochlorid, gegebenenfalls in Gegenwart eines Säureakzeptors, wie z.B. Kaliumhydroxid, und gegebenenfalls in Gegenwart eines Verdünnungsmittels, wie z.B. Methanol, und anschließend mit einem disubstituierten Alkan der allgemeinen Formel (III) - oben - gegebenenfalls in Gegenwart eines Säureakzeptors, wie z.B. Kaliumcarbonat, analog zum erfindungsgemäßen Verfahren (a) bei Temperaturen zwischen 0°C und 150°C umsetzt (vgl. die Herstellungsbeispiele).

Die Vorprodukte der Formel (XIV) sind bekannt und/oder können nach an sich bekannten Verfahren hergestellt werden (vgl. EP-A 386561, EP-A 498188, Herstellungsbeispiele).

Die beim erfindungsgemäßen Verfahren (c) zur Herstellung der Verbindungen der allgemeinen Formel (I) weiter als Ausgangsstoffe zu verwendenden Verbindungen sind durch die Formel (VII) allgemein definiert. In der Formel (VII) haben Q und Z vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für Q und Z angegeben wurden.

Die Ausgangsstoffe der Formel (VII) sind bekannte organische Synthesechemikalien.

Die beim erfindungsgemäßen Verfahren (d) zur Herstellung der Verbindungen der allgemeinen Formel (I) als Ausgangsstoffe zu verwendenden Hydroxyalkoxyamide sind durch die Formel (VIII) allgemein definiert. In der Formel (VIII) haben A, Ar, E, G und Z vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für A, Ar, E, G und Z angegeben wurden.

Die Ausgangsstoffe der Formel (VIII) sind noch nicht aus der Literatur bekannt; sie sind als neue Stoffe Gegenstand der vorliegenden Anmeldung.

Man erhält die neuen Hydroxyalkoxyamide der Formel (VIII), wenn man Carbonsäurederivate der allgemeinen Formel (XV)

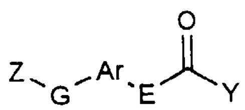

(XV)

in welcher

Ar, E, G und Z die oben angegebene Bedeutung haben und

Y für Halogen, Hydroxy oder Alkoxy steht,

mit Hydroxylaminen der allgemeinen Formel (XVI)

$$H_2N\text{-}O\text{-}A\text{-}OH \qquad (XVI)$$

in welcher

A die oben angegebene Bedeutung hat,

gegebenenfalls in Gegenwart eines Säureakzeptors, wie z.B. Triethylamin, Pyridin oder 4-Dimethylamino-pyridin, und gegebenenfalls in Gegenwart eines Verdünnungsmittels, wie z.B. Methylenchlorid, Toluol oder Tetrahydrofuran, bei Temperaturen zwischen 0°C und 150°C umsetzt (vgl. die Herstellungsbeispiele).

Die als Vorprodukte benötigten Carbonsäurederivate der Formel (XV) sind bekannt und/oder können nach an sich bekannten Verfahren hergestellt werden (vgl. EP-A 178826, EP-A 242081, EP-A 382375, EP-A 493711).

Die weiter als Vorprodukte benötigten Hydroxylamine der Formel (XVI) sind ebenfalls bekannt und/oder können nach an sich bekannten Verfahren hergestellt werden (vgl. J. Chem. Soc. Perkin Trans. I 1987, 2829-2832).

Die erfindungsgemäßen Verfahren (a), (b) und (c) werden vorzugsweise in Gegenwart eines geeigneten Säureakzeptors durchgeführt. Als solche kommen alle üblichen anorganischen oder organischen Basen infrage. Hierzu gehören beispielsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethylbenzylamin, Pyridin, N-Methylpiperidin, N,N-Dimethylaminopyridin, Diazabicyclooctan (DABCO), Diazabicyclononen (DBN) oder Diazabicycloundecen (DBU).

Als Verdünnungsmittel zur Durchführung der erfindungsgemäßen Verfahren (a), (b) und (c) kommen Wasser und organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan, Ether, wie Diethylether, Diisopropylether, Dioxan, Tetrahydrofuran oder Ethylenglykol-dimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid,N,N-Dimethylacetamid, N-Methylformanilid, N-Methyl-pyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykol-monomethylether, Ethylenglykol-monoethyl ether, Diethylenglykolmonomethylether, Diethylenglykolmonoethylether, deren Gemische mit Wasser oder reines Wasser.

Das erfindungsgemäße Verfahren (d) wird in vorzugsweise in Gegenwart eines Dehydratisierungsmittels durchgeführt. Als Dehydratisierungsmittel sind hierbei die üblichen wasser-entziehenden Chemikalien, insbesondere Säureanhydride, wie z.B. Phosphor(V)-oxid (Phosphorpentoxid) geeignet.

Als Verdünnungsmittel zur Durchführung des erfindungsgemäßen Verfahrens (d) kommen die üblichen inerten organischen Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methyl-pyrrolidon oder Hexamethylphosphorsäuretriamid; Ester wie Essigsäuremethylester oder Essigsäureethylester, Sulfoxide, wie Dimethylsulfoxid.

Die Reaktionstemperaturen können bei der Durchführung der erfindungsgemäßen Verfahren (a), (b), (c) und (d) in einem größeren Bereich variiert werden. Im allgemeinen arbeitet man bei Temperaturen zwischen -20°C und +200°C, vorzugsweise bei Temperaturen zwischen 0°C und 150°C.

Zur Durchführung des erfindungsgemäßen Verfahrens (a) setzt man je Mol an Carbonsäurederivat der Formel (II) im allgemeinen 1 bis 5 Mol, vorzugsweise 1,0 bis 2,5 Mol an Hydroxylamin oder Hydroxylamin-Hydrogenhalogenid und im allgemeinen 1 bis 10 Mol, vorzugsweise 1,0 bis 5,0 Mol eines disubstituierten Alkans der Formel (III) ein.

Zur Durchführung des erfindungsgemäßen Verfahrens (b) setzt man pro Mol an Hydroxyarylverbindung der Formel (IV) im allgemeinen 0,5 bis 2,0 Mol, vorzugsweise 0,9 bis 1,2 Mol einer Verbindung der Formel (V) ein.

Zur Durchführung des erfindungsgemäßen Verfahrens (c) setzt man pro Mol an Halogenverbindung der Formel (VI) im allgemeinen 1 bis 5 Mol, vorzugsweise 1,5 bis 3 Mol einer Verbindung der Formel (VII) ein.

Zur Durchführung des erfindungsgemäßen Verfahrens (d) setzt man pro Mol an Hydroxyalkoxyamid der Formel (VIII) im allgemeinen 1 bis 5 Mol, vorzugsweise 1,5 bis 4 Mol eines Dehydratisierungsmittels ein.

Die Reaktionsdurchführung, Aufarbeitung und Isolierung der Reaktionsprodukte erfolgt jeweils nach bekannten Methoden (vgl. die Herstellungsbeispiele).

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:

Pythium-Arten, wie beispielsweise Pythium ultimum;

Phytophthora-Arten, wie beispielsweise Phytophthora infestans;

Pseudoperonospora-Arten, wie beispielsweise Pseudoperonospora humuli oder Pseudoperonospora cubense;

Plasmopara-Arten, wie beispielsweise Plasmopara viticola;

Peronospora-Arten, wie beispielsweise Peronospora pisi oder Peronospora brassicae;

Erysiphe-Arten, wie beispielsweise Erysiphe graminis;

Sphaerotheca-Arten, wie beispielsweise Sphaerotheca fuliginea;

Podosphaera-Arten, wie beispielsweise Podosphaera leucotricha;

Venturia-Arten, wie beispielsweise Venturia inaequalis;

Pyrenophora-Arten, wie beispielweise Pyrenophora teres oder Pyrenophora graminea (Konidienform: Drechslera, Synonym: Helminthosporium);

Cochliobolus-Arten, wie beispielsweise Cochliobolus sativus (Konidienform: Drechslera, Synonym: Helminthosporium);

Uromyces-Arten, wie beispielsweise Uromyces appendiculatus;

Puccinia-Arten, wie beispielsweise Puccinia recondita;

Tilletia-Arten, wie beispielsweise Tilletia caries;

Ustilago-Arten, wie beispielsweise Ustilago nuda oder Ustilago avenae;

Pellicularia-Arten, wie beispielsweise Pellicularia sasakii;

Pyricularia-Arten, wie beispielsweise Pyricularia oryzae;

Fusarium-Arten, wie beispielsweise Fusarium culmorum;

Botrytis-Arten, wie beispielsweise Botrytis cinerea;

Septoria-Arten, wie beispielsweise Septoria nodorum;

Leptosphaeria-Arten, wie beispielsweise Leptosphaeria nodorum;

Cercospora-Arten, wie beispielsweise Cercospora canescens;

Alternaria-Arten, wie beispielsweise Alternaria brassicae;

Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Dabei können die erfindungsgemäßen Wirkstoffe mit besonders gutem Erfolg zur Bekämpfung von Krankheiten im Obst- und Gemüseanbau, wie beispielsweise gegen Phytophthora-Arten, oder zur Bekämpfung von Getreidekrankheiten wie beispielsweise gegen Pyrenophora-Arten eingesetzt werden.

Daneben zeigen die erfindungsgemäßen Wirkstoffe eine gute Wirkung beispielsweise gegen Erisyphe graminis, Cochliobolus sativus, Leptosphaeria nodorum, Pseudocercosporella herpotrichoides und Fusarien an Getreide, gegen Pyricularia oryzae und Pellicularia sasakii an Reis sowie eine breite in vitro-Wirksamkeit.

Die Wirkstoffe können in Abhängigkeit von ihrenj eweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und -Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische

Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z. B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Besonders günstige Mischpartner sind z.B. die folgenden Verbindungen:

**Fungizide:**

2-Aminobutan;2-Anilino-4-methyl-6-cyclopropyl-pyrimidin;2',6'-Dibromo-2-methyl-4'-trifluoromethoxy-4'-trifluoromethyl-1,3-thiazole-5-carboxanilid; 2,6-Dichloro-N-(4-trifluoromethylbenzyl)benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)pyrimidin-4-yloxy]phenyl}-3-methoxyacrylat; Methyl-(E)-methoximino[alpha-(o-tolyloxy)-o-tolyl] acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodin, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetate, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat, Kupferoxychlorid, Kupfersulfat, Kupferoxid, Oxin-Kupfer and Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickeldimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram

**Bakterizide:**

Bronopol, Dichlorophen, Nitrapyrin, Nickel Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

**Insektizide / Akarizide / Nematizide:**

Abamectin, Abamectin, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, 4-Bromo-2-(4-chlorphenyl)-1-(ethoxymethyl)-5-(trifluoromethyl)-1H-pyrrole-3-carbonitrile, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, Chloethocarb, Chlorethoxyfos, Chloretoxyfos, Chlorfenvinphos, Chlorfluazuron, Chlormephos, N-[(6-Chloro-3-pyridinyl)-methyl]-N'-cyano-N-methyl-ethanimidamide, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etofenprox, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivemectin, Lamda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, Nitenpyram
Omethoat, Oxamyl, Oxydemethon M, Oxydeprofos,
Parathion A, Parathion M, Permethrin, Phenthoat, Phorat, Phosalon, Phosmet, Phosphamdon, Phoxim, Pirimicarb, Pirimiphos M, Primiphos A, Profenofos, Profenophos, Promecarb, Propaphos, Propoxur, Prothiofos, Prothiophos, Prothoat, Pymetrozin, Pyrachlophos, Pyraclofos, Pyraclophos, Pyradaphenthion, Pyresmethrin, Pyrethrum, Pyridaben, Pyrimidifen, Pyriproxifen,
Quinalphos,
Salithion, Sebufos, Silafluofen, Sulfotep, Sulprofos,
Tebufenozide, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden:

Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Herstellungsbeispiele:

Beispiel 1

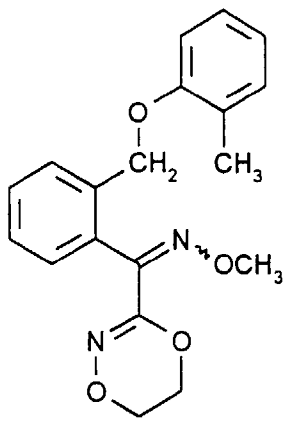

1,8 g (25 mMol) Hydroxylamin-Hydrochlorid werden bei 20°C in 20 ml Methanol vorgelegt und langsam mit einer Lösung von 3,3 g Kaliumhydroxid (86%ig) in 20 ml Methanol versetzt. Anschließend werden 4,0 g (12,8 mMol) α-Methoximino-α-[2-(2-methyl-phenoxy-methyl)-phenyl]-essigsäure-methylester portionsweise dazu gegeben und das Reaktionsgemisch wird dann bis zum Ende der Umsetzung (dünnschichtchromatographische Kontrolle) bei 40°C gerührt. Dann werden zunächst 1,7 g (12,8 mMol) Kaliumcarbonat und anschließend 10,8 g (59 mMol) 1,2-Dibrom-ethan zur Reaktionsmischung gegeben. Die Mischung wird dann 12 Stunden bei 65°C gerührt, anschließend auf 20°C abgekühlt und filtriert. Das Filtrat wird im Wasserstrahlvakuum eingeengt und der Rückstand durch Säulenchromatographie an Kieselgel (Toluol/Aceton, Vol.: 9:1) gereinigt.

Man erhält 1,4 g (33% der Theorie) 3-{α-Methoximino-α-[2-(2-methyl-phenoxymethyl)-phenyl]-methyl}-5,6-dihydro-1,4,2-dioxazin.

Brechungsindex $n_D^{20}$=1,5705.

Analog Beispiel 1 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

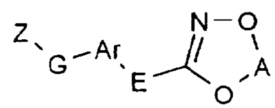

(I)

Die $^1$H-NMR-Spektren wurden aufgenommen in $CDCl_3$ mit Tetramethylsilan als innerem Standard. Angegeben sind in der Regel die chemischen Verschiebungen als δ-Werte.

Tabelle 1: Beispiele für die Verbindungen der Formel (I)

| Bsp.-Nr. | Z | G | Ar | E | A | Physikal. Daten |
|---|---|---|---|---|---|---|
| 2 | 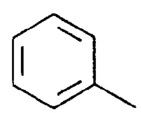 | 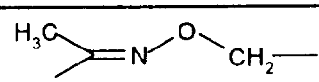 | 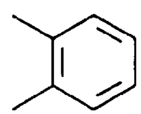 | 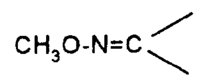 | $CH_2CH_2$ | $^1$H-NMR: 2,25; 3,95; 4,05-4,51; 5,15; 7,0-7,68 ppm |
| 3 | 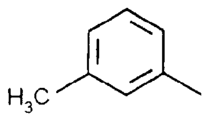 | 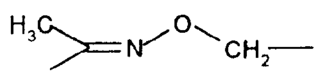 | 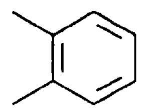 | 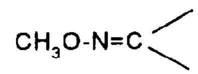 | $CH_2CH_2$ | $^1$H-NMR: 2,23; 2,25; 3,98; 4,1-4,52; 7,05-7,55 ppm |

| Bsp.-Nr. | Z | G | Ar | E | A | Physikal. Daten |
|---|---|---|---|---|---|---|
| 4 | 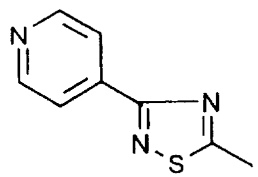 | O | 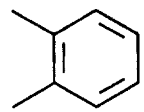 | $CH_3O-CH=C$ | $CH_2CH_2$ | |
| 5 | 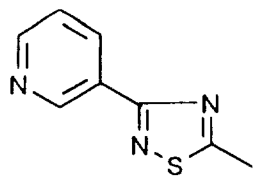 | O | 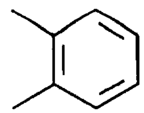 | $CH_3O-CH=C$ | $CH_2CH_2$ | |
| 6 | 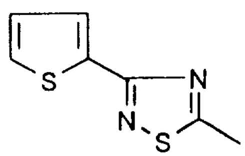 | O | 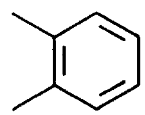 | $CH_3O-CH=C$ | $CH_2CH_2$ | |
| 7 | 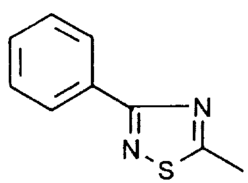 | O | 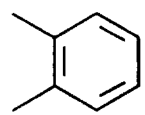 | $CH_3O-CH=C$ | $CH_2CH_2$ | |

| Bsp.-Nr. | Z | G | Ar | E | A | Physikal. Daten |
|---|---|---|---|---|---|---|
| 11 | 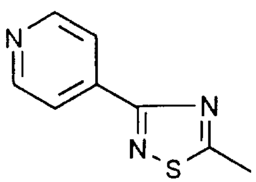 | O | 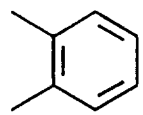 | 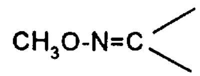 | $CH_2CH_2$ | Fp.: 138°C |
| 12 | 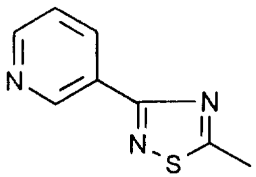 | O | 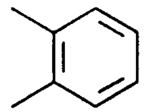 | 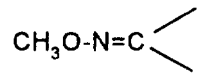 | $CH_2CH_2$ | Fp.: 65°C |
| 13 | 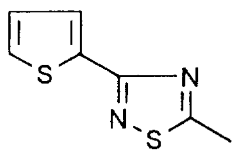 | O | 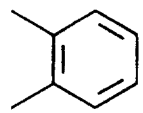 | 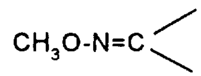 | $CH_2CH_2$ | (amorph)<br>H-NMR: ($CDCl_3$)<br>$\delta$ = 3,75 (s.3H) |
| 14 | 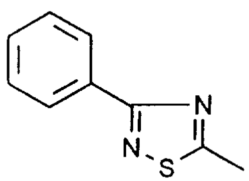 | O | 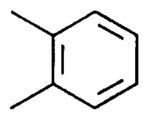 | 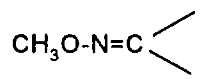 | $CH_2CH_2$ | Fp.: 70°C |

| Bsp.-Nr. | Z | G | Ar | E | A | Physikal. Daten |
|---|---|---|---|---|---|---|
| 8 | 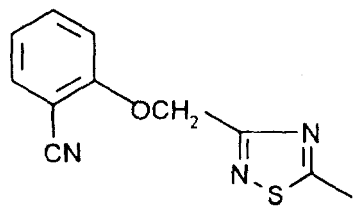 | O | 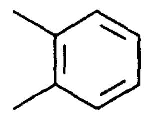 | 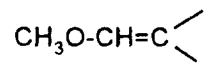 | $CH_2CH_2$ | |
| 9 | 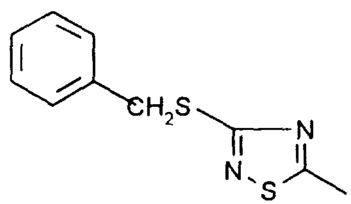 | O | 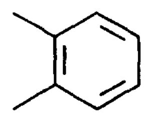 | 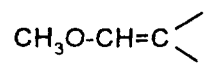 | $CH_2CH_2$ | |
| 10 | 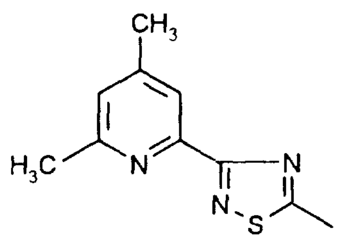 | O | 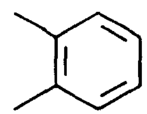 | 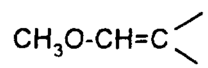 | $CH_2CH_2$ | |

| Bsp.-Nr. | Z | G | Ar | E | A | Physikal. Daten |
|---|---|---|---|---|---|---|
| 15 | 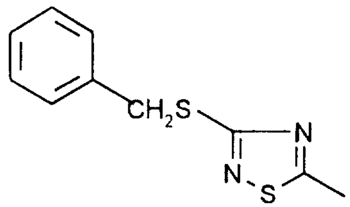 | O | 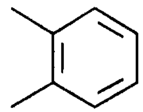 | 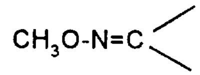 | $CH_2CH_2$ | Öl<br>$^1$H-NMR ($CDCl_3$)<br>$\delta$ = 3.80 (s,3H) |
| 16 | 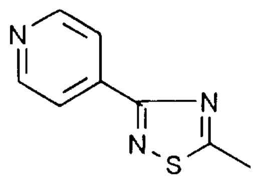 | $SO_2CH_2$ | 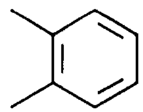 | 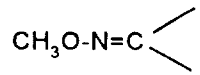 | $CH_2CH_2$ | |
| 17 | 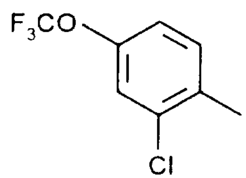 | $-OCH_2-$ | 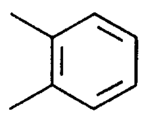 | 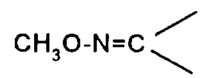 | $CH_2CH_2$ | |
| 18 | 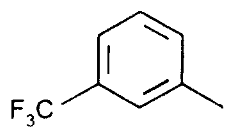 | $-OCH_2-$ | 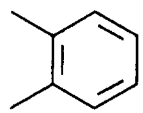 | 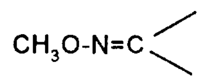 | $CH_2CH_2$ | amorph<br>H-NMR: 1,55; 3,97; 4,16; 4,48; 5,03; 7,08-7,51 ppm |

| Bsp.-Nr. | Z | G | Ar | E | A | Physikal. Daten |
|---|---|---|---|---|---|---|
| 19 | 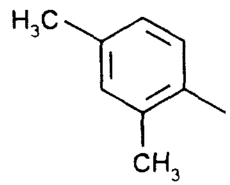 | $-OCH_2-$ | 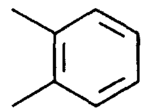 | 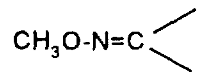 | $CH_2CH_2$ | Fp.: 129°C |
| 20 | 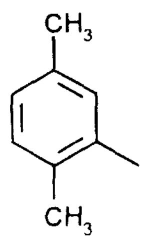 | $-OCH_2-$ | 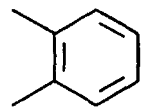 | 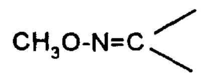 | $CH_2CH_2$ | (amorph) H-NMR: 2,24; 2,28; 3,99; 4,15; 4,46; 4,98; 6,62-7,59 ppm |
| 21 | 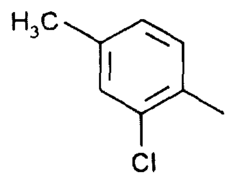 | $-OCH_2-$ | 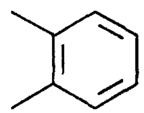 | 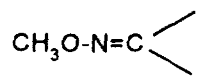 | $CH_2CH_2$ | |
| 22 | 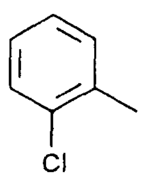 | $-OCH_2-$ | 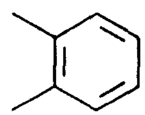 | 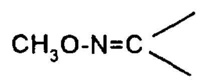 | $CH_2CH_2$ | Fp.: 123°C |

| Bsp.-Nr. | Z | G | Ar | E | A | Physikal. Daten |
|---|---|---|---|---|---|---|
| 23 | 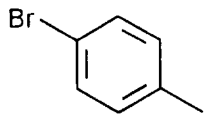 | $-OCH_2-$ | 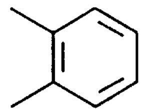 | 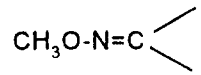 | $CH_2CH_2$ | |
| 24 | 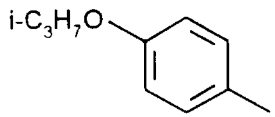 | $-OCH_2-$ | 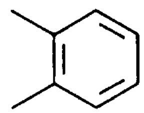 | 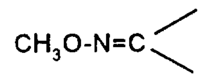 | $CH_2CH_2$ | |
| 25 | 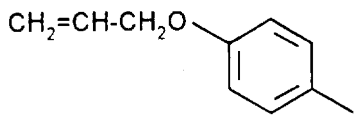 | $-OCH_2-$ | 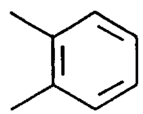 | 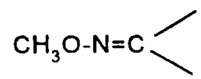 | $CH_2CH_2$ | |
| 26 | 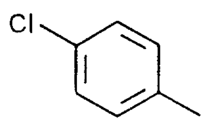 | $-OCH_2-$ | 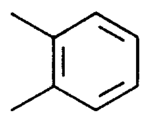 | 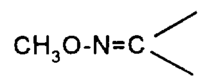 | $CH_2CH_2$ | |
| 27 | 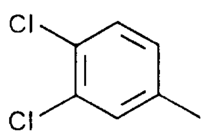 | $-OCH_2-$ | 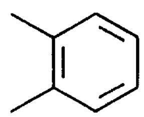 | 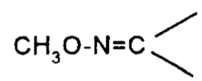 | $CH_2CH_2$ | |

| Bsp.-Nr. | Z | G | Ar | E | A | Physikal. Daten |
|---|---|---|---|---|---|---|
| 28 | 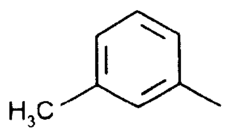 | O | 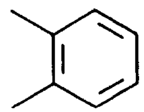 | 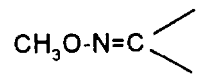 | $CH_2CH_2$ | |
| 29 | 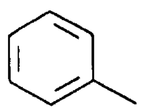 | O | 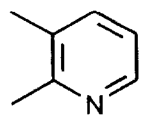 | 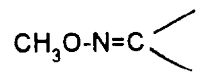 | $CH_2CH_2$ | |
| 30 | 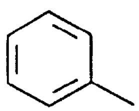 | O | 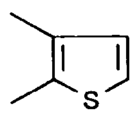 | 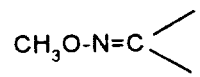 | $CH_2CH_2$ | Öl |

| Bsp.-Nr. | Z | G | Ar | E | A | Physikal. Daten |
|---|---|---|---|---|---|---|
| 31 | 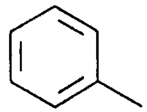 | 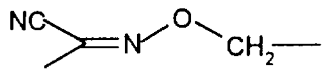 | 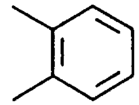 | 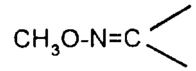 | $CH_2CH_2$ | |
| 32 | 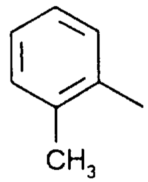 | 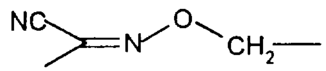 | 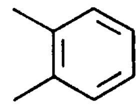 | 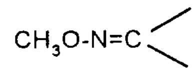 | $CH_2CH_2$ | |
| 33 | 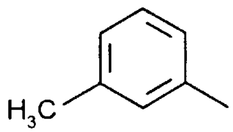 | 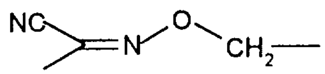 | 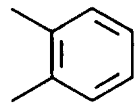 | 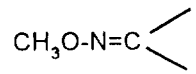 | $CH_2CH_2$ | |
| 34 | 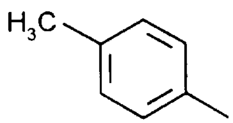 | 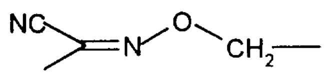 | 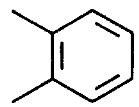 | 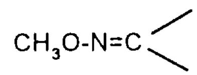 | $CH_2CH_2$ | |
| 35 | 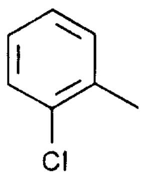 | 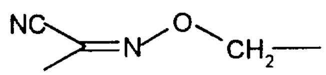 | 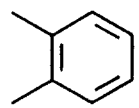 | 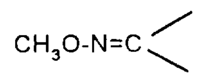 | $CH_2CH_2$ | |

| Bsp.-Nr. | Z | G | Ar | E | A | Physikal. Daten |
|---|---|---|---|---|---|---|
| 36 | 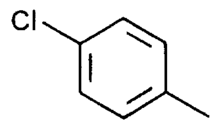 | 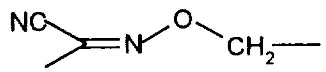 | 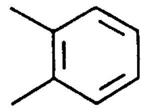 | 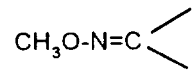 | $CH_2CH_2$ | |
| 37 | 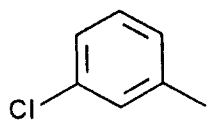 | 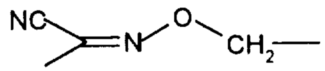 | 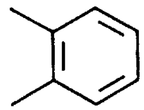 | 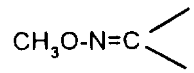 | $CH_2CH_2$ | |
| 38 | 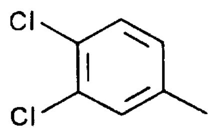 | 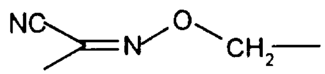 | 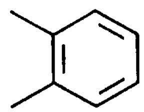 | 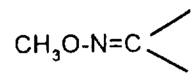 | $CH_2CH_2$ | |
| 39 | 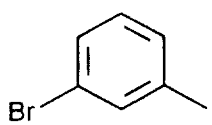 | 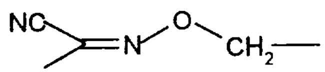 | 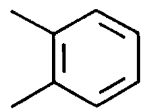 | 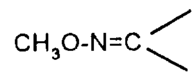 | $CH_2CH_2$ | |
| 40 | 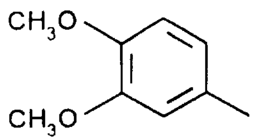 | 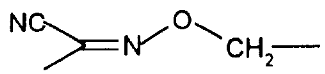 | 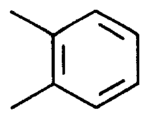 | 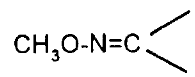 | $CH_2CH_2$ | |

| Bsp.-Nr. | Z | G | Ar | E | A | Physikal. Daten |
|---|---|---|---|---|---|---|
| 41 | 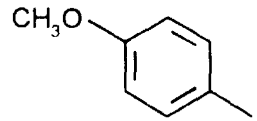 | 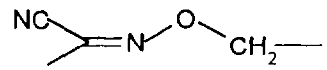 | 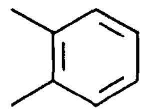 | 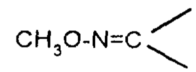 | $CH_2CH_2$ | |
| 42 | 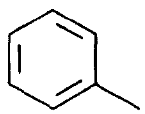 | 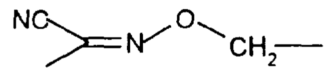 | 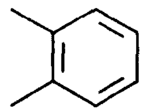 | 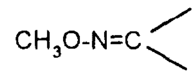 | $CH_2CH_2$ | |
| 43 | 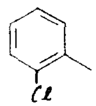 | 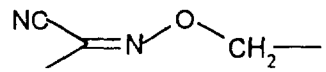 | 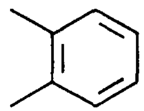 | 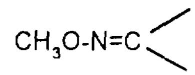 | $CH_2CH_2$ | |
| 44 | 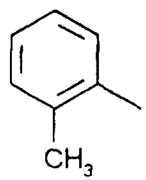 | 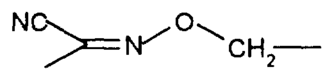 | 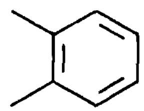 | 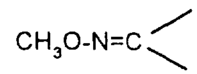 | $CH_2CH_2$ | |
| 45 | 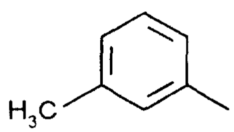 | 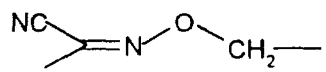 | 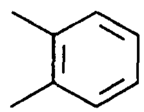 | 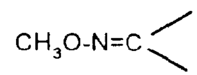 | $CH_2CH_2$ | |

| Bsp.-Nr. | Z | G | Ar | E | A | Physikal. Daten |
|---|---|---|---|---|---|---|
| 46 | 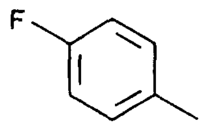 | 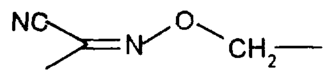 | 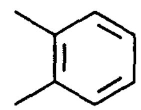 | 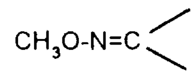 | $CH_2CH_2$ | |
| 47 | 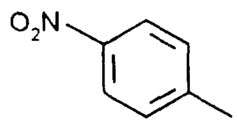 | 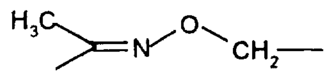 | 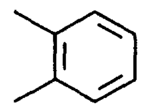 | 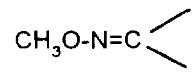 | $CH_2CH_2$ | |
| 48 | 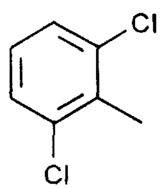 | 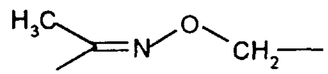 | 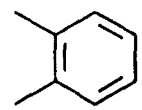 | 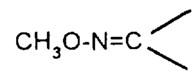 | $CH_2CH_2$ | |
| 49 | 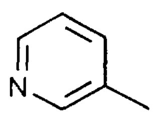 | 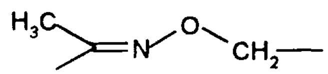 | 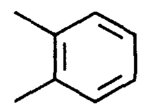 | 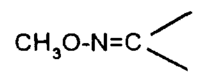 | $CH_2CH_2$ | |
| 50 | 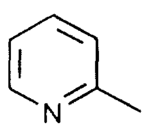 | 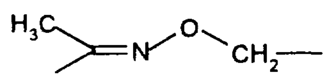 | 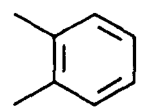 | 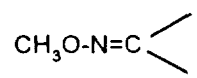 | $CH_2CH_2$ | |

| Bsp.-Nr. | Z | G | Ar | E | A | Physikal. Daten |
|---|---|---|---|---|---|---|
| 51 | 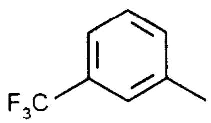 | 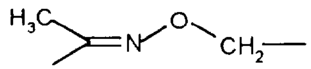 | 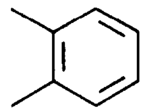 | 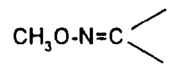 | $CH_2CH_2$ | $^1H$-NMR ($CDCl_3$, δ): 2,26 (3H); 3,96 (3H); 4,1-4,2 (2H); 4,4-4,5 (2H); 5,204 (2H); 7,0-8,0 (8H) ppm |
| 52 | 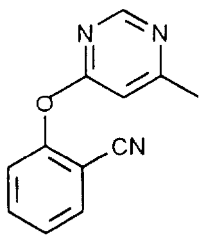 | O | 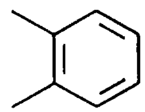 | 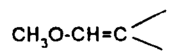 | $CH_2CH_2$ | |
| 53 | 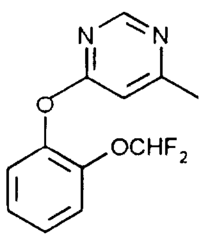 | O | 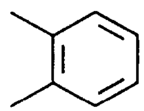 | 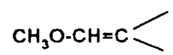 | $CH_2CH_2$ | |

| Bsp.-Nr. | Z | G | Ar | E | A | Physikal. Daten |
|---|---|---|---|---|---|---|
| 54 | 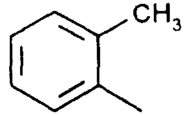 | $-OCH_2-$ | 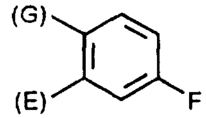 | 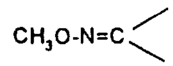 | $CH_2CH_2$ | $^1$H-NMR ($CDCl_3$, δ): 2,30 (3H); 3,98 (3H); 4,1-4,2 (2H); 4,4-4,5 (2H); 4,97 (2H); 6,4-7,4 (7H) ppm |
| 55 | 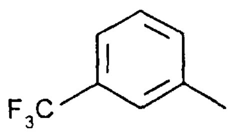 | 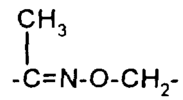 | 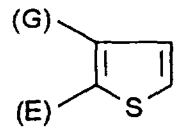 | 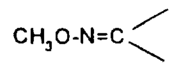 | $CH_2CH_2$ | $^1$H-NMR ($CDCl_3$, δ): 2,25 (3H); 4,0 (3H); 4,1-4,2 (2H); 4,4-4,5 (2H); 5,176 (2H); 7,114-7,131 (1H); 7,44-7,45 (1H); 7,4-8,0 (4H) ppm |
| 56 | 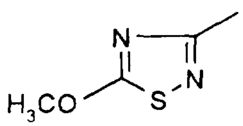 | $-CH_2O-$ | 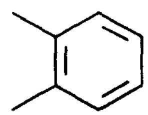 | 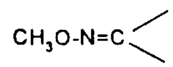 | $CH_2CH_2$ | Öl |

| Bsp.-Nr. | Z | G | Ar | E | A | Physikal. Daten |
|---|---|---|---|---|---|---|
| 57 | 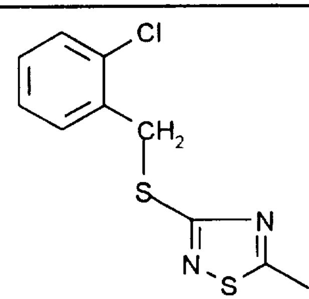 | -O- | 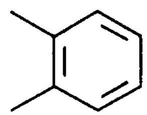 | 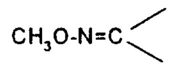 | $CH_2CH_2$ | (amorph)<br>$^1$H-NMR ($CDCl_3$)<br>δ = 3.80 (s,3H) |
| 58 | 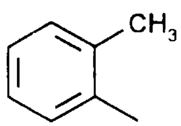 | $-CH_2O-$ | 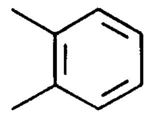 | 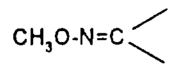 | $CH_2CH_2$ | Fp.: 142°C |
| 59 | 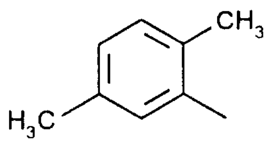 | $-CH_2O-$ | 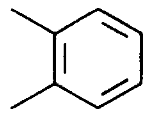 | 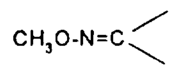 | $CH_2CH_2$ | Öl<br>δ = 3,95 (s,3H) |
| 60 | 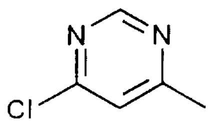 | -O- | 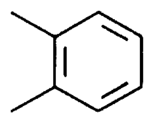 | 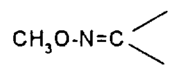 | $CH_2CH_2$ | m.p. 106°C |

| Bsp.-Nr. | Z | G | Ar | E | A | Physikal. Daten |
|---|---|---|---|---|---|---|
| 61 | 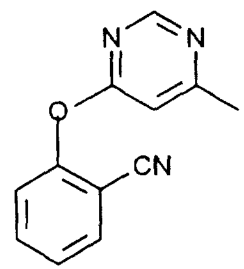 | -O- | 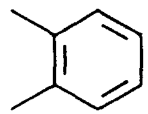 | 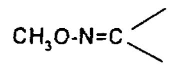 | $CH_2CH_2$ | Fp.: 82°C |
| 62 | 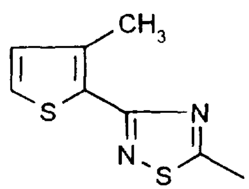 | -O- | 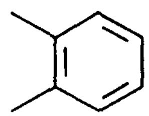 | 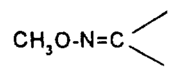 | $CH_2CH_2$ | Öl<br>$^1$H-NMR ($CDCl_3$)<br>$\delta$ = 3,80 (s,3H) |
| 63 | 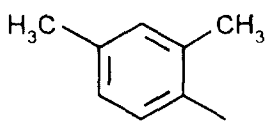 | $-CH_2O-$ | 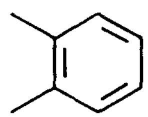 | 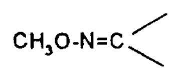 | $CH_2CH_2$ | Fp.: 123°C |
| 64 | 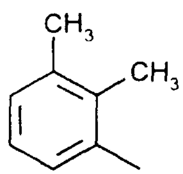 | $-OCH_2-$ | 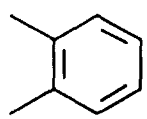 | 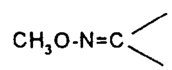 | $CH_2CH_2$ | Fp.: 75°C |

| Bsp.-Nr. | Z | G | Ar | E | A | Physikal. Daten |
|---|---|---|---|---|---|---|
| 65 | 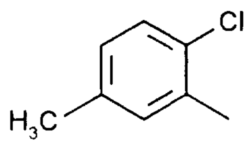 | $-OCH_2-$ | 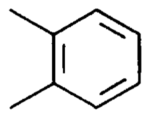 | 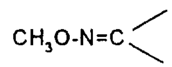 | $CH_2CH_2$ | Fp.: 119°C |
| 66 | 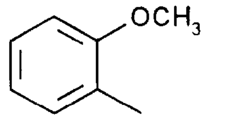 | $-OCH_2-$ | 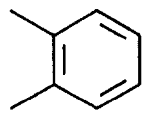 | 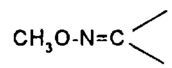 | $CH_2CH_2$ | Fp.: 83°C |
| 67 | 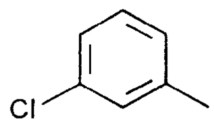 | $-OCH_2-$ | 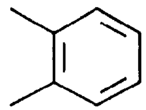 | 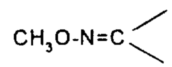 | $CH_2CH_2$ | (amorph) $^1$H-NMR: 3,98; 4,15; 4,47; 4,98; 6,80; 7,55 ppm |
| 68 | 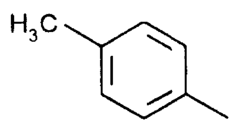 | $-OCH_2-$ | 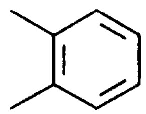 | 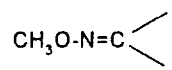 | $CH_2CH_2$ | (amorph) $^1$H-NMR: 2,27; 3,97; 4,14; 4,47; 4,96; 6,18-7,53 ppm |
| 69 | 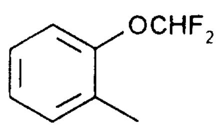 | $-OCH_2-$ | 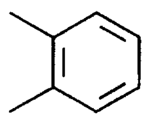 | 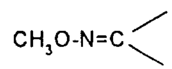 | $CH_2CH_2$ | (amorph) $^1$H-NMR: 3,95; 4,15; 4,48; 5,07; 6,92-7,54 ppm |

| Bsp.-Nr. | Z | G | Ar | E | A | Physikal. Daten |
|---|---|---|---|---|---|---|
| 70 | 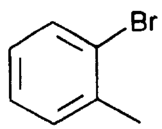 | $-OCH_2-$ | 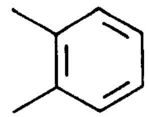 | 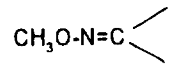 | $CH_2CH_2$ | Fp.: 131°C |
| 71 | 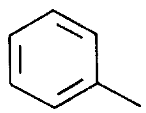 | -O- | 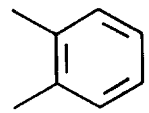 | 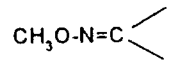 | $CH_2CH_2$ | Fp.: 95°C |
| 72 | 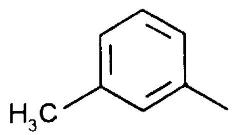 | -O- | 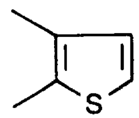 | 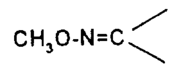 | $CH_2CH_2$ | Öl |
| 73 | 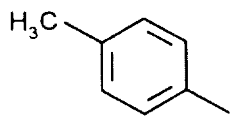 | -O- | 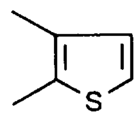 | 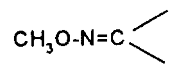 | $CH_2CH_2$ | Fp.: 119°C |
| 74 | 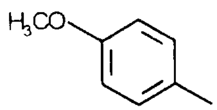 | -O- | 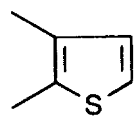 | 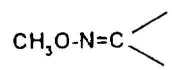 | $CH_2CH_2$ | Öl |

| Bsp.-Nr. | Z | G | Ar | E | A | Physikal. Daten |
|---|---|---|---|---|---|---|
| 75 | 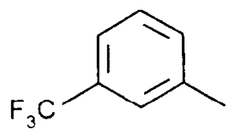 | -O- | 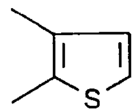 | 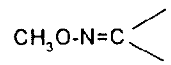 | $CH_2CH_2$ | Öl |
| 76 | 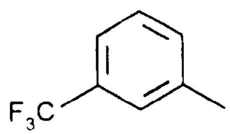 | -O- | 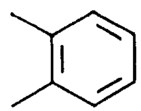 | 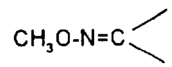 | $CH_2CH_2$ | (amorph) |
| 77 | 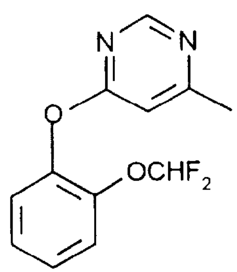 | -O- | 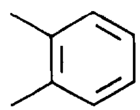 | 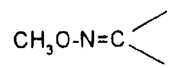 | $CH_2CH_2$ | Öl $^1$H-NMR($CDCl_3$) $\delta$ = 3.80 (s, 3H) |
| 78 | 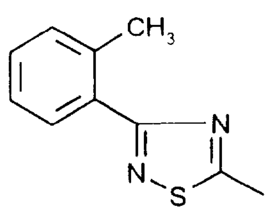 | $-CH_2S-$ | 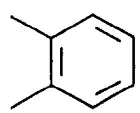 | 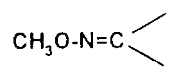 | $CH_2CH_2$ | |

| Bsp.-Nr. | Z | G | Ar | E | A | Physikal. Daten |
|---|---|---|---|---|---|---|
| 79 | 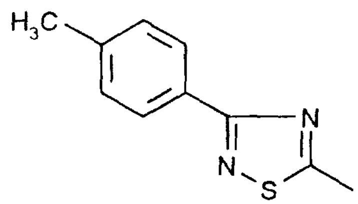 | $-SCH_2-$ | 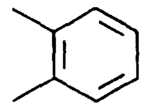 | 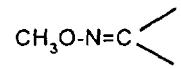 | $CH_2CH_2$ | |
| 80 | 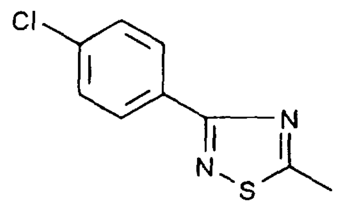 | $-CH_2S-$ | 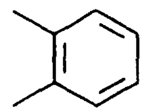 | 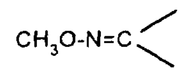 | $CH_2CH_2$ | |
| 81 | 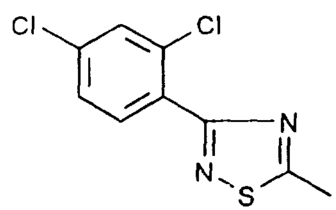 | $-SCH_2-$ | 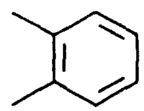 | 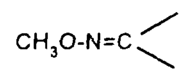 | $CH_2CH_2$ | |
| 82 | 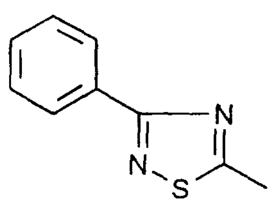 | S | 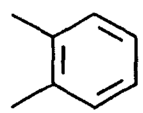 | 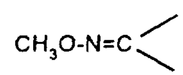 | $CH_2CH_2$ | |

| Bsp.-Nr. | Z | G | Ar | E | A | Physikal. Daten |
|---|---|---|---|---|---|---|
| 83 | 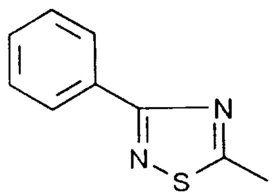 | - | 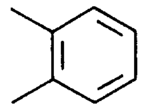 | 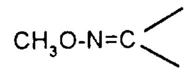 | $CH_2CH_2$ | |
| 84 | 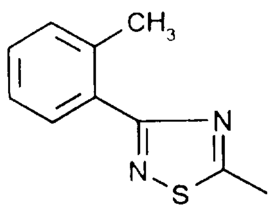 | -O- | 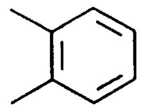 | 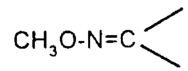 | $CH_2CH_2$ | |
| 85 | 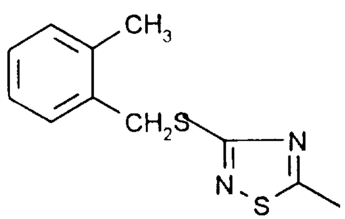 | -O- | 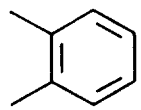 | 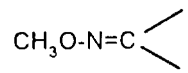 | $CH_2CH_2$ | Öl $^1H$-NMR($CDCl_3$) $\delta = 3.80$ (s,3H) |

| Bsp.-Nr. | Z | G | Ar | E | A | Physikal. Daten |
|---|---|---|---|---|---|---|
| 86 | 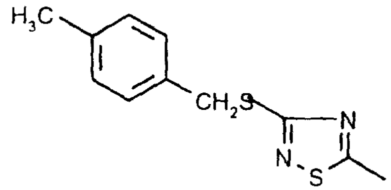 | -O- | 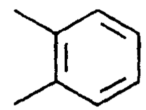 | 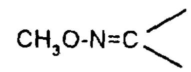 | $CH_2CH_2$ | Fp.: 128°C |
| 87 | 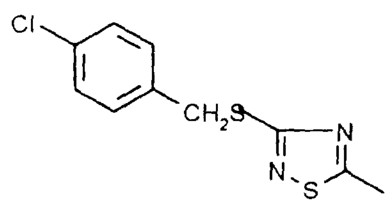 | -O- | 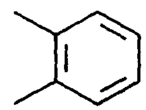 | 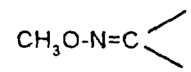 | $CH_2CH_2$ | Öl $^1$H-NMR($CDCl_3$) $\delta$ = 3.80 (s,3H) |
| 88 | 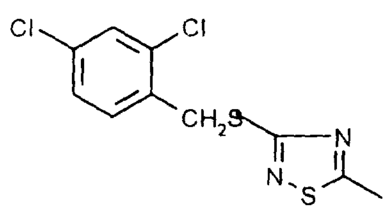 | -O- | 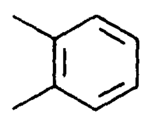 | 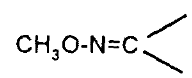 | $CH_2CH_2$ | |

| Bsp.-Nr. | Z | G | Ar | E | A | Physikal. Daten |
|---|---|---|---|---|---|---|
| 89 | 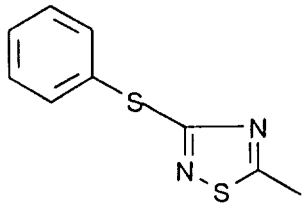 | -O- | 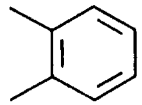 | 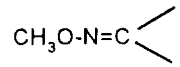 | $CH_2CH_2$ | |
| 90 | 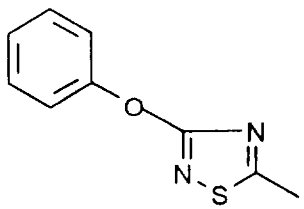 | -O- | 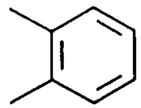 | 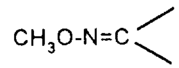 | $CH_2CH_2$ | |
| 91 | 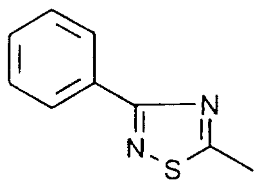 | -O- | 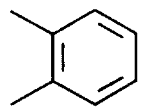 | 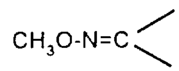 | $CH_2CH_2$ | m.p. 70°C |
| 92 | 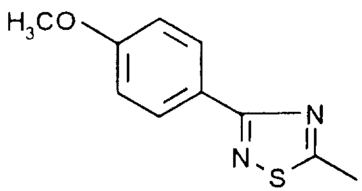 | -O- | 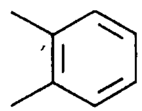 | 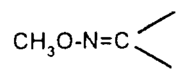 | $CH_2CH_2$ | m.p. 70-73°C |

| Bsp.-Nr. | Z | G | Ar | E | A | Physikal. Daten |
|---|---|---|---|---|---|---|
| 93 | 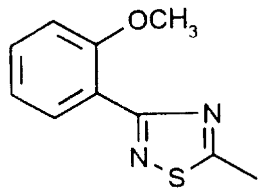 | -O- | 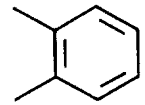 | 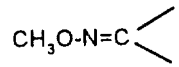 | $CH_2CH_2$ | amorph<br>$^1$H-NMR($CDCl_3$)<br>$\delta$ = 3.85 (s,3H) |
| 94 | 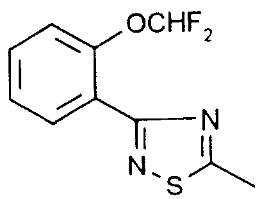 | -O- | 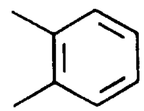 | 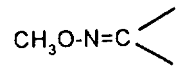 | $CH_2CH_2$ | Öl<br>$\delta$ = 3.85 (s, 3H) |
| 95 | 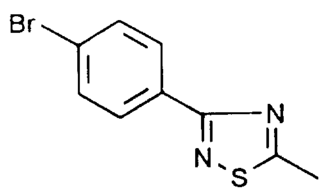 | -O- | 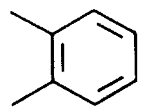 | 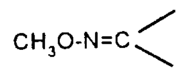 | $CH_2CH_2$ | Fp.: 132°C |
| 96 | 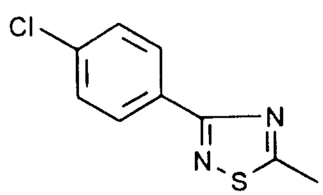 | -O- | 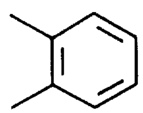 | 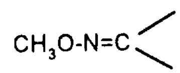 | $CH_2CH_2$ | |

| Bsp.-Nr. | Z | G | Ar | E | A | Physikal. Daten |
|---|---|---|---|---|---|---|
| 97 | 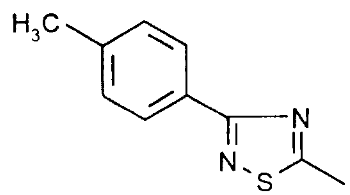 | -O- | 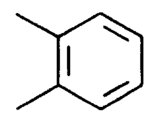 | 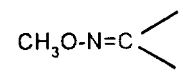 | $CH_2CH_2$ | |
| 98 | 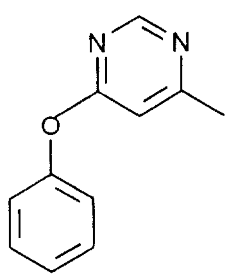 | -O- | 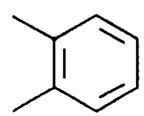 | 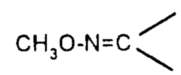 | $CH_2CH_2$ | Öl $^1$H-NMR($CDCl_3$) $\delta$ = 3.80 (s,3H) |
| 99 | 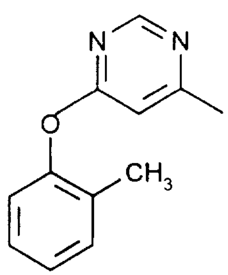 | -O- | 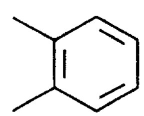 | 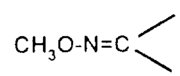 | $CH_2CH_2$ | Öl $\delta$ = 3.80 (s, 3H) |

| Bsp.-Nr. | Z | G | Ar | E | A | Physikal. Daten |
|---|---|---|---|---|---|---|
| 100 | 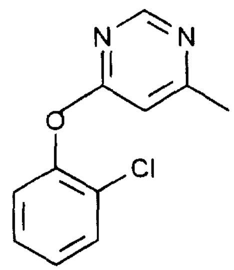 | -O- | 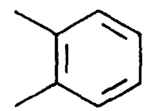 | 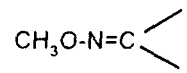 | $CH_2CH_2$ | amorph<br>$^1H$-NMR($CDCl_3$)<br>$\delta$ = 3.80 (s, 3H) |
| 101 | 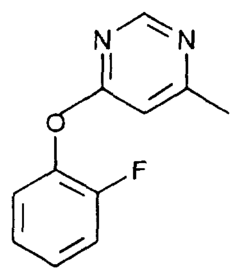 | -O- | 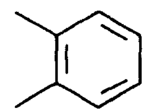 | 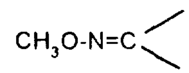 | $CH_2CH_2$ | |
| 102 | 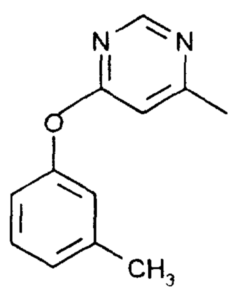 | -O- | 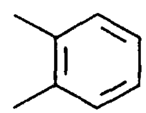 | 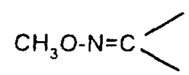 | $CH_2CH_2$ | amorph<br>$\delta$ = 3.80 (s, 3H) |

| Bsp.-Nr. | Z | G | Ar | E | A | Physikal. Daten |
|---|---|---|---|---|---|---|
| 103 | 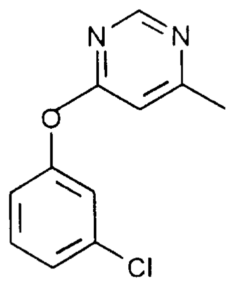 | -O- | 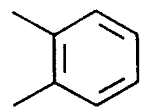 | 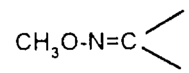 | $CH_2CH_2$ | amorph $^1$H-NMR($CDCl_3$) $\delta$ = 3.80 (s,3H) |
| 104 | 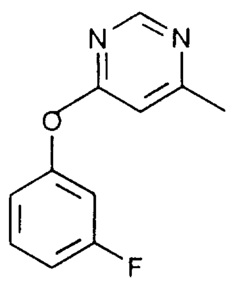 | -O- | 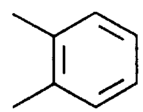 | 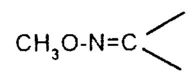 | $CH_2CH_2$ | |

| Bsp.-Nr. | Z | G | Ar | E | A | Physikal. Daten |
|---|---|---|---|---|---|---|
| 105 | 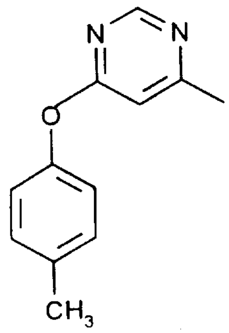 | -O- | 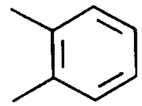 | 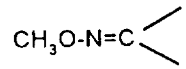 | $CH_2CH_2$ | amorph<br>$^1$H-NMR($CDCl_3$)<br>$\delta$ = 3.80 (s, 3H) |
| 106 | 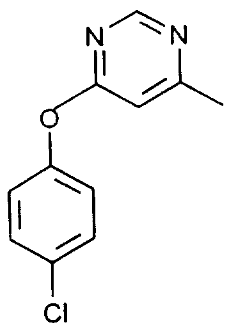 | -O- | 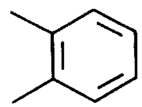 | 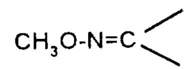 | $CH_2CH_2$ | amorph<br>$^1$H-NMR($CDCl_3$)<br>$\delta$ = 3.80 (s, 3H) |

| Bsp.-Nr. | Z | G | Ar | E | A | Physikal. Daten |
|---|---|---|---|---|---|---|
| 107 | 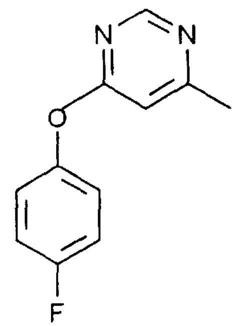 | -O- | 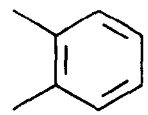 | 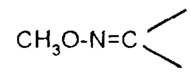 | $CH_2CH_2$ | |
| 108 | 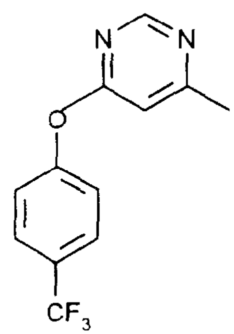 | -O- | 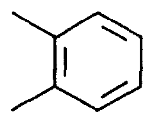 | 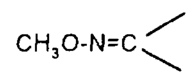 | $CH_2CH_2$ | Öl<br>$^1$HNMR ($CDCl_3$)<br>$\delta$ = 3.80 (s, 3H) |

| Bsp.-Nr. | Z | G | Ar | E | A | Physikal. Daten |
|---|---|---|---|---|---|---|
| 109 | 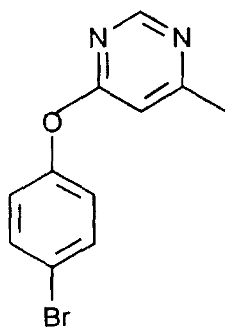 | -O- | 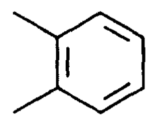 | 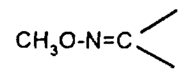 | $CH_2CH_2$ | |
| 110 | 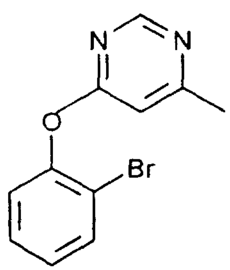 | -O- | 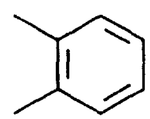 | 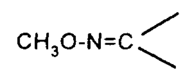 | $CH_2CH_2$ | amorph<br>$^1$HNMR ($CDCl_3$)<br>$\delta$ = 3.80 (s, 3H) |

| Bsp.-Nr. | Z | G | Ar | E | A | Physikal. Daten |
|---|---|---|---|---|---|---|
| 111 | 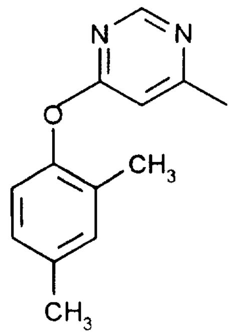 | -O- | 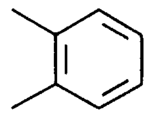 | 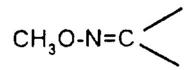 | $CH_2CH_2$ | amorph<br>$^1$HNMR ($CDCl_3$)<br>$\delta$ = 3.80 (s, 3H) |
| 112 | 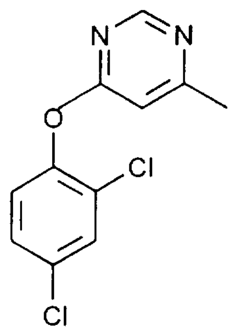 | -O- | 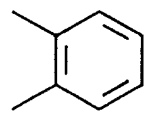 | 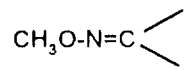 | $CH_2CH_2$ | amorph<br>$\delta$ = 3.80 (s, 3H) |

| Bsp.-Nr. | Z | G | Ar | E | A | Physikal. Daten |
|---|---|---|---|---|---|---|
| 113 | 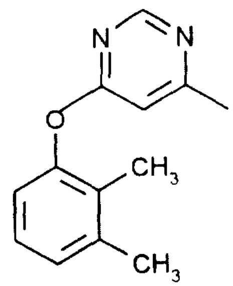 | -O- | 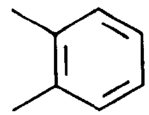 | 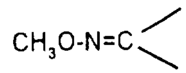 | $CH_2CH_2$ | Öl<br>$^1$HNMR ($CDCl_3$)<br>$\delta = 3.80$ (s, 3H) |
| 114 | 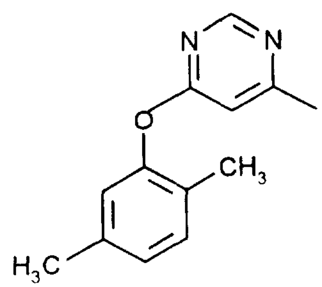 | -O- | 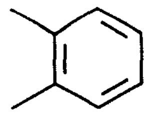 | 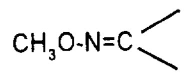 | $CH_2CH_2$ | amorph<br>$\delta = 3.80$ (s, 3H) |
| 115 | 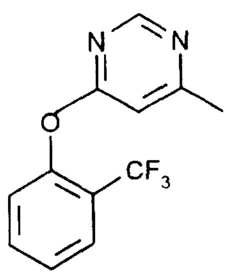 | -O- | 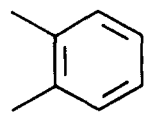 | 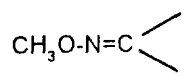 | $CH_2CH_2$ | |

| Bsp.-Nr. | Z | G | Ar | E | A | Physikal. Daten |
|---|---|---|---|---|---|---|
| 116 | 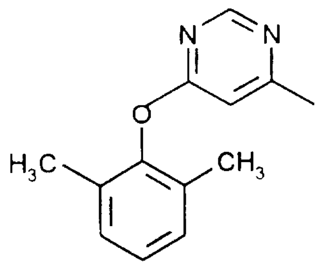 | -O- | 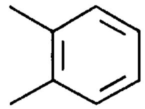 | 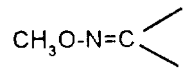 | $CH_2CH_2$ | amorph<br>$^1$HNMR ($CDCl_3$)<br>$\delta = 3.80$ (s, 3H) |
| 117 | 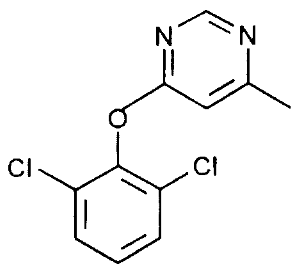 | -O- | 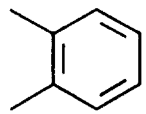 | 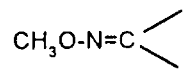 | $CH_2CH_2$ | |

| Bsp.-Nr. | Z | G | Ar | E | A | Physikal. Daten |
|---|---|---|---|---|---|---|
| 118 | 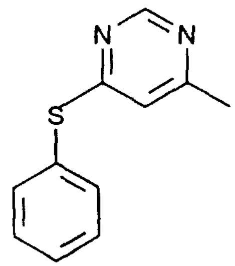 | -O- | 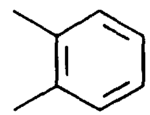 | $CH_3O-N=C$ | $CH_2CH_2$ | amorph<br>$^1$HNMR ($CDCl_3$)<br>$\delta = 3.80$ (s, 3H) |
| 119 | 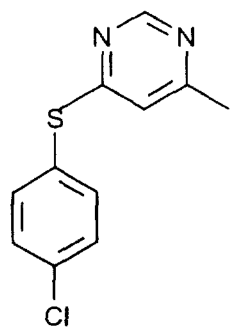 | -O- | 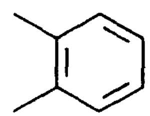 | $CH_3O-N=C$ | $CH_2CH_2$ | amorph<br>$\delta = 3.80$ (s, 3H) |

| Bsp.-Nr. | Z | G | Ar | E | A | Physikal. Daten |
|---|---|---|---|---|---|---|
| 120 | 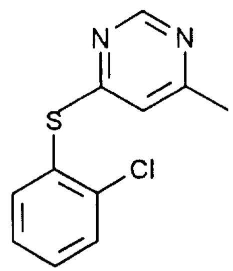 | -O- | 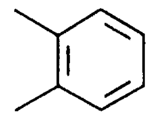 | 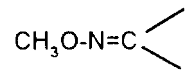 | $CH_2CH_2$ | amorph<br>$^1$HNMR ($CDCl_3$)<br>δ = 3.80 (s, 3H) |
| 121 | 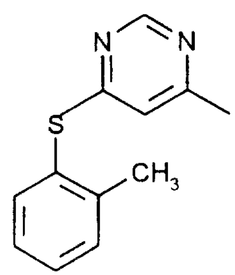 | -O- | 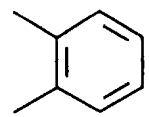 | 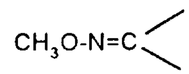 | $CH_2CH_2$ | |
| 122 | 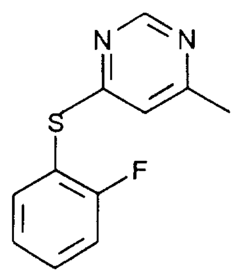 | -O- | 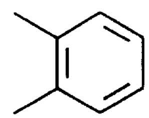 | 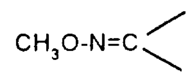 | $CH_2CH_2$ | |

| Bsp.-Nr. | Z | G | Ar | E | A | Physikal. Daten |
|---|---|---|---|---|---|---|
| 123 | 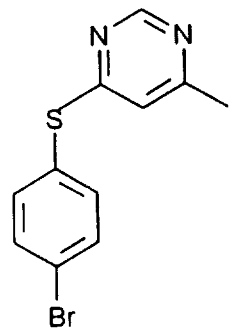 | -O- | 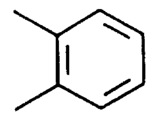 | $CH_3O-N=C<$ | $CH_2CH_2$ | |
| 124 | 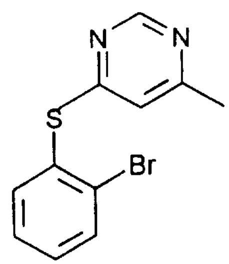 | -O- | 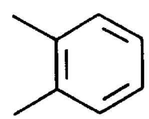 | $CH_3O-N=C<$ | $CH_2CH_2$ | |

| Bsp.-Nr. | Z | G | Ar | E | A | Physikal. Daten |
|---|---|---|---|---|---|---|
| 125 | 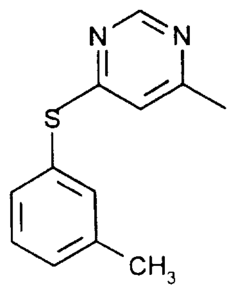 | -O- | 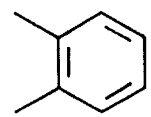 | 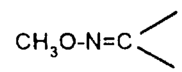 | $CH_2CH_2$ | |
| 126 | 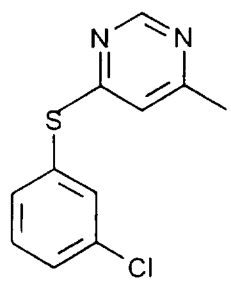 | -O- | 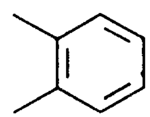 | 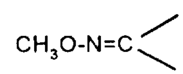 | $CH_2CH_2$ | |

| Bsp.-Nr. | Z | G | Ar | E | A | Physikal. Daten |
|---|---|---|---|---|---|---|
| 127 | 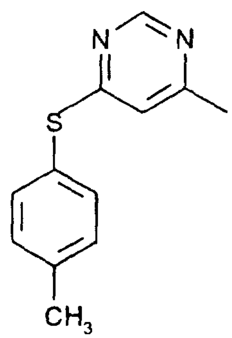 | -O- | 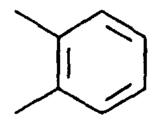 | 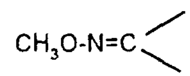 | $CH_2CH_2$ | |
| 128 | 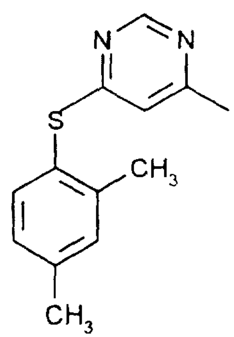 | -O- | 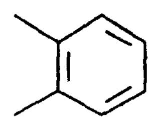 | 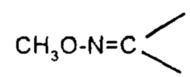 | $CH_2CH_2$ | |

| Bsp.-Nr. | Z | G | Ar | E | A | Physikal. Daten |
|---|---|---|---|---|---|---|
| 129 | 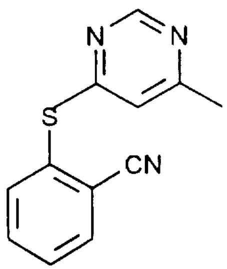 | -O- | 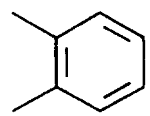 | 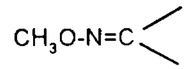 | $CH_2CH_2$ | |
| 130 | 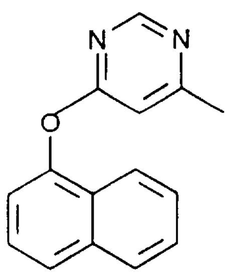 | -O- | 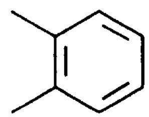 | 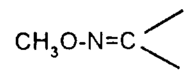 | $CH_2CH_2$ | amorph |
| 131 | 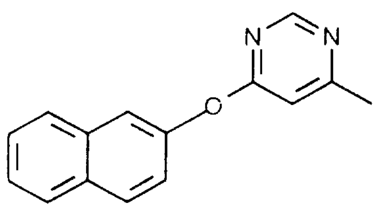 | -O- | 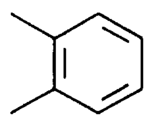 | 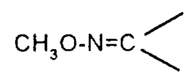 | $CH_2CH_2$ | |

| Bsp.-Nr. | Z | G | Ar | E | A | Physikal. Daten |
|---|---|---|---|---|---|---|
| 132 | 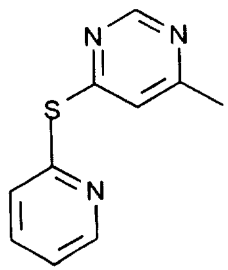 | -O- | 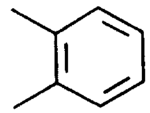 | 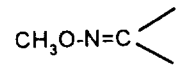 | $CH_2CH_2$ | amorph<br>$^1$H-NMR ($CDCl_3$)<br>$\delta$ = 3,85 (s,3H) |
| 133 | 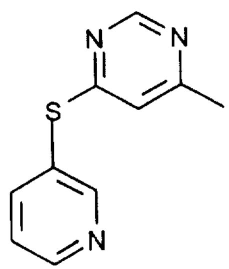 | -O- | 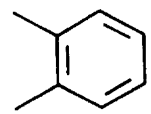 | 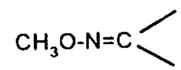 | $CH_2CH_2$ | |
| 134 | 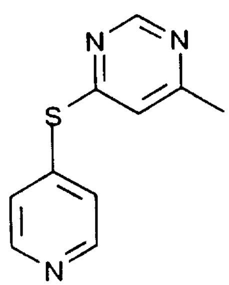 | -O- | 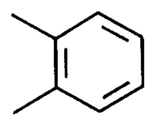 | 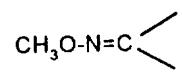 | $CH_2CH_2$ | |

| Bsp.-Nr. | Z | G | Ar | E | A | Physikal. Daten |
|---|---|---|---|---|---|---|
| 135 | 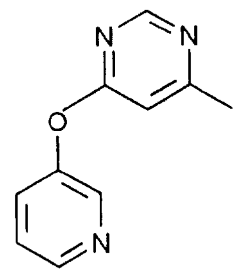 | -O- | 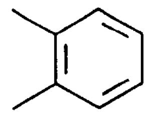 | 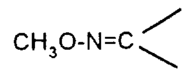 | $CH_2CH_2$ | amorph<br>$^1$HNMR ($CDCl_3$)<br>$\delta = 3.85$ (s, 3H) |
| 136 | 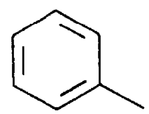 | $-CH_2O-$ | 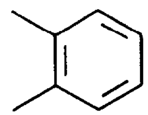 | 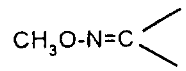 | $CH_2CH_2$ | |
| 137 | 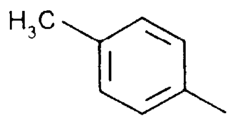 | $-CH_2O-$ | 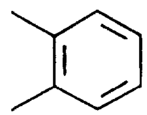 | 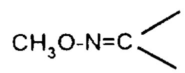 | $CH_2CH_2$ | |
| 138 | 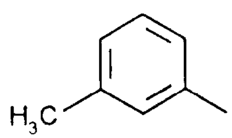 | $-CH_2O-$ | 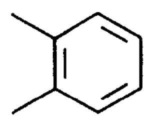 | 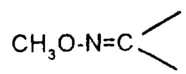 | $CH_2CH_2$ | |

| Bsp.-Nr. | Z | G | Ar | E | A | Physikal. Daten |
|---|---|---|---|---|---|---|
| 139 | 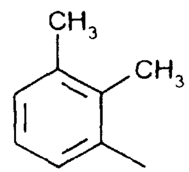 | $-CH_2O-$ | 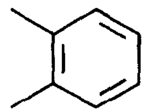 | 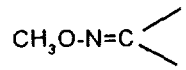 | $CH_2CH_2$ | Fp.: 146°C |
| 140 | 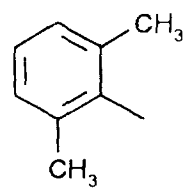 | $-CH_2O-$ | 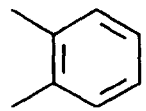 | 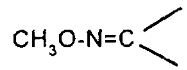 | $CH_2CH_2$ | |
| 141 | 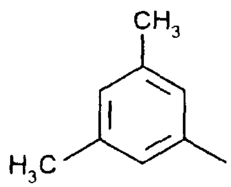 | $-CH_2O-$ | 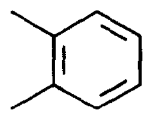 | 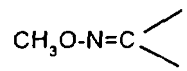 | $CH_2CH_2$ | |
| 142 | 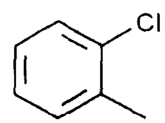 | $-CH_2O-$ | 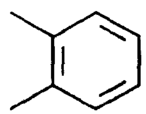 | 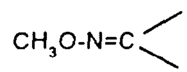 | $CH_2CH_2$ | |

| Bsp.-Nr. | Z | G | Ar | E | A | Physikal. Daten |
|---|---|---|---|---|---|---|
| 143 | 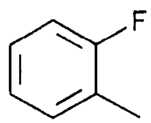 | $-CH_2O-$ | 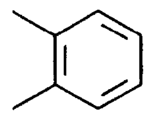 | 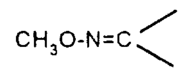 | $CH_2CH_2$ | |
| 144 | 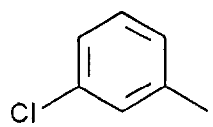 | $-CH_2O-$ | 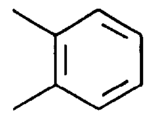 | 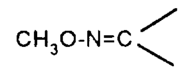 | $CH_2CH_2$ | |
| 145 | 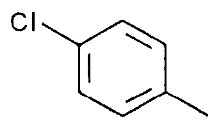 | $-CH_2O-$ | 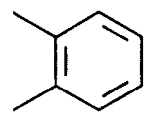 | 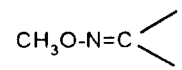 | $CH_2CH_2$ | |
| 146 | 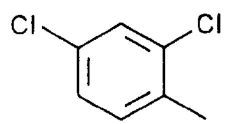 | $-CH_2O-$ | 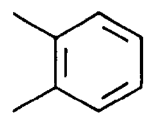 | 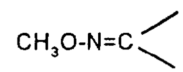 | $CH_2CH_2$ | |
| 147 | 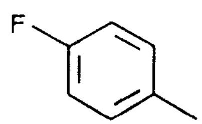 | $-CH_2O-$ | 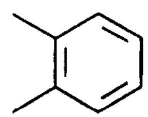 | 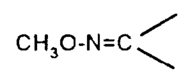 | $CH_2CH_2$ | |

| Bsp.-Nr. | Z | G | Ar | E | A | Physikal. Daten |
|---|---|---|---|---|---|---|
| 148 | 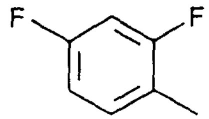 | $-CH_2O-$ | 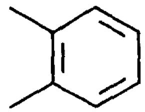 | 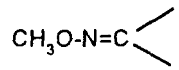 | $CH_2CH_2$ | |
| 149 | 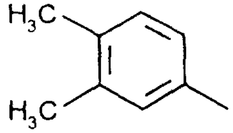 | $-CH_2O-$ | 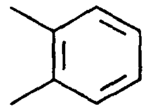 | 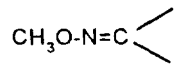 | $CH_2CH_2$ | |
| 150 | 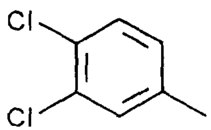 | $-CH_2O-$ | 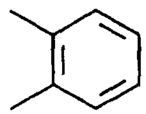 | 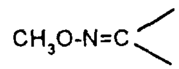 | $CH_2CH_2$ | |
| 151 | 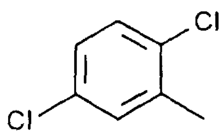 | $-CH_2O-$ | 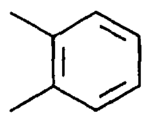 | 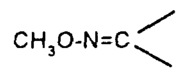 | $CH_2CH_2$ | |
| 152 | 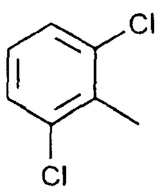 | $-CH_2O-$ | 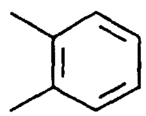 | 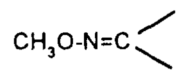 | $CH_2CH_2$ | |

| Bsp.-Nr. | Z | G | Ar | E | A | Physikal. Daten |
|---|---|---|---|---|---|---|
| 153 | 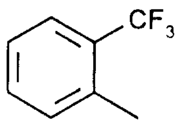 | $-CH_2O-$ | 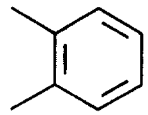 | 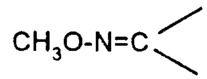 | $CH_2CH_2$ | |
| 154 | 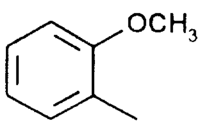 | $-CH_2O-$ | 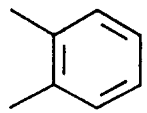 | 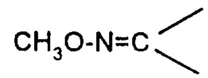 | $CH_2CH_2$ | |
| 155 | 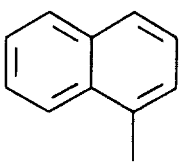 | $-CH_2O-$ | 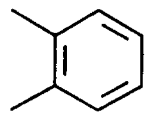 | 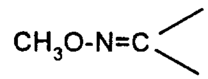 | $CH_2CH_2$ | |
| 156 | 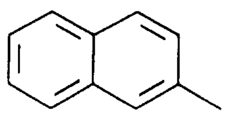 | $-CH_2O-$ | 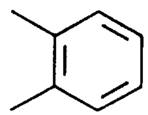 | 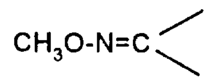 | $CH_2CH_2$ | |
| 157 | 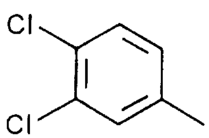 | - | 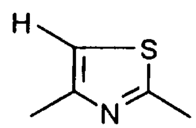 | 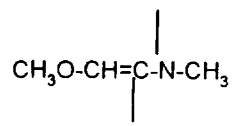 | $CH_2CH_2$ | |

| Bsp.-Nr. | Z | G | Ar | E | A | Physikal. Daten |
|---|---|---|---|---|---|---|
| 158 | 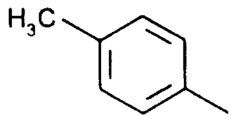 | - | 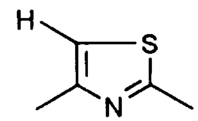 | 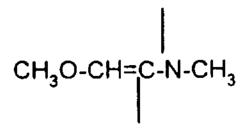 | $CH_2CH_2$ | |
| 159 | 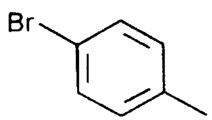 | - | 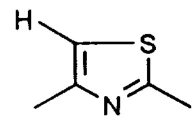 | 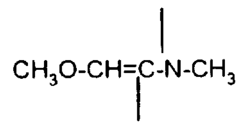 | $CH_2CH_2$ | |
| 160 | 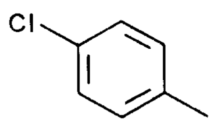 | - | 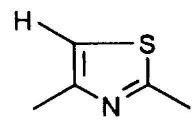 | 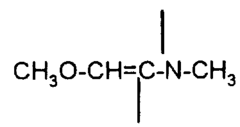 | $CH_2CH_2$ | |
| 161 | 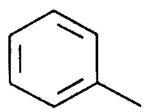 | - | 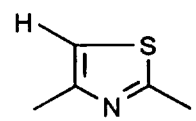 | 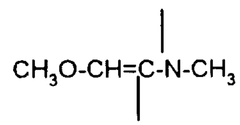 | $CH_2CH_2$ | |
| 162 | 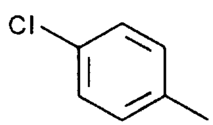 | - | 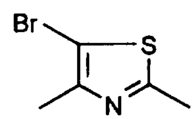 | 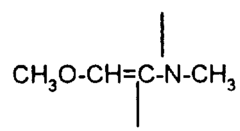 | $CH_2CH_2$ | |

| Bsp.-Nr. | Z | G | Ar | E | A | Physikal. Daten |
|---|---|---|---|---|---|---|
| 163 | 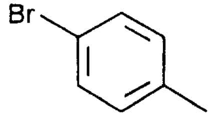 | - | 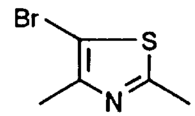 | $CH_3O-CH=C-N-CH_3$ | $CH_2CH_2$ | |
| 164 | 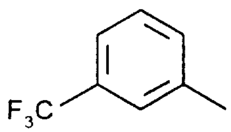 | - | 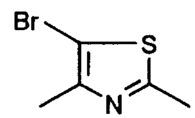 | $CH_3O-CH=C-N-CH_3$ | $CH_2CH_2$ | |
| 165 | 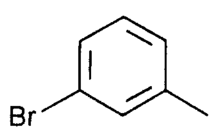 | - | 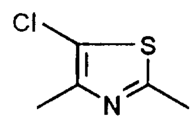 | $CH_3O-CH=C-N-CH_3$ | $CH_2CH_2$ | |
| 166 | 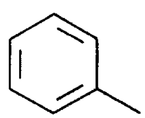 | - | 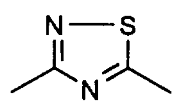 | $CH_3O-CH=C-N-CH_3$ | $CH_2CH_2$ | |

| Bsp.-Nr. | Z | G | Ar | E | A | Physikal. Daten |
|---|---|---|---|---|---|---|
| 167 | 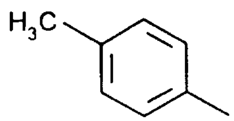 | - | 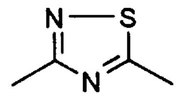 | $CH_3O-CH=C-N-CH_3$ | $CH_2CH_2$ | |
| 168 | 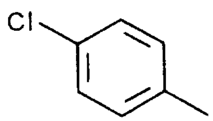 | - | 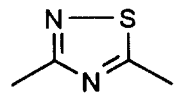 | $CH_3O-CH=C-N-CH_3$ | $CH_2CH_2$ | |
| 169 | 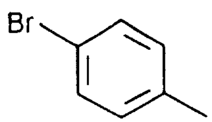 | - | 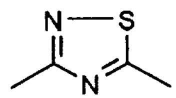 | $CH_3O-CH=C-N-CH_3$ | $CH_2CH_2$ | |
| 170 | 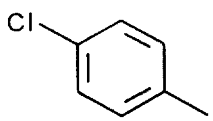 | - | 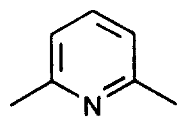 | $CH_3O-CH=C-N-CH_3$ | $CH_2CH_2$ | |
| 171 | 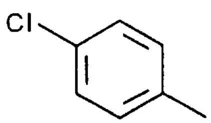 | - | 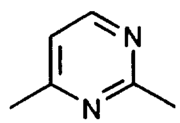 | $CH_3O-CH=C-N-CH_3$ | $CH_2CH_2$ | |

| Bsp.-Nr. | Z | G | Ar | E | A | Physikal. Daten |
|---|---|---|---|---|---|---|
| 172 | 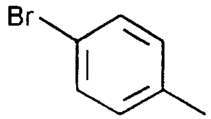 | - | 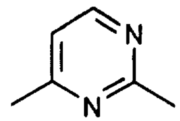 | 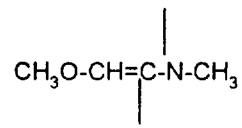 | $CH_2CH_2$ | |
| 173 | 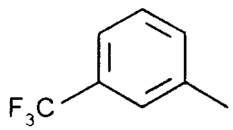 | - | 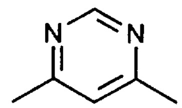 | 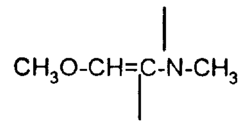 | $CH_2CH_2$ | |
| 174 | 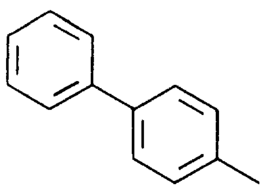 | - | 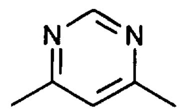 | 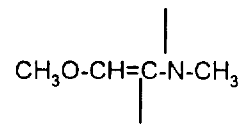 | $CH_2CH_2$ | |
| 175 | 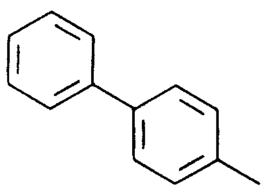 | - | 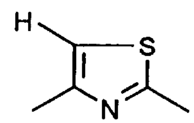 | 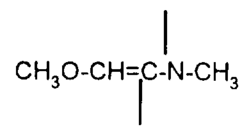 | $CH_2CH_2$ | |

| Bsp.-Nr. | Z | G | Ar | E | A | Physikal. Daten |
|---|---|---|---|---|---|---|
| 176 | 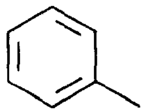 | - | 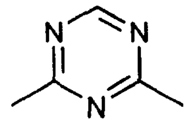 | 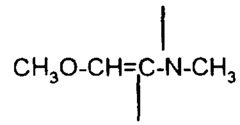 | $CH_2CH_2$ | |
| 177 | 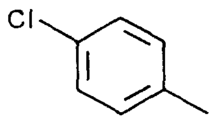 | - | 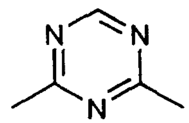 | 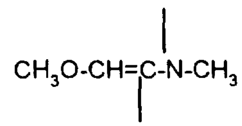 | $CH_2CH_2$ | |
| 178 | 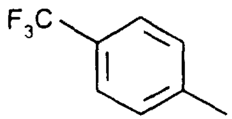 | - | 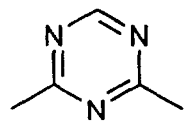 | 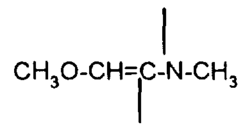 | $CH_2CH_2$ | |
| 179 | 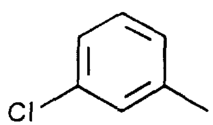 | - | 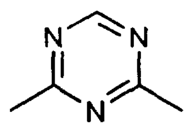 | 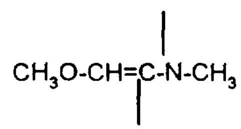 | $CH_2CH_2$ | |

| Bsp.-Nr. | Z | G | Ar | E | A | Physikal. Daten |
|---|---|---|---|---|---|---|
| 180 | 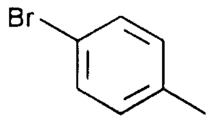 | - | 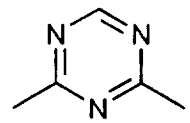 | $CH_3O-CH=C-N-CH_3$ | $CH_2CH_2$ | |
| 181 | 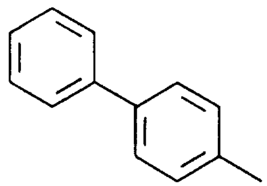 | - | 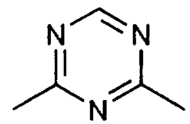 | $CH_3O-CH=C-N-CH_3$ | $CH_2CH_2$ | |
| 182 | 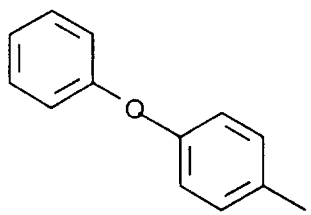 | - | 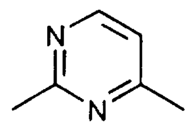 | $CH_3O-CH=C-N-CH_3$ | $CH_2CH_2$ | |
| 183 | 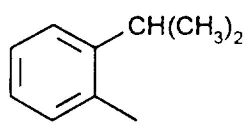 | $-OCH_2-$ | 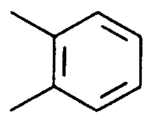 | $CH_3O-N=C$ | $CH_2CH_2$ | (amorph) $^1H$-NMR: 1,23; 1,26; 3,39-3,46; 4,0; 4,15; 4,48; 5,0; 6,8-7,6 ppm |

| Bsp.-Nr. | Z | G | Ar | E | A | Physikal. Daten |
|---|---|---|---|---|---|---|
| 184 | 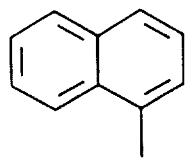 | $-OCH_2-$ | 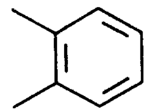 | 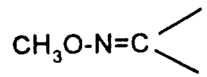 | $CH_2CH_2$ | Fp.: 115°C |
| 185 | 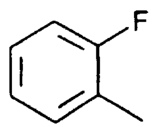 | $-OCH_2-$ | 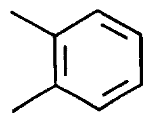 | 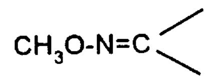 | $CH_2CH_2$ | Fp.: 103°C |
| 186 | 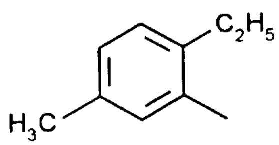 | $-OCH_2-$ | 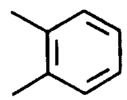 | 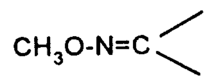 | $CH_2CH_2$ | Fp.: 62°C |
| 187 | 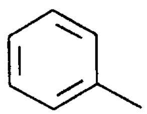 | - | 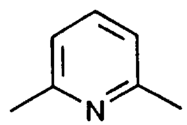 | 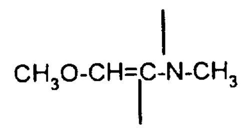 | $CH_2CH_2$ | |
| 188 | 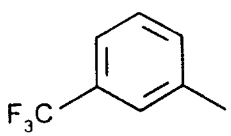 | - | 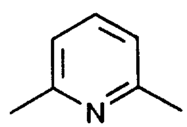 | 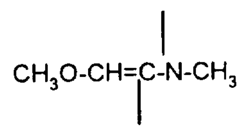 | $CH_2CH_2$ | |

| Bsp.-Nr. | Z | G | Ar | E | A | Physikal. Daten |
|---|---|---|---|---|---|---|
| 189 | 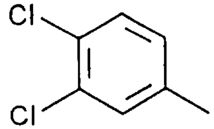 | - | 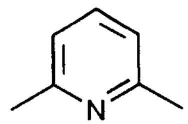 | $CH_3O\text{-}CH{=}C\text{-}N\text{-}CH_3$ | $CH_2CH_2$ | |
| 190 | 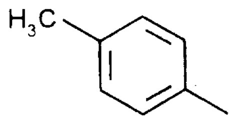 | - | 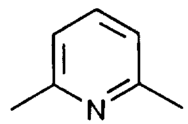 | $CH_3O\text{-}CH{=}C\text{-}N\text{-}CH_3$ | $CH_2CH_2$ | |
| 191 | 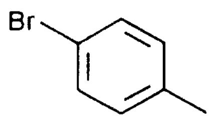 | - | 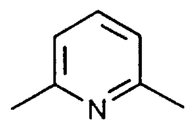 | $CH_3O\text{-}CH{=}C\text{-}N\text{-}CH_3$ | $CH_2CH_2$ | |
| 192 | 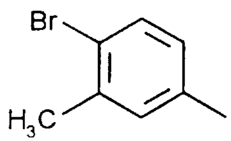 | - | 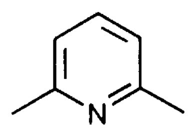 | $CH_3O\text{-}CH{=}C\text{-}N\text{-}CH_3$ | $CH_2CH_2$ | |

| Bsp.-Nr. | Z | G | Ar | E | A | Physikal. Daten |
|---|---|---|---|---|---|---|
| 193 | 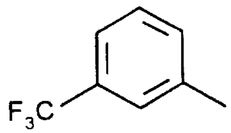 | -O- | 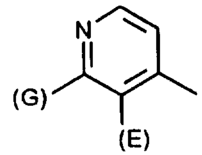 | 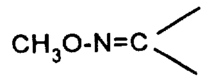 | $CH_2CH_2$ | Fp.: 118°C |
| 194 | 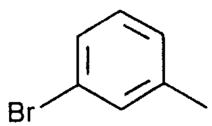 | -O- | 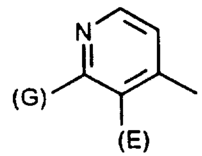 | 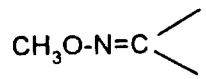 | $CH_2CH_2$ | amorph |
| 195 | 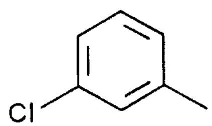 | -O- | 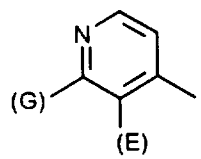 | 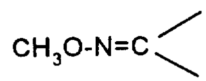 | $CH_2CH_2$ | |

| Bsp.-Nr. | Z | G | Ar | E | A | Physikal. Daten |
|---|---|---|---|---|---|---|
| 196 | 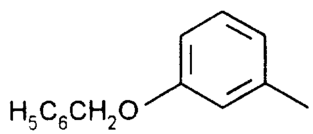 | -O- | 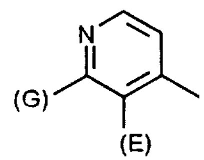 | 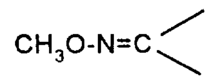 | $CH_2CH_2$ | |
| 197 | 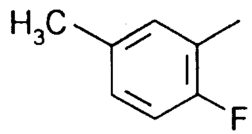 | $-OCH_2-$ | 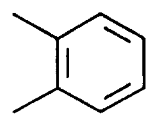 | 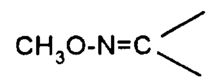 | $CH_2CH_2$ | |
| 198 | 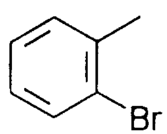 | $-OCH_2-$ | 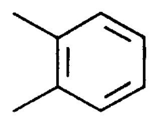 | 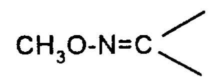 | $CH_2CH_2$ | $^1H$-NMR: 3,98; 4,16; 4,49; 5,09; 6,82-7,56 ppm |
| 199 | 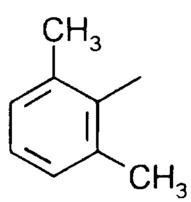 | $-OCH_2-$ | 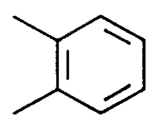 | 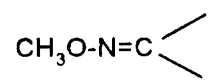 | $CH_2CH_2$ | $^1H$-NMR: 2,29; 3,95; 4,15; 4,47; 4,71; 6,92-7,82 ppm |

| Bsp.-Nr. | Z | G | Ar | E | A | Physikal. Daten |
|---|---|---|---|---|---|---|
| 200 | 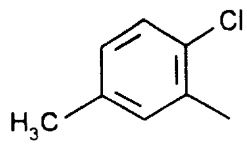 | $-OCH_2-$ | 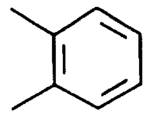 | 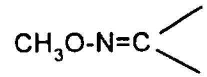 | $CH_2CH_2$ | $^1$H-NMR: 2,26; 3,99; 4,19; 4,5; 5,06; 6,67-7,62 ppm |
| 201 | 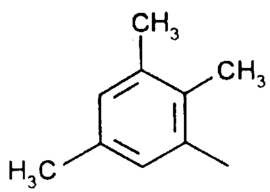 | $-OCH_2-$ | 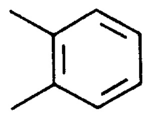 | 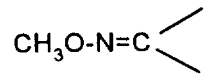 | $CH_2CH_2$ | $^1$H-NMR: 2,17-2,29; 3,98; 4,14; 4,48; 4,96; 6,5-7,6 ppm |
| 202 | 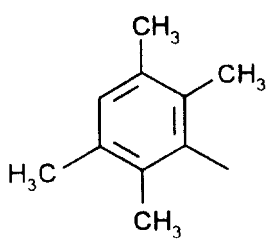 | $-OCH_2-$ | 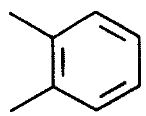 | 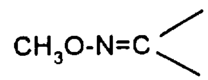 | $CH_2CH_2$ | |
| 203 | 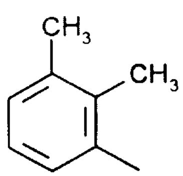 | $-OCH_2-$ | 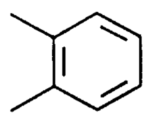 | 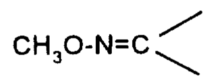 | $CH_2CH_2$ | $^1$H-NMR: 2,20; 2,28; 3,97; 4,15; 4,46; 4,98; 6,68-7,58 ppm |

| Bsp.-Nr. | Z | G | Ar | E | A | Physikal. Daten |
|---|---|---|---|---|---|---|
| 204 | 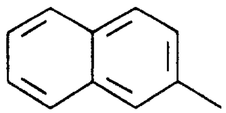 | $-OCH_2-$ | 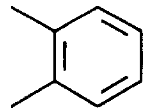 | 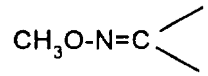 | $CH_2CH_2$ | |
| 205 | 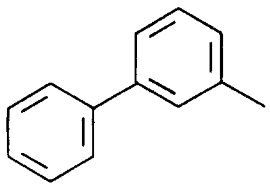 | $-OCH_2-$ | 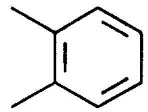 | 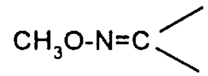 | $CH_2CH_2$ | |
| 206 | 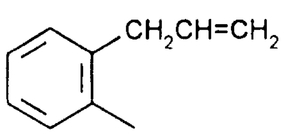 | $-OCH_2-$ | 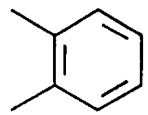 | 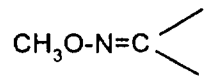 | $CH_2CH_2$ | |
| 207 | 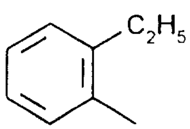 | $-OCH_2-$ | 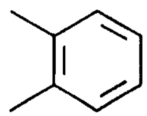 | 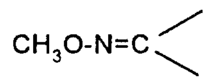 | $CH_2CH_2$ | |
| 208 | 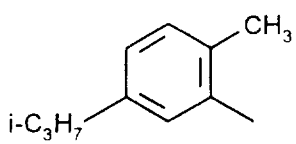 | $-OCH_2-$ | 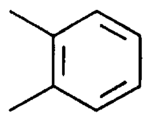 | 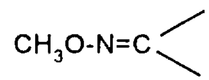 | $CH_2CH_2$ | $^1$H-NMR: 1,19; 1,21; 2,25; 2,82; 3,98; 4,15; 4,45; 5,0; 6,66-7,58 ppm |

| Bsp.-Nr. | Z | G | Ar | E | A | Physikal. Daten |
|---|---|---|---|---|---|---|
| 209 | 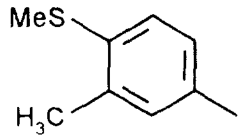 | $-OCH_2-$ | 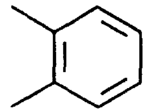 | 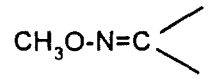 | $CH_2CH_2$ | $^1$H-NMR: 2,34; 2,38; 3,98; 4,15; 4,47; 4,97; 6,73-7,52 ppm |
| 210 | 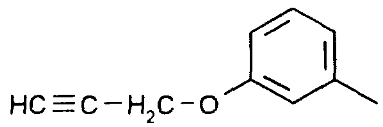 | $-OCH_2-$ | 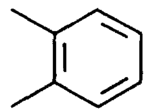 | 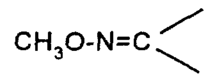 | $CH_2CH_2$ | |
| 211 | 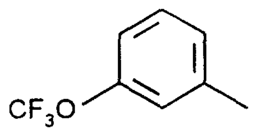 | $-OCH_2-$ | 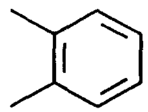 | 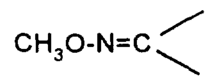 | $CH_2CH_2$ | |
| 212 | 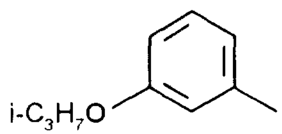 | $-OCH_2-$ | 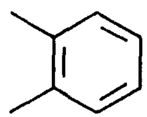 | 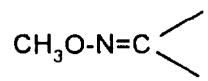 | $CH_2CH_2$ | |
| 213 | 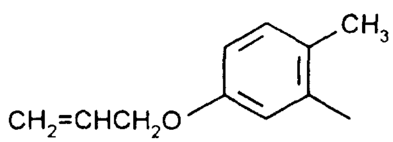 | $-OCH_2-$ | 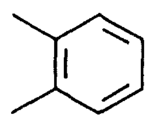 | 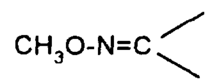 | $CH_2CH_2$ | |

| Bsp.-Nr. | Z | G | Ar | E | A | Physikal. Daten |
|---|---|---|---|---|---|---|
| 214 | 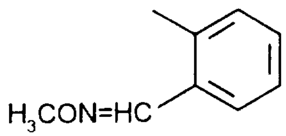 | $-OCH_2-$ | 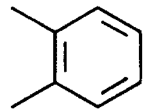 | 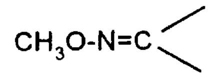 | $CH_2CH_2$ | amorph $^1$H-NMR: 3,97; 4,13; 4,45; 5,03; 6,86-8,50 ppm |
| 215 | 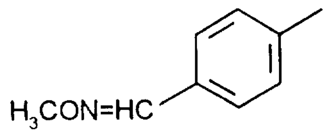 | $-OCH_2-$ | 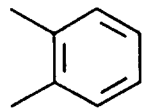 | 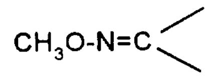 | $CH_2CH_2$ | $^1$H-NMR: 3,94; 3,97; 4,15; 4,47; 5,02; 6,90 - 8,0 ppm |
| 216 | 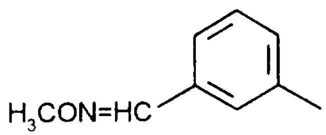 | $-OCH_2-$ | 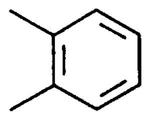 | 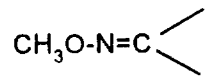 | $CH_2CH_2$ | $^1$H-NMR: 3,98; 4,15; 4,47; 5,01; 6,92-8,02 ppm |
| 217 | 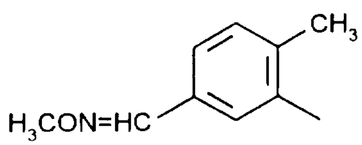 | $-OCH_2-$ | 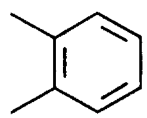 | 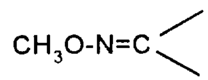 | $CH_2CH_2$ | |

| Bsp.-Nr. | Z | G | Ar | E | A | Physikal. Daten |
|---|---|---|---|---|---|---|
| 218 | 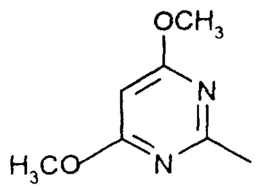 | O | 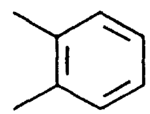 | 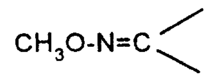 | $CH_2CH_2$ | Fp.: 133°C |
| 219 | 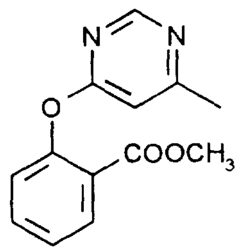 | O | 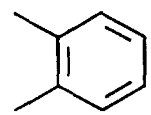 | 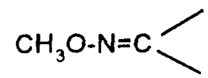 | $CH_2CH_2$ | amorph $^1H$-NMR ($CDCl_3$) $\delta$ = 3,15 (s,3H) |
| 220 | 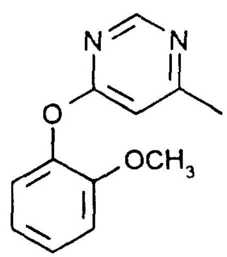 | O | 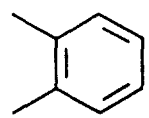 | 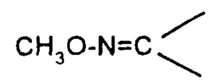 | $CH_2CH_2$ | amorph $\delta$ = 3,80 (s,3H) |

| Bsp.-Nr. | Z | G | Ar | E | A | Physikal. Daten |
| --- | --- | --- | --- | --- | --- | --- |
| 221 | 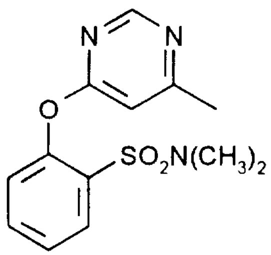 | O | 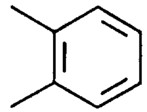 | 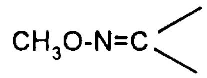 | $CH_2CH_2$ | Fp.: >200°C |
| 222 | 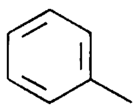 | $-OCH_2-$ | 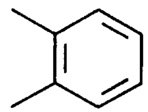 | 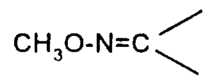 | $CH_2CH_2$ | amorph $^1$H-NMR: 3,97; 4,14; 4,46; 5,0; 6,91-7,54 ppm |
| 223 | 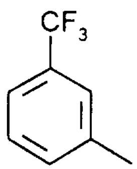 | $-OCH_2-$ | 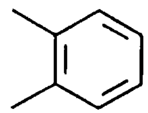 | 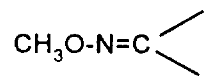 | $CH_2CH_2$ | amorph $^1$H-NMR: 3,97; 4,14; 4,48; 5,03; 7,08-7,51 ppm |
| 224 | 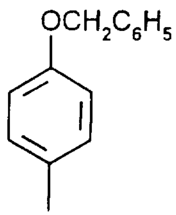 | O | 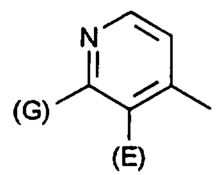 | 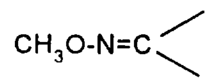 | $CH_2CH_2$ | amorph |

| Bsp.-Nr. | Z | G | Ar | E | A | Physikal. Daten |
| --- | --- | --- | --- | --- | --- | --- |
| 225 | 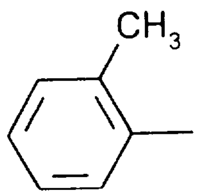 | $-OCH_2$ | 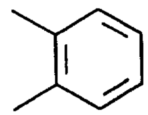 | 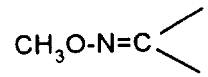 | $CH_2$ | $^1$H-NMR: 2,25; 4,0; 5,0; 5,86; 6,75-7,6 ppm |
| 226 | 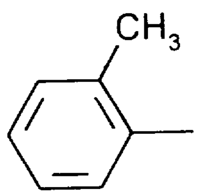 | $-OCH_2-$ | 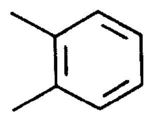 | 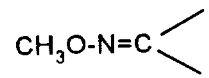 | $CH(CH_3)$ | |
| 227 | 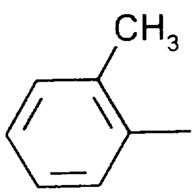 | $-OCH_2-$ | 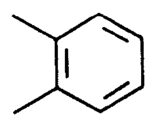 | 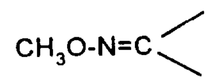 | $CH_2CH(CH_3)$ | $^1$H-NMR: 1,4; 2,3; 3,6-4,5; 5,0; 6,8-7,6 ppm |

Die in der Tabelle 1 als Beispiel 60 aufgeführte Verbindung kann beispielsweise wie folgt hergestellt werden:

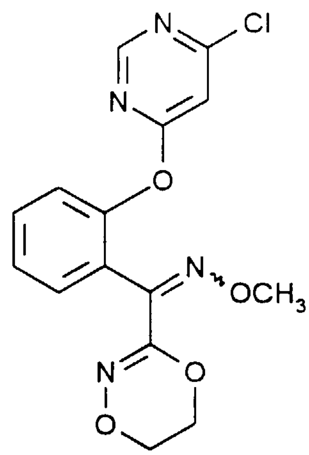

Zu einer Mischung aus 1,5 g (6 mmol) 3-[α-Methoximino-α-(2-hydroxy-phenyl)methyl]-5,6-dihydro-1,4,2-dioxazin, 0,9 g (6 mmol) 4,6-Dichlor-pyrimidin und 30 ml N,N-Dimethyl-formamid werden unter Eiskühlung 0,3 g (6 mmol) einer 6o%igen Natriumhydrid-Suspension in Weißöl gegeben. Nach Entfernen des Eisbads wird das Reaktionsgemisch 15 Stunden bei 20°C gerührt. Dann wird im Ölpumpenvakuum eingeengt, der Rückstand in Essigsäure-ethylester aufgenommen, mit Wasser gewaschen, mit Natriumsulfat getrocknet und filtriert. Vom Filtrat wird das Lösungsmittel im Wasserstrahlvakuum sorgfältig abdestilliert.

Man erhält 1,9 g (86% der Theorie) 3-{α-Methoximino-α-[2-(6-chlor-pyrimidin-4-yloxy)-phenyl]-methyl}-5,6-dihydro-1,4,2-dioxazin als öligen Rückstand.

Die in der Tabelle 1 als Beispiel 61 aufgeführte Verbindung kann beispielsweise wie folgt hergestellt werden:

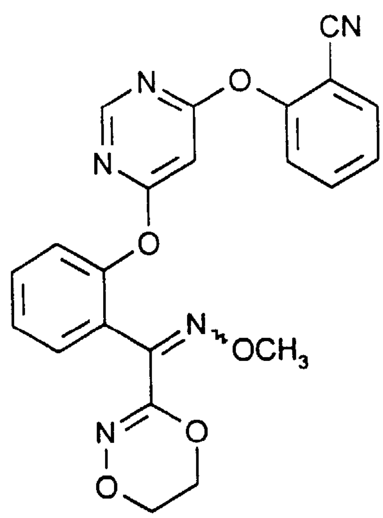

Eine Mischung aus 0,3 g (0,9 mmol) 3-{α-Methoximino-α-[2-(6-chlor-pyrimidin-4-yl-oxy)-phenyl]-methyl}-5,6-dihydro-1,4,2-dioxazin, 0,1 g (0,9 mmol) 2-Hydroxybenzonitril, 0,1 g (0,9 mmol) Kaliumcarbonat, einer Spatelspitze Kupfer (I)-chlorid und 5 ml N,N-Dimethyl-formamid wird bei 100°C 15 Stunden gerührt. Dann wird im Ölpumpenvakuum eingeengt, der Rückstand in Essigsäure-ethylester aufgenommen, mit Wasser gewaschen, mit Natriumsulfat getrocknet und filtriert. Das Filtrat wird eingeengt und der Rückstand durch Säulenchromatographie an Kieselgel (mit Hexan/Aceton, Vol.: 7:3) gereinigt.

Man erhält 0,3 g (81% der Theorie) 3-{α-Methoximino-α-[2-(6-(2-cyano-phenoxy)pyrimidin-4-yl-oxy)-phenyl]-methyl}-5,6-dihydro-1,4,2-dioxazin vom Schmelzpunkt 82°C.

Die in der Tabelle 1 als Beispiel 58 aufgeführte Verbindung kann beispielsweise wie folgt hergestellt werden:

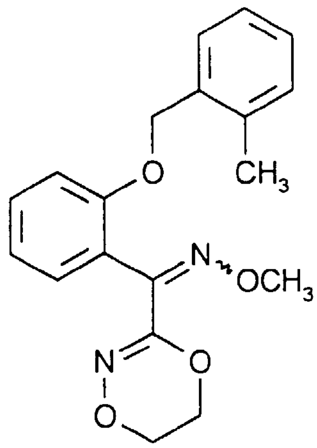

Eine Mischung aus 0,5 g (2 mmol) 3-[α-Methoximino-α-(2-hydroxy-phenyl)-methyl]-5,6-dihydro-1,4,2-dioxazin, 0,3 g (2,2 mmol) 2-Methyl-benzylchlorid, 0,4 g (2,5 mmol) Kaliumcarbonat und 10 ml Acetonitril wird 15 Stunden unter Rückfluß erhitzt. Dann wird eingeengt, der Rückstand in Methylenchlorid aufgenommen, mit Wasser gewaschen, mit Natriumsulfat getrocknet und filtriert. Vom Filtrat wird das Lösungsmittel im Wasserstrahlvakuum sorgfältig abdestilliert.

Man erhält 0,4 g (59% der Theorie) 3-{α-Methoximino-α-[2-(2-methyl-benzyloxy)phenyl]-methyl}-5,6-dihydro-1,4,2-dioxazin vom Schmelzpunkt 142°C.

Die gemäß Beispiel 1 erhältliche Verbindung kann beispielsweise auch wie folgt hergestellt werden:

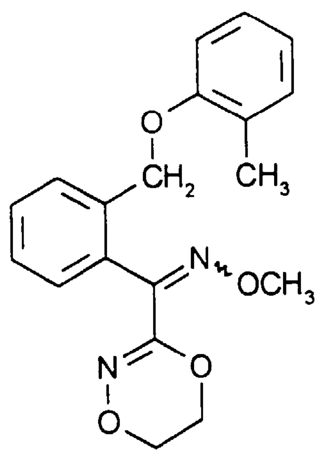

0,75 g (2,4 mMol) 3-[α-Methoximino-α-(2-brommethyl-phenyl)-methyl]-5,6-dihydro-1,4,2-dioxazin und 0,70 g (6,4 mMol) 2-Methyl-phenol werden in 15 ml Dimethylformamid gelöst und nach Abkühlen der Mischung auf -10°C werden 0,21 g (7,0 mMol) Natriumhydrid (80%ig) langsam dazugegeben. Nach Entfernen des Kühlbades wird das Reaktionsgemisch 14 Stunden bei maximal 25°C gerührt und anschließend auf etwa das doppelte Volumen Wasser gegossen. Nach Schütteln mit Essigsäureethylester wird die organische Phase abgetrennt, mit 2N-Natronlauge gewaschen, mit Natriumsulfat getrocknet und filtriert. Vom Filtrat wird das Lösungsmittel unter vermindertem Druck sorgfältig abdestilliert.

Man erhält 0,40 g (49% der Theorie) 3-{α-Methoximino-α-[2-(2-methyl-phenoxy-methyl)-phenyl]-methyl}-5,6-dihydro-1,4,2-dioxazin (Brechungsindex: $n_D^{20}$ = 1,5705).

Die in der Tabelle 1 als Beispiel 19 aufgeführte Verbindung kann beispielsweise wie folgt hergestellt werden:

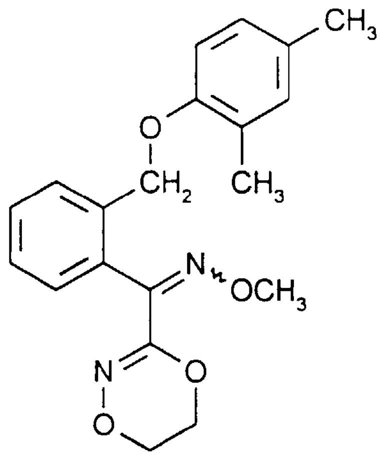

0,20 g (0,56 mMol) N-(2-Hydroxy-ethoxy)-α-methoximino-α-[2-(2,4-dimethyl-phenoxy-methyl)-phenyl]-acetamid werden in 3 ml Chloroform gelöst und bei 0°C mit 0,25 g (1,76 mMol) Phosphor(V)-oxid versetzt. Das Reaktionsgemisch wird eine Stunde bei 20°C und dann 4 Stunden unter Rückfluß gerührt, anschließend auf etwa das doppelte Volumen Wasser gegossen und geschüttelt. Nach Abtrennen der organischen Phase wird die wäßrige Phase dreimal mit Chloroform nachextrahiert. Die vereinigten organischen Extrakte werden mit Magnesiumsulfat getrocknet, dann eingeengt und durch Säulenchromatographie (Kieselgel; Toluol/Aceton, 10:1) gereinigt.

Man erhält 84 mg (42% der Theorie) 3-{α-Methoximino-α-[2-(2,4-dimethyl-phenoxy-methyl)-phenyl]-methyl}-5,6-dihydro-1,4,2-dioxazin.

$^1$H-NMR ($D_6$-DMSO, δ): 4,87; 3,84; 4,38; 4,10 ppm.

Ausgangsstoffe der Formel (IV) :

Beispiel (IV-1)

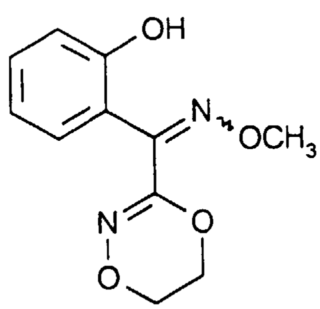

9,0 g (28 mmol) 3-[α-Methoximino-α-(2-tetrahydropyran-2-yl-oxy)-benzyl]-5,6-dihydro-1,4,2-dioxazin werden mit 1,8 g Ionenaustauscher "Lewatit SPC 108" in 90 ml Methanol 15 Stunden bei 20°C gerührt. Die Mischung wird dann im Wasserstrahlvakuum eingeengt, der Rückstand in Methylenchlorid aufgenommen und filtriert. Das Filtrat wird im Wasserstrahlvakuum eingeengt und der Rückstand durch Säulenchromatographie an Kieselgel (mit Hexan/Aceton, Vol.: 7:3) gereinigt.

Man erhält als 1. Fraktion 0,6 g (9% der Theorie) Z-{3-[α-Methoximino-α-(2-hydroxy-phenyl)-methyl]-5,6-dihydro-1,4,2-dioxazin} als amorphes Produkt und als 2. Fraktion 3,3 g (50% der Theorie) E-{3-[α-Methoximino-α-(2-hydroxy-phenyl)methyl]-5,6-dihydro-1,4,2-dioxazin} vom Schmelzpunkt 153°C.

Ausgangsstoffe der Formel (IX):

Beispiel (IX-1)

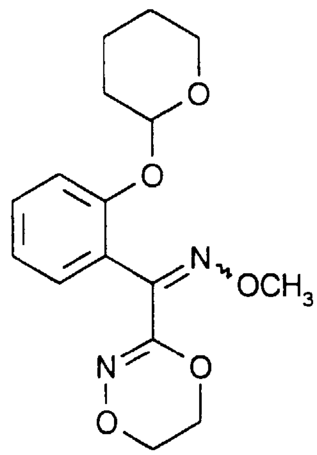

Zu 6,8 g (98 mmol) Hydroxylamin-Hydrochlorid in 290 ml Methanol werden 13,9 g (211 mmol) einer 85%igen wässrigen Kaliumhydroxid-Lösung und 17 g (58 mmol) α-Methoximino-α-(2-tetrahydropyran-2-yl-oxy-phenyl)-essigsäure-methylester gegeben und die Mischung wird eine Stunde bei 40°C gerührt. Dann werden 7,7 g (56 mmol) Kaliumcarbonat dazu gegeben und 42,5 g (226 mmol) 1,2-Dibrom-ethan zugetropft. Die Mischung wird dann 15 Stunden unter Rückfluß erhitzt und anschließend im Wasserstrahlvakuum eingeengt. Der Rückstand wird in Methylenchlorid aufgenommen, mit Wasser gewaschen, mit Natriumsulfat getrocknet und filtriert. Das Filtrat wird eingeengt und der Rückstand durch Säulenchromatographie an Kieselgel (mit Hexan/Aceton, Vol.: 7/3) gereinigt.

Man erhält 9,0 g (49% der Theorie) 3-[α-Methoximino-α-(2-tetrahydropyran-2-yl-oxy)-benzyl]-5,6-dihydro-1,4,2-dioxazin als öliges Produkt.

Ausgangsstoffe der Formel (X):

Beispiel (X-1)

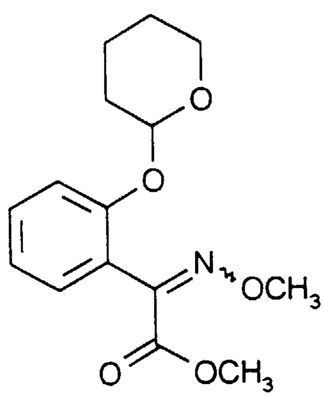

203 g (1,81 mol) Kalium-t-butylat werden in 2 Liter t-Butanol vorgelegt und zu dieser Lösung werden 564 g (4,93 mol) t-Butyl-nitrit und 411 g (1,64 mol) 2-Tetrahydropyranyloxy-phenylessigsäure-methylester - gelöst in 500 ml t-Butanol - tropfenweise gegeben. Nach 90 Minuten werden 350 g (2,47 mol) Methyliodid zugetropft und die Mischung wird 15 Stunden bei 20°C gerührt. Dann wird im Wasserstrahlvakuum eingeengt, der Rückstand in Methyl-t-butylether aufgenommen mit Natriumsulfat getrocknet und filtriert. Der Rückstand wird durch Digerieren mit Diethylether zur Kristallisation gebracht und das Produkt wird durch Absaugen isoliert.

Man erhält 69,3 g (15% der Theorie) α-Methoximino-α-(2-tetrahydropyran-2-yl-oxy-phenyl)-essigsäure-methylester vom Schmelzpunkt 79°C.

Ausgangsstoffe der Formel (XI):

Beispiel (XI-1)

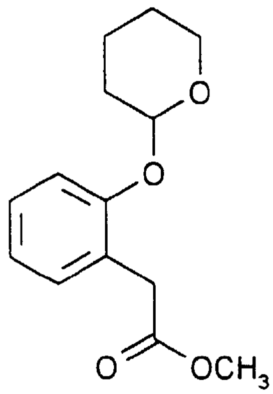

Eine Mischung aus 500 g (3,0 mol) 2-Hydroxy-phenylessigsäure-methylester, 506 g (6,0 mol) 3,4-Dihydro-pyran, einer Spatelspitze p-Toluol-sulfonsäure und 2,5 Liter Tetrahydrofuran wird 15 Stunden bei 20°C gerührt, dann mit eiskalter 10%iger wässriger Kaliumhydroxid-Lösung verrührt, mit Natriumsulfat versetzt und filtriert. Vom Filtrat wird das Lösungsmittel im Wasserstrahlvakuum sorgfältig abdestilliert.

Man erhält 698 g (99% der Theorie) 2-Tetrahydropyranyloxy-phenylessigsäuremethylester als öligen Rückstand.

Ausgangsstoffe der Formel (VI):

Beispiel (VI-1)

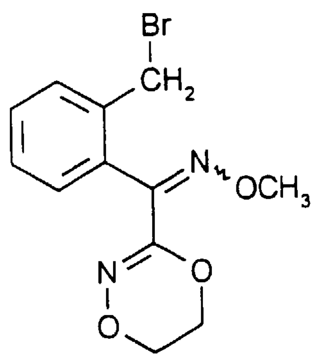

0,50 g (2,13 mMol) 3-[α-Methoximino-α-(2-methyl-phenyl)-methyl]-5,6-dihydro-1,4,2-dioxazin und 0,57 g (3,2 mMol) N-Brom-succinimid werden in 10 ml Tetrachlormethan vorgelegt und nach Zugabe von 200 mg Azoisobutyronitril 4 Stunden unter Rückfluß erhitzt. Nach Zugabe von weiteren 0,57 g (3,2 mMol) N-Brom-succinimid wird das Gemisch eine weitere Stunde unter Rückfluß erhitzt. Anschließend wird abgekühlt, filtriert, das Filtrat eingeengt und der Rückstand chromatographiert (Kieselgel; Toluol/Aceton, 10:1).

Man erhält 20 mg (30% der Theorie) 3-[α-Methoximino-α-(2-brommethyl-phenyl)methyl]-5,6-dihydro-1,4,2-dioxazin.

$^1$H-NMR ($CDCl_3$, δ): 4,4 ppm.

Ausgangsstoffe der Formel (XIII):

Beispiel (XIII-1)

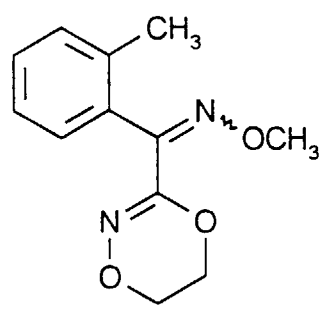

19,6 g (0,283 Mol) Hydroxylamin-hydrochlorid werden in 150 ml Methanol vorgelegt und langsam mit einer Lösung von 36,9 g (0,565 Mol) Kaliumhydroxid (86%ig) in 150 ml Methanol versetzt. Dann werden 30 g (0,145 Mol) α-Methoximino-α-(2-methyl-phenyl)-essigsäure-methylester portionsweise dazugegeben. Das Gemisch wird 3 Stunden bei 50°C gerührt. Anschließend werden bei 20°C 20 g (0,145 Mol) Kaliumcarbonat und 122 g (0,65 Mol) 1,2-Dibrom-ethan dazugegeben und die Reaktionsmischung 17 Stunden bei 65°C gerührt. Nach dem Abkühlen wird abgesaugt, das Filtrat eingeengt und der Rückstand chromatographiert (Kieselgel; Toluol/Aceton, 15:1).

Man erhält 15,2 g (45% der Theorie) 3-[α-Methoximino-α-(2-methyl-phenyl)-methyl]-5,6-dihydro-1,4,2-dioxazin.

$^1$H-NMR ($CDCl_3$, δ): 2,2 ppm.

Ausgangsstoffe der Formel (XIV):

Beispiel (XIV-1)

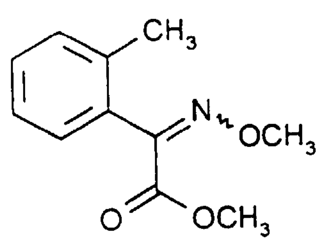

187,5 g (1,673 Mol) Kalium-t-butylat werden in 1875 ml t-Butanol gelöst. Hierzu werden 471,5 g (4,57 Mol) t-Butylnitrit und 250 g (1,525 Mol) 2-Methyl-phenylessigsäure-methylester - gelöst in 500 ml t-Butanol - so eindosiert, daß die Innentemperatur 50°C nicht übersteigt. Das Gemisch wird 90 Minuten bei 20°C bis 30°C gerührt. Dann werden 326,5 g (2,3 Mol) Methyliodid tropfenweise dazugegeben und die Reaktionsmischung wird 14 Stunden bei 20°C gerührt. Anschließend wird das Lösungsmittel im Wasserstrahlvakuum abdestilliert, der Rückstand in 2 Liter Wasser aufgenommen und dreimal mit Essigsäureethylester extrahiert. Die vereinigten organischen Phasen werden über Natriumsulfat getrocknet und filtriert. Das Filtrat wird eingeengt, der Rückstand in 250 ml Isopropanol aufgenommen und bei Rückfluß bis zur Trübung mit Wasser versetzt.

Nach Abkühlen auf 0°C und 60 Minuten Rühren wird das kristallin angefallene Produkt durch Absaugen isoliert.

Man erhält 84,5 g (27% der Theorie) α-Methoximino-α-(2-methyl-phenyl)-essigsäure-methylester vom Schmelzpunkt 53°C.

$^1$H-NMR ($CDCl_3$, δ): 2,19 ppm.

Ausgangsstoffe der Formel (VIII):

Beispiel (VIII-1)

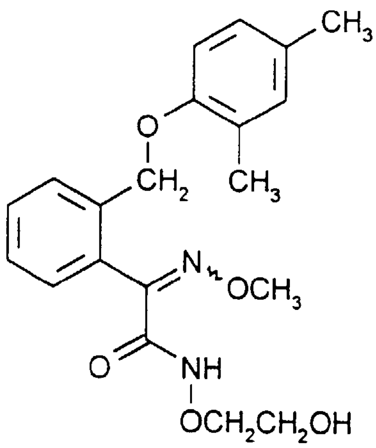

0,8 g (2,36 mMol) α-Methoximino-α-[2-(2,4-dimethylphenoxy-methyl)-phenyl]-essigsäurechlorid werden in 10 ml Tetrahydrofuran gelöst und mit 0,26 g (2,6 mMol) Triethylamin versetzt. Dann werden bei 0°C 0,25 g (2,6 mMol) O-(2-Hydroxy-ethyl)-hydroxylamin, gelöst in 10 ml Tetrahydrofuran zugetropft. Die Reaktionsmischung wird 2 Stunden bei 20°C gerührt, dann auf Wasser gegossen und mit Essigsäureethylester extrahiert. Die Extraktionslösung wird mit Magnesiumsulfat getrocknet, eingeengt und chromatographiert (Kieselgel; Toluol/Aceton, 10:1).

Man erhält 0,4 g (50% der Theorie) N-(2-Hydroxy-ethoxy)-α-methoximino-α-[2-(2,4-dimethyl-phenoxy-methyl)-phenyl]-acetamid.

$^1$H-NMR ($CDCl_3$, δ): 3,65; 3,90; 9,15 ppm.

Ausgangsstoffe der Formel (XV):

Beispiel (XV-1)

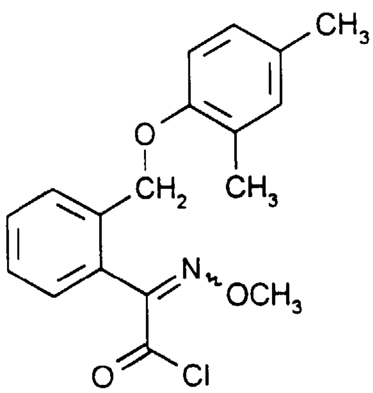

0,93 g (2,95 mMol) α-Methoximino-α-[2-(2,4-dimethyl-phenoxy-methyl)-phenyl]-essigsäure werden mit 4,0 g (2,9 mMol) Thionylchlorid und 50 mg Dimethylformamid vermischt und die Mischung wird 30 Minuten unter Rückfluß gerührt. Dann werden die leichter flüchtigen Komponenten unter vermindertem Druck sorgfältig abdestilliert.

Man erhält 0,95 g α-Methoximino-α-[2-(2,4-dimethyl-phenoxy-methyl)-phenyl]-essigsäurechlorid als öligen Rückstand.

Beispiel (XV-2)

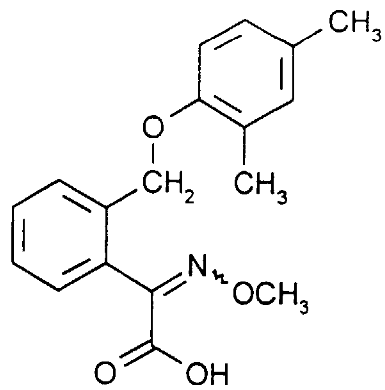

2,0 g (6,1 mMol) α-Methoximino-α-{2-(2,4-dimethyl-phenoxy-methyl)-phenyl)essigsäure-methylester werden in 20 ml Isopropanol gelöst und mit 30 ml 1N-Natronlauge versetzt. Die Mischung wird 14 Stunden bei 40°C gerührt und dann auf Wasser gegossen. Dann wird mit 2N-Salzsäure auf pH 6 eingestellt und das kristallin angefallene Produkt durch Absaugen isoliert.

Man erhält 1,5 g (78% der Theorie) α-Methoximino-α-[2-(2,4-dimethyl-phenoxy-methyl)-phenyl]-essigsäure.

$^1$H-NMR ($CDCl_3$, δ): 3,9; 4,85 ppm.

Anwendungsbeispiele

Beispiel A

**Pyrenophora teres-Test (Gerste) / protektiv**

| | |
|---|---|
| Lösungsmittel | 10 Gewichtsteile N-Methyl-pyrrolidon |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Abtrocknen des Spritzbelages werden die Pflanzen mit einer Konidiensuspension von Pyrenophora teres besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt.

7 Tage nach der Inokulation erfolgt die Auswertung.

In diesem Test zeigte beispielsweise die Verbindung gemäß Herstellungsbeispiel 1 bei einer Aufwandmenge von 400 g/ha einen Wirkungsgrad von 100%.

Beispiel B

**Phytophthora-Test (Tomate)** / systemisch

| | |
|---|---|
| Lösungsmittel | 4,7 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf systemische Eigenschaften wird die Wirkstoffzubereitung auf Einheitserde gegossen, in der sich junge versuchsbereite Pflanzen befinden. 3 Tage nach der Behandlung werden die Pflanzen mit einer wäßrigen Sporensuspension von Phytophthora infestans inokuliert.

Die Pflanzen werden in einer Inkubationskabine mit 100% relativer Luftfeuchtigkeit und ca. 20°C aufgestellt.

3 Tage nach der Inokulation erfolgt die Auswertung.

In diesem Test zeigte beispielsweise die Verbindung gemäß Herstellungsbeispiel 1 bei einer Wirkstoffkonzentration von 100 ppm einen Wirkungsgrad von 58%.

Beispiel C

**Pyricularia-Test (Reis)** / systemisch

| | |
|---|---|
| Lösungsmittel | 12,5 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100% bis zur Auswertung.

4 Tage nach der Inokulation erfolgt die Auswertung des Krankheitsbefalls.

In diesem Test zeigte beispielsweise die Verbindung gemäß Herstellungsbeispiel 1 bei einer Aufwandmenge von 100 mg/100 ml einen Wirkungsgrad von 80%.

**Patentansprüche**

**1.** Azadioxacycloalkene der allgemeinen Formel (I),

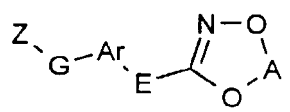

(I)

in welcher

A für gegebenenfalls substituiertes Alkandiyl (Alkylen) steht,

Ar für jeweils gegebenenfalls substituiertes Arylen oder Heteroarylen steht,

E für eine 1-Alken-1,1-diyl-Gruppierung steht, die in 2-Position einen Rest $R^1$ enthält, oder für eine 2-Aza-1-alken-1,1-diyl-Gruppierung steht, die in 2-Position einen Rest $R^2$ enthält, oder für eine 3-Oxa- oder 3-Thia-1-propen-2,3-diyl-Gruppierung steht, die in 1-Position einen Rest $R^1$ enthält, oder für eine 3-Aza-1-propen-2,3-diyl-Gruppierung steht, die in 3-Position einen Rest R und in 1-Position einen Rest $R^1$ enthält, oder für eine 1-Aza-1-propen-2,3-diyl-Gruppierung steht, die in 1-Position einen Rest $R^2$ enthält, oder für eine 3-Oxa- oder 3-Thia-1-aza-propen-2,3-diyl-Gruppierung steht, die in 1-Position einen Rest $R^2$ enthält, oder für eine 1,3-Diaza-1-propen-2,3-diyl-Gruppierung steht, die in 3-Position einen Rest R und in 1-Position einen Rest $R^2$ enthält, oder für eine gegebenenfalls substituierte Imino-Gruppierung ("Azamethylen", N-$R^3$) steht, wobei

R für Alkyl steht,

$R^1$ für Wasserstoff, Halogen, Cyano oder für jeweils gegebenenfalls substituiertes Alkyl, Alkoxy, Alkylthio,

Alkylamino oder Dialkylamino steht,

$R^2$ für Wasserstoff, Amino, Cyano oder für jeweils gegebenenfalls substituiertes Alkyl, Alkoxy, Alkylamino oder Dialkylamino steht, und

$R^3$ für Wasserstoff, Cyano oder für jeweils gegebenenfalls substituiertes Alkyl, Alkenyl, Alkinyl, Cycloalkyl oder Cycloalkylalkyl steht,

G für eine Einfachbindung, für Sauerstoff, für jeweils gegebenenfalls durch Halogen, Hydroxy, Alkyl, Halogenalkyl oder Cycloalkyl substituiertes Alkandiyl, Alkendiyl, Oxaalkendiyl, Alkindiyl oder eine der nachstehenden Gruppierungen steht
-Q-CQ-, -CQ-Q-, $-CH_2$-Q-; -Q-$CH_2$-, -CQ-Q-$CH_2$-, $CH_2$-Q-CQ-, -Q-CQ-$CH_2$-, -Q-CQ-Q-$CH_2$-, -N=N-, -S(O)$_n$-, -$CH_2$-S(O)$_n$-, -CQ-, -S(O)$_n$-$CH_2$-, -C($R^4$)=N-O-, -C($R^4$)=N-O-$CH_2$-, -N($R^5$)-, -CQ-N($R^5$)-, -N($R^5$)-CQ-, -Q-CQ-N($R^5$)-, -N=C($R^4$)-Q-$CH_2$-, -$CH_2$-O-N=C($R^4$)-, -N($R^5$)-CQ-Q-, -CQ-N($R^5$)-CQ-Q-, -N($R^5$)-CQ-Q-$CH_2$-, -CQ-$CH_2$- oder -N=N-C($R^4$)=N-O-,
wobei

n für die Zahlen 0, 1 oder 2 steht,

Q für Sauerstoff oder Schwefel steht,

$R^4$ für Wasserstoff, Cyano oder jeweils gegebenenfalls substituiertes Alkyl, Alkoxy, Alkylthio, Alkylamino, Dialkylamino oder Cycloalkyl steht, und

$R^5$ für Wasserstoff, Hydroxy, Cyano oder jeweils gegebenenfalls substituiertes Alkyl, Alkoxy oder Cycloalkyl steht, und

Z für jeweils gegebenenfalls substituiertes Alkyl, Alkenyl, Alkinyl, Cycloalkyl, Aryl oder Heterocyclyl steht.

**2.** Verbindungen der Formel (I) nach Anspruch 1, in welcher

A für gegebenenfalls durch Halogen oder durch Alkyl oder Halogenalkyl mit jeweils 1 bis 4 Kohlenstoffatomen substituiertes Alkandiyl mit 1 bis 3 Kohlenstoffatomen steht,

Ar für jeweils gegebenenfalls substituiertes Phenylen oder Naphthylen oder für Heteroarylen mit 5 oder 6 Ringgliedern, von denen mindestens eines für Sauerstoff, Schwefel oder Stickstoff steht und gegebenenfalls ein oder zwei weitere für Stickstoff stehen, steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl, Alkenyloxy oder Alkinyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,

E für eine der nachstehenden Gruppierungen steht

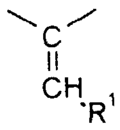 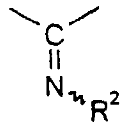 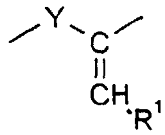 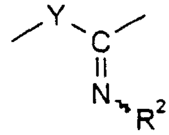 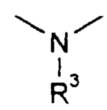

worin

Y für Sauerstoff, Schwefel, Methylen ($CH_2$) oder Alkylimino (N-R) steht,

R für Alkyl mit 1 bis 6 Kohlenstoffatomen steht,

$R^1$ für Wasserstoff, Halogen, Cyano oder für jeweils gegebenenfalls durch Halogen, Cyano oder $C_1$-$C_4$-Alkoxy substituiertes Alkyl, Alkoxy, Alkylthio, Alkylamino oder Dialkylamino mit jeweils 1 bis 6 Kohlenstoffatomen in den Alkylresten steht,

$R^2$ für Wasserstoff, Amino, Cyano oder für jeweils gegebenenfalls durch Halogen, Cyano oder $C_1$-$C_4$-Alkoxy substituiertes Alkyl, Alkoxy, Alkylamino oder Dialkylamino mit jeweils 1 bis 6 Kohlenstoffatomen in den Alkylresten steht, und

$R^3$ für Wasserstoff, Cyano oder für jeweils gegebenenfalls durch Halogen, Cyano oder $C_1$-$C_4$-Alkoxy substituiertes Alkyl, Alkenyl oder Alkinyl mit jeweils bis zu 6 Kohlenstoffatomen oder für jeweils gegebenenfalls durch Halogen, Cyano, Carboxy, $C_1$-$C_4$-Alkyl oder $C_1$-$C_4$-Alkoxy-carbonyl substituiertes Cycloalkyl oder Cycloalkylalkyl mit 3 bis 6 Kohlenstoffatomen in den Cycloalkylteilen und gegebenenfalls 1 bis 4 Kohlenstoffatomen im Alkylteil steht,

G für eine Einfachbindung, für Sauerstoff, für jeweils gegebenenfalls durch Halogen, Hydroxy, $C_1$-$C_4$-Alkyl, $C_1$-$C_4$-Halogenalkyl oder $C_3$-$C_6$-Cycloalkyl substituiertes Alkandiyl, Alkendiyl, Oxaalkendiyl, Alkindiyl mit jeweils bis zu 4 Kohlenstoffatomen oder eine der nachstehenden Gruppierungen steht
-Q-CQ-, -CQ-Q-, $-CH_2$-Q-; -Q-$CH_2$-, -CQ-Q-$CH_2$-, $CH_2$-Q-CQ-, -Q-CQ-$CH_2$-, -Q-CQ-Q-$CH_2$-, -N=N-, $-S(O)_n-$, $-CH_2-S(O)_n-$, -CQ-, $-S(O)_n-CH_2-$, $-C(R^4)$=N-O-, $-C(R^4)$=N-O-$CH_2$-, $-N(R^5)-$, -CQ-$N(R^5)$-, $-N(R^5)$-CQ-, -Q-CQ-$N(R^5)$-, -N=$C(R^4)$-Q-$CH_2$-, $-CH_2$-O-N=$C(R^4)$-, $-N(R^5)$-CQ-Q-, -CQ-$N(R^5)$-CQ-Q-, $-N(R^5)$-CQ-Q-$CH_2$-, -CQ-$CH_2$- oder -N=N-$C(R^4)$=N-O-,
wobei

n für die Zahlen 0, 1 oder 2 steht,

Q für Sauerstoff oder Schwefel steht,

$R^4$ für Wasserstoff, Cyano, für gegebenenfalls durch Halogen, Cyano oder $C_1$-$C_4$-Alkoxy substituiertes Alkyl, Alkoxy, Alkylthio, Alkylamino oder Dialkylamino mit jeweils 1 bis 6 Kohlenstoffatomen in den Alkylgruppen oder für jeweils gegebenenfalls durch Halogen, Cyano, Carboxy, $C_1$-$C_4$-Alkyl oder $C_1$-$C_4$-Alkoxy-carbonyl substituiertes Cycloalkyl mit 3 bis 6 Kohlenstoffatomen steht, und

$R^5$ für Wasserstoff, Hydroxy, Cyano oder für gegebenenfalls durch Halogen, Cyano oder $C_1$-$C_4$-Alkoxy substituiertes Alkyl mit 1 bis 6 Kohlenstoffatomen oder für gegebenenfalls durch Halogen, Cyano, Carboxy, $C_1$-$C_4$-Alkyl oder $C_1$-$C_4$-Alkoxy-carbonyl substituiertes Cycloalkyl mit 3 bis 6 Kohlenstoffatomen steht, und

Z für gegebenenfalls durch Halogen, Cyano, Hydroxy, Amino, $C_1$-$C_4$-Alkoxy, $C_1$-$C_4$-Alkylthio, $C_1$-$C_4$-Alkylsulfinyl oder $C_1$-$C_4$-Alkylsulfonyl (welche jeweils gegebenenfalls durch Halogen substituiert sind) substituiertes Alkyl mit 1 bis 8 Kohlenstoffatomen, für jeweils gegebenenfalls durch Halogen substituiertes Alkenyl oder Alkinyl mit jeweils bis zu 8 Kohlenstoffatomen, für jeweils gegebenenfalls durch Halogen, Cyano, Carboxy, Phenyl (welches gegebenenfalls durch Halogen, Cyano, $C_1$-$C_4$-Alkyl, $C_1$-$C_4$-Halogenalkyl, $C_1$-$C_4$-Alkoxy oder $C_1$-$C_4$-Halogenalkoxy substituiert ist), $C_1$-$C_4$-Alkyl oder $C_1$-$C_4$-Alkoxy-carbonyl substiuiertes Cycloalkyl mit 3 bis 6 Kohlenstoffatomen, für jeweils gegebenenfalls substituiertes Phenyl, Naphthyl oder (gegebenenfalls benzannelliertes) Heterocyclyl mit 5 oder 6 Ringgliedern, von denen mindestens eines für Sauerstoff, Schwefel oder Stickstoff und gegebenenfalls ein oder zwei weitere für Stickstoff stehen, steht, wobei die möglichen Substituenten vorzugsweise aus der nachstehenden Aufzählung ausgewählt sind:
Sauerstoff (als Ersatz für zwei geminale Wasserstoffatome), Halogen, Cyano, Nitro, Amino, Hydroxy, Formyl, Carboxy, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl oder Alkenyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen, Cycloalkyl mit 3 bis 6 Kohlenstoffatomen, Heterocyclyl oder Heterocyclyl-methyl mit jeweils 3 bis 7 Ringgliedern, von denen jeweils 1 bis 3 gleiche oder verschiedene Heteroatome sind - insbesondere Stickstoff, Sauerstoff und/oder Schwefel -, sowie jeweils gegebenenfalls im Phenylteil einfach oder mehrfach, gleich oder verschieden durch Halogen, Cyano, Nitro, Carboxy, Carbamoyl und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen und/oder geradkettiges oder verzweigtes Alkoxy mit 1 bis 4 Kohlenstoffatomen und/oder geradkettiges oder verzweigtes Halogenalkoxy mit 1 bis 4 Kohlenstoffatomen und 1 bis 9 gleichen oder verschiedenen Halogenatomen und/oder Alkylcarbonyl oder Alkoxycarbonyl mit jeweils bis zu 5 Kohlenstoffatomen substituiertes Phenyl, Phenoxy, Benzyl, Benzyloxy, Phenylethyl oder Phenylethyloxy.

**3.** Verbindungen der Formel (I) nach Anspruch 1, in welcher

A für jeweils gegebenenfalls durch Fluor, Chlor, Methyl, Ethyl oder Trifluormethyl substituiertes Methylen oder Dimethylen (Ethan-1,2-diyl) steht

Ar für jeweils gegebenenfalls substituiertes ortho-, meta- oder paraphenylen, für Furandiyl, Thiophendiyl, Pyrroldiyl, Pyrazoldiyl, Triazoldiyl, Oxazoldiyl, Isoxazoldiyl, Thiazoldiyl, Isothiazoldiyl, Oxadiazoldiyl, Thiadiazoldiyl, Pyridindiyl, Pyrimidindiyl, Pyridazindiyl, Pyrazindiyl, 1,3,4-Triazindiyl oder 1,2,3-Triazindiyl steht, wobei die möglichen Substituenten insbesondere aus der nachstehenden Aufzählung ausgewählt sind:
Fluor, Chlor, Cyano, Methyl, Ethyl, Trifluormethyl, Methoxy, Ethoxy, Methylthio, Methylsulfinyl oder Methylsulfonyl,

E für eine der nachstehenden Gruppierungen steht

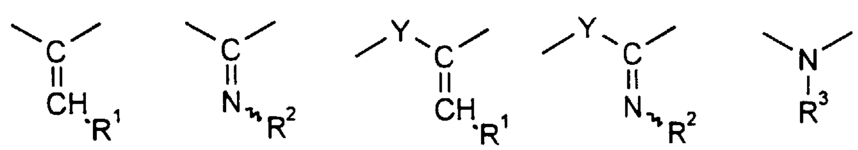

worin

Y für Sauerstoff, Schwefel, Methylen ($CH_2$) oder Alkylimino (N-R) steht,

R für Methyl, Ethyl, n- oder i-Propyl, n-, i- oder s-Butyl steht,

$R^1$ für Wasserstoff, Fluor, Chlor, Brom, Cyano oder für jeweils gegebenenfalls durch Fluor, Chlor, Cyano, Methoxy oder Ethoxy substituiertes Methyl, Ethyl, Propyl, Methoxy, Ethoxy, Methylthio, Ethylthio, Methylamino, Ethylamino oder Dimethylamino steht,

$R^2$ für Wasserstoff, Amino, Cyano oder für jeweils gegebenenfalls durch Fluor, Chlor, Cyano, Methoxy oder Ethoxy substituiertes Methyl, Ethyl, Methoxy, Ethoxy, Methylamino, Ethylamino oder Dimethylamino steht, und

$R^3$ für Wasserstoff, Cyano oder für jeweils gegebenenfalls durch Fluor, Cyano, Methoxy oder Ethoxy substituiertes Methyl, Ethyl n- oder i-Propyl, n-, i- oder s-Butyl, für Allyl oder Propargyl oder für jeweils gegebenenfalls durch Fluor, Chlor, Cyano, Carboxy, Methyl, Ethyl, n- oder i-Propyl, Methoxy-carbonyl oder Ethoxy-carbonyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht,

G für eine Einfachbindung, für Sauerstoff, für jeweils gegebenenfalls durch Fluor, Chlor, Hydroxy, Methyl, Ethyl, n- oder i-Propyl, Trifluormethyl, Cyclopropyl, Cyclobutyl, Cyclopentyl oder Cyclohexyl substituiertes Methylen, Dimethylen (Ethan-1,2-diyl), Ethen-1,2-diyl, Ethin-1,2-diyl oder eine der nachstehenden Gruppierungen -Q-CQ-, -CQ-Q-, $-CH_2-Q-$; $-Q-CH_2-$, $-CQ-Q-CH_2-$, $CH_2-Q-CQ-$, $-Q-CQ-CH_2-$, $-Q-CQ-Q-CH_2-$, -N=N-, $-S(O)_n-$, $-CH_2-S(O)_n-$, -CQ-, $-S(O)_n-CH_2-$, $-C(R^4)=N-O-$, $-C(R^4)=N-O-CH_2-$, $-N(R^5)-$, $-CQ-N(R^5)-$, $-N(R^5)-CQ-$, $-Q-CQ-N(R^5)-$, $-N=C(R^4)-Q-CH_2-$, $-CH_2-O-N=C(R^4)-$, $-N(R^5)-CQ-Q-$, $-CQ-N(R^5)-CQ-Q-$, $-N(R^5)-CQ-Q-CH_2-$, $-CQ-CH_2-$ oder $-N=N-C(R^4)=N-O-$,
wobei

n für die Zahlen 0, 1 oder 2 steht,

Q für Sauerstoff oder Schwefel steht,

$R^4$ für Wasserstoff, Cyano, für gegebenenfalls durch Fluor, Chlor, Cyano, Methoxy oder Ethoxy substituiertes Methyl, Ethyl, n- oder i-Propyl, n-, i- oder s-Butyl, Methoxy, Ethoxy, Propoxy, Butoxy, Methylthio, Ethylthio, Propylthio, Butylthio, Methylamino, Ethylamino, Propylamino, Dimethylamino oder Diethylamino oder für jeweils gegebenenfalls durch Fluor, Chlor, Cyano, Carboxy, Methyl, Ethyl, n- oder i-Propyl, Methoxycarbonyl oder Ethoxy-carbonyl substituiertes Cyclopropyl, Cyclobutyl, Cyclopentyl oder Cyclohexyl steht, und

$R^5$ für Wasserstoff, Hydroxy, Cyano oder für jeweils gegebenenfalls durch Fluor, Chlor, Cyano, Methoxy oder Ethoxy substituiertes Methyl, Ethyl, n- oder i-Propyl, n-, i-, s- oder t-Butyl oder für gegebenenfalls durch Fluor, Chlor, Cyano, Carboxy, Methyl, Ethyl, n- oder i-Propyl, Methoxy-carbonyl oder Ethoxy-carbonyl substituiertes Cyclopropyl, Cyclobutyl, Cyclopentyl oder Cyclohexyl steht, und

Z für gegebenenfalls durch Fluor, Chlor, Brom, Cyano, Hydroxy, Amino, Methoxy, Ethoxy, Methylthio, Ethylthio, Methylsulfinyl, Ethylsulfinyl, Methylsulfonyl, Ethylsulfonyl (welche jeweils gegebenenfalls durch Fluor und/ oder Chlor substituiert sind) substituiertes Methyl, Ethyl, n- oder i-Propyl, n-, i-, s- oder t-Butyl, für jeweils gegebenenfalls durch Fluor, Chlor oder Brom substituiertes Allyl, Crotonyl, 1-Methyl-allyl, Propargyl oder 1-Methyl-propargyl, für jeweils gegebenenfalls durch Fluor, Chlor, Brom, Cyano, Carboxy, Phenyl (welches gegebenenfalls durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiert ist), Methyl, Ethyl, n- oder i-Propyl, Methoxy-carbonyl oder Ethoxy-carbonyl substiuiertes Cyclopropyl, Cyclobutyl, Cyclopentyl oder Cyclohexyl , für jeweils gegebenenfalls substituiertes Phenyl, Naphthyl, Furyl, Tetrahydrofuryl, Benzofuryl, Tetrahydropyranyl, Thienyl, Benzothienyl, Pyrrolyl, Dihydropyrrolyl, Tetrahydropyrrolyl, Benzopyrrolyl, Benzodihydropyrrolyl, Oxazolyl, Benzoxazolyl, Isoxazolyl, Thiazolyl, Benzthiazolyl, Isothiazolyl, Imidazolyl, Benzimidazolyl, Oxadiazolyl, Thiadiazolyl, Pyridinyl, Pyrimidinyl, Pyridazinyl, Pyrazinyl, 1,2,3-Triazinyl, 1,2,4-Triazinyl oder 1,3,5-Triazinyl steht, wobei die möglichen Substituenten vorzugsweise aus der nachstehenden Aufzählung ausgewählt sind:
Sauerstoff (als Ersatz für zwei geminale Wasserstoffatome), Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl; jeweils gegebenenfalls einfach oder mehrfach, gleich oder verschieden durch Fluor, Chlor, Methyl, Ethyl, n- oder i-Propyl substituiertes Trimethylen (Propan-1,3-diyl), Methylendioxy oder

Ethylendioxy, Cyclopropyl, Cyclobutyl, Cyclopentyl oder Cyclohexyl, sowie jeweils gegebenenfalls im Phenylteil einfach oder mehrfach, gleich oder verschieden durch Fluor, Chlor, Brom, Cyano, Nitro, Carboxy, Carbamoyl, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Acetyl, Methoxycarbonyl oder Ethoxycarbonyl substituiertes Phenyl, Phenoxy, Benzyl oder Benzyloxy.

**4.** Verbindungen der Formel (I) nach Anspruch 1, in welcher

A für Dimethylen (Ethan-1,2-diyl) steht,

Ar für ortho-Phenylen, Pyridin-2,3-diyl oder Thiophen-2,3-diyl steht,

E für eine der nachstehenden Gruppierungen steht

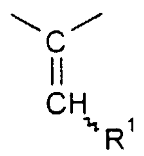

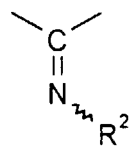

worin

$R^1$ und $R^2$ jeweils für Methoxy stehen,

G für Sauerstoff, Methylen oder eine der nachstehenden Gruppierungen $-CH_2-O-$, $-O-CH_2-$, $-S(O)_n-$, $-CH_2-S(O)_n-$, $-S(O)_n-CH_2-$,$-C(R^4)=N-O$, $-O-N=C(R^4)-$, $-C(R^4)=N-O-CH_2-$, $-N(R^5)-$ oder-$CH_2-O-N=C(R^4)-$ steht,
wobei

n für die Zahlen 0, 1 oder 2 steht,

$R^4$ für Wasserstoff, Methyl oder Ethyl steht und

$R^5$ für Wasserstoff, Methyl oder Ethyl steht, und

Z für jeweils gegebenenfalls substituiertes Phenyl, Pyridinyl, Pyrimidinyl, Pyridazinyl, Pyrazinyl, 1,2,3-Triazinyl, 1,2,4-Triazinyl oder 1,3,5-Triazinyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s-oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluormethylthio, Trifluormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl, jeweils gegebenenfalls einfach oder mehrfach, gleich oder verschieden durch Fluor, Chlor, Methyl oder Ethyl substituiertes Methylendioxy oder Ethylendioxy, sowie jeweils gegebenenfalls im Phenylteil einfach oder mehrfach, gleich oder verschieden durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, Phenoxy, Benzyl oder Benzyloxy.

**5.** Fungizide Mittel, gekennzeichnet durch einen Gehalt an mindestens einer Verbindung der Formel (I) gemäß Anspruch 1.

**6.** Verfahren zur Bekämpfung von unerwünschten Pilzen, dadurch gekennzeichnet, daß man Verbindungen der Formel (I) gemäß Anspruch 1 auf Pilze und/oder ihren Lebensraum einwirken läßt.

**7.** Verfahren zur Herstellung von Azadioxacycloalkenen der allgemeinen Formel (I)

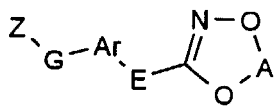

(I)

in welcher

A für gegebenenfalls substituiertes Alkandiyl (Alkylen) steht,

Ar für jeweils gegebenenfalls substituiertes Arylen oder Heteroarylen steht,

E für eine l-Alken-1,1-diyl-Gruppierung steht, die in 2-Position einen Rest R' enthält, oder für eine 2-Aza-1-alken-1,1-diyl-Gruppierung steht, die in 2-Position einen Rest $R^2$ enthält, oder für eine 3-Oxa- oder 3-Thia-1-propen-2,3-diyl-Gruppierung steht, die in 1-Position einen Rest $R^1$ enthält, oder für eine 3-Aza-1-propen-2,3-diyl-Gruppierung steht, die in 3-Position einen Rest R und in 1-Position einen Rest $R^1$ enthält, oder für eine 1-Aza-1-propen-2,3-diyl-Gruppierung steht, die in 1-Position einen Rest $R^2$ enthält, oder für eine 3-Oxa- oder 3-Thia-1-aza-propen-2,3-diyl-Gruppierung steht, die in 1-Position einen Rest $R^2$ enthält, oder für eine 1,3-Diaza-1-propen-2,3-diyl-Gruppierung steht, die in 3-Position einen Rest R und in 1-Position einen Rest $R^2$ enthält, oder für eine gegebenenfalls substituierte Imino-Gruppierung ("Azamethylen", N-$R^3$) steht,
wobei

R für Alkyl steht,

$R^1$ für Wasserstoff, Halogen, Cyano oder für jeweils gegebenenfalls substituiertes Alkyl, Alkoxy, Alkylthio, Alkylamino oder Dialkylamino steht,

$R^2$ für Wasserstoff, Amino, Cyano oder für jeweils gegebenenfalls substituiertes Alkyl, Alkoxy, Alkylamino oder Dialkylamino steht, und

$R^3$ für Wasserstoff, Cyano oder für jeweils gegebenenfalls substituiertes Alkyl, Alkenyl, Alkinyl, Cycloalkyl oder Cycloalkylalkyl steht,

G für eine Einfachbindung, für Sauerstoff, für jeweils gegebenenfalls durch Halogen, Hydroxy, Alkyl, Halogenalkyl oder Cycloalkyl substituiertes Alkandiyl, Alkendiyl, Oxaalkendiyl, Alkindiyl oder eine der nachstehenden Gruppierungen steht
-Q-CQ-, -CQ-Q-, -$CH_2$-Q-, -Q-$CH_2$-, -CQ-Q-$CH_2$-, $CH_2$-Q-CQ-, -Q-CQ-$CH_2$-, -Q-CQ-Q-$CH_2$-, -N=N-, -$S(O)_n$-, -$CH_2$-$S(O)_n$-, -CQ-, -$S(O)_n$-$CH_2$-, -C($R^4$)=N-D-, -C($R^4$)=N-O-$CH_2$-, -N($R^5$)-, -CQ-N($R^5$)-, -N($R^5$)-CQ-, -Q-CQ-N($R^5$)-, -N=C($R^4$)-Q-$CH_Q$-, -$CH_2$-O-N=C($R^4$)-, -N($R^5$)-CQ-Q-, -CQ-N($R^5$)-CQ-Q-, -N($R^5$)-CQ-Q-$CH_2$-, -CQ-$CH_2$- oder -N=N-C($R^4$)=N-D-,
wobei

n für die Zahlen 0, 1 oder 2 steht,

Q für Sauerstoff oder Schwefel steht,

$R^4$ für Wasserstoff, Cyano oder jeweils gegebenenfalls substituiertes Alkyl, Alkoxy, Alkylthio, Alkylamino, Dialkylamino oder Cycloalkyl steht, und

$R^5$ für Wasserstoff, Hydroxy, Cyano oder jeweils gegebenenfalls substituiertes Alkyl, Alkoxy oder Cycloalkyl steht, und

Z für jeweils gegebenenfalls substituiertes Alkyl, Alkenyl, Alkinyl, Cycloalkyl, Aryl oder Heterocyclyl steht.

dadurch gekennzeichnet, daß man

(a) Carbonsäurederivate der allgemeinen Formel (II)

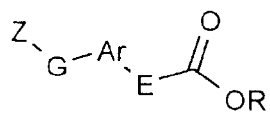

(II)

in welcher

Ar, E, G und Z die oben angegebene Bedeutung haben, und

R für Alkyl steht,

in einer ersten Stufe mit Hydroxylamin oder mit einem Hydrogenhalogenid hiervon, gegebenenfalls in Gegenwart eines Säureakzeptors und gegebenenfalls in Gegenwart eines Verdünnungsmittels umsetzt und in situ, d.h. ohne Zwischenisolierung des Produktes der ersten Stufe, in einer zweiten Stufe mit disubstituierten Alkanen der allgemeinen Formel (III)

X-A-X (III)

in welcher

A die oben angegebene Bedeutung hat und

X für Halogen, Alkylsulfonyloxy oder Arylsulfonyloxy steht,

gegebenenfalls in Gegenwart eines Säureakzeptors und gegebenenfalls in Gegenwart eines Verdünnungsmittels umsetzt, oder wenn man

(b) für den Fall, daß in der Formel (I) G für Sauerstoff oder die Gruppierung $-CH_2-O-$ steht und A, Ar, E und Z die in Anspruch 1 angegebene Bedeutung haben,
Hydroxyarylverbindungen der allgemeinen Formel (IV),

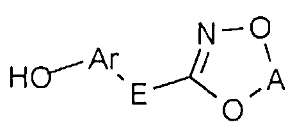

(IV)

in welcher

A, Ar und E die oben angegebene Bedeutung haben,

mit Verbindungen der allgemeinen Formel (V),

$Z-(CH_2)_m-X$ (V)

in welcher

X und Z die oben angegebene Bedeutung haben und

m für die Zahlen 0 oder 1 steht,

gegebenenfalls in Gegenwart eines Säureakzeptors und gegebenenfalls in Gegenwart eines Verdünnungsmittels umsetzt und gegebenenfalls anschließend an der Gruppierung Z nach üblichen Methoden Substitutionsreaktionen durchführt, oder wenn man

(c) für den Fall, daß in der Formel (I) G für die Gruppierung -Q-$CH_2$-steht und A, Ar, E und Z die oben angegebene Bedeutung haben,
Halogenmethylverbindungen der allgemeinen Formel (VI)

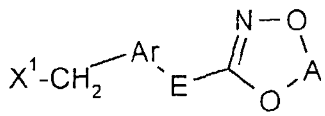

(VI)

in welcher

A, Ar und E die oben angegebene Bedeutung haben und

$X^1$ für Halogen steht,

mit Verbindungen der allgemeinen Formel (VII)

Z-Q-H (VII)

in welcher

Q und Z die oben angegebene Bedeutung haben,

gegebenenfalls in Gegenwart eines Säureakzeptors und gegebenenfalls in Gegenwart eines Verdünnungsmittels umsetzt, oder wenn man

(d) Hydroxyalkoxyamide der allgemeinen Formel (VIII)

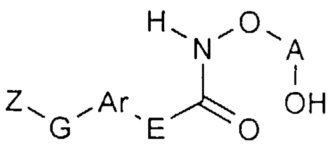

(VIII)

in welcher

A, Ar, E, G und Z die oben angegebenen Bedeutung haben,

mit einem Wasser-entziehenden Mittel, gegebenenfalls in Gegenwart eines Verdünnungsmittels dehydratisierend cyclisiert.

**8.** Verwendung von Verbindungen der Formel (I) gemäß Anspruch 1 als Fungizide.

**9.** Verfahren zur Herstellung von fungiziden Mitteln, dadurch gekennzeichnet, daß man Verbindungen der Formel (I) gemäß Anspruch 1 mit Streckmitteln und/oder oberflächenaktiven Stoffen vermischt.

**10.** Verbindungen der Formel (IV)

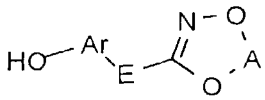 (IV)

in welcher A, Ar und E die in Anspruch 1 angegebenen Bedeutungen haben.

**11.** Verfahren zur Herstellung von Verbindungen der Formel (IV), dadurch gekennzeichnet, daß man Tetrahydropyranyloxyverbindungen der allgemeinen Formel (IX)

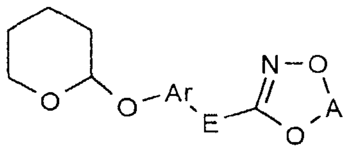 (IX)

in welcher

A, Ar und E die in Anspruch 10 angegebene Bedeutung haben,

mit einer Säure, oder einem sauren Ionenaustauscher, gegebenenfalls in Gegenwart eines Verdünnungsmittels bei Temperaturen zwischen 0°C und 100°C umsetzt.

**12.** Verbindungen der Formel (IX)

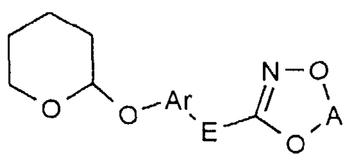 (IX)

in welcher A, Ar und E die in Anspruch 1 angegebenen Bedeutungen haben.

**13.** Verfahren zur Herstellung von Verbindungen der Formel (IX), dadurch gekennzeichnet, daß man Ester der allgemeinen Formel (X)

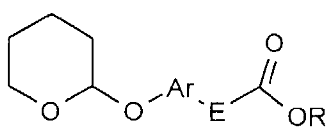 (X)

in welcher

Ar, E und R die in Anspruch 12 angegebene Bedeutung haben,

mit Hydroxylamin - oder gegebenenfalls mit dessen Hydrochlorid - gegebenenfalls in Gegenwart eines Säureakzeptors, und gegebenenfalls in Gegenwart von Verdünnungsmitteln, umsetzt und das hierbei gebildete Intermediat in situ weiter mit Dihalogenalkanen der allgemeinen Formel (III) - wie in Anspruch 7 angegeben - gegebenenfalls in Gegenwart eines Säureakzeptors bei Temperaturen zwischen 0°C und 100°C umsetzt.

**14.** Verbindungen der Formel (VI)

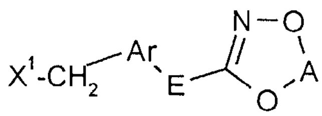

(VI)

in welcher
A, Ar und E die in Anspruch 1 angegebenen Bedeutungen haben und $X^1$ für Halogen steht.

**15.** Verfahren zur Herstellung von Verbindungen der Formel (VI), dadurch gekennzeichnet, daß man Methylverbindungen der allgemeinen Formel (XIII)

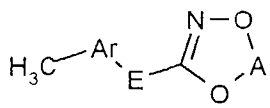

(XIII)

in welcher

A, Ar und E die in Anspruch 14 angegebene Bedeutung haben,

mit einem Halogenierungsmittel gegebenenfalls in Gegenwart eines Katalysators und gegebenenfalls in Gegenwart eines Verdünnungsmittels, wie z.B. Tetrachlormethan, bei Temperaturen zwischen 0°C und 150°C umsetzt.

**16.** Verbindungen der Formel (XIII)

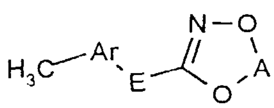

(XIII)

in welcher A, Ar und E die in Anspruch 1 angegebenen Bedeutungen haben.

**17.** Verfahren zur Herstellung von Verbindungen der Formel (XIII), dadurch gekennzeichnet, daß wenn man Ester der allgemeinen Formel (XIV)

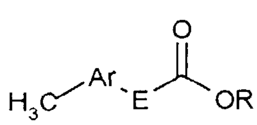

(XIV)

in welcher

Ar, E und R die in Anspruch 16 angegebene Bedeutung haben,

mit Hydroxylamin oder Hydroxylamin-Hydrochlorid, gegebenenfalls in Gegenwart eines Säureakzeptors und gegebenenfalls in Gegenwart eines Verdünnungsmittels und anschließend mit einem disubstituierten Alkan der allgemeinen Formel (III) - wie in Anspruch 7 angegeben - gegebenenfalls in Gegenwart eines Säureakzeptors analog zum erfindungsgemäßen Verfahren (a) nach Anspruch 7 bei Temperaturen zwischen 0°C und 150°C umsetzt.

**18.** Verbindungen der Formel (VIII)

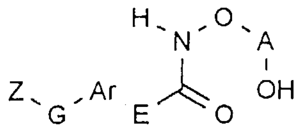

(VIII)

in welcher A, Ar, E, G und Z die in Anspruch 1 angegebenen Bedeutungen haben.

**19.** Verfahren zur Herstellung von Verbindungen der Formel (VIII), dadurch gekennzeichnet, daß man Carbonsäurederivate der allgemeinen Formel (XV)

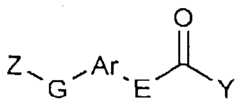

(XV)

in welcher

Ar, E, G und Z die in Anspruch 18 angegebene Bedeutung haben und

Y für Halogen, Hydroxy oder Alkoxy steht,

mit Hydroxylaminen der allgemeinen Formel (XVI)

$$H_2N\text{-}O\text{-}A\text{-}OH \qquad (XVI)$$

in welcher

A die oben angegebene Bedeutung hat,

gegebenenfalls in Gegenwart eines Säureakzeptors und gegebenenfalls in Gegenwart eines Verdünnungsmittels bei Temperaturen zwischen 0°C und 150°C umsetzt.

## Claims

**1.** Azadioxacycloalkenes of the general formula (I)

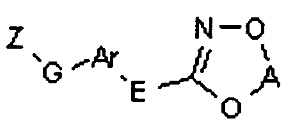

(I)

in which

A represents optionally substituted alkanediyl (alkylene),

Ar represents in each case optionally substituted arylene or heteroarylene,

E represents a 1-alkene-1,1-diyl group with a radical $R^1$ in the 2-position, or a 2-aza-1-alkene-1,1-diyl group with a radical $R^2$ in the 2-position, or a 3-oxa- or 3-thia-1-propene-2,3-diyl group with a radical $R^1$ in the 1-position, or represents a 3-aza-1-propene-2,3-diyl group with a radical R in the 3-position and a radical $R^1$

in the 1-position, or represents a 1-aza-1-propene-2,3-diyl group with a radical $R^2$ in the 1-position, or represents a 3-oxa- or 3-thia-l-aza-propene-2,3-diyl group with a radical $R^2$ in the 1-position, or represents a 1,3-diaza-1-propene-2,3-diyl group with a radical R in the 3-position and a radical $R^2$ in the 1-position, or represents an optionally substituted imino group ("azamethylene", N-$R^3$),
where

R represents alkyl,

$R^1$ represents hydrogen, halogen, cyano or in each case optionally substituted alkyl, alkoxy, alkylthio, alkylamino or dialkylamino,

$R^2$ represents hydrogen, amino, cyano or in each case optionally substituted alkyl, alkoxy, alkylamino or dialkylamino, and

$R^3$ represents hydrogen, cyano or in each case optionally substituted alkyl, alkenyl, alkinyl, cycloalkyl or cycloalkylalkyl,

G represents a single bond, oxygen, or represents alkanediyl, alkenediyl, oxaalkenediyl, alkinediyl, each of which is optionally substituted by halogen, hydroxyl, alkyl, halogenoalkyl or cycloalkyl, or represents one of the groups below
-Q-CQ-, -CQ-Q-, -$CH_2$-Q-; -Q-$CH_2$-, -CQ-Q-$CH_2$-, -$CH_2$-Q-CQ-, -Q-CQ-$CH_2$-, -Q-CQ-Q-$CH_2$-, -N=N-, -S $(O)_n$-, -$CH_2$-S$(O)_n$-, -CQ-, -S$(O)_n$-$CH_2$-, -C($R^4$)=N-O-, -C($R^4$)=N-O-$CH_2$-, -N($R^5$)-, -CQ-N($R^5$)-, -N($R^5$)-CQ-, -Q-CQ-N($R^5$)-, -N=C($R^4$)-Q-$CH_2$-, -$CH_2$-O-N=C($R^4$)-, -N($R^5$)-CQ-Q-, -CQ-N($R^5$) -CQ-Q-, -N($R^5$)-CQ-Q-$CH_2$-, -CQ-$CH_2$- or -N=N-C ($R^4$)=N-O-,
where

n represents the numbers 0, 1 or 2,

Q represents oxygen or sulphur,

$R^4$ represents hydrogen, cyano, or represents alkyl, alkoxy, alkylthio, alkylamino, dialkylamino or cycloalkyl, each of which is optionally substituted, and

$R^5$ represents hydrogen, hydroxyl, cyano, or represents alkyl, alkoxy or cycloalkyl, each of which is optionally substituted, and

Z represents in each case optionally substituted alkyl, alkenyl, alkinyl, cycloalkyl, aryl or heterocyclyl.

**2.** Compounds of the formula (I) according to Claim 1, in which

A represents alkanediyl having 1 to 3 carbon atoms which is optionally substituted by halogen or by alkyl or halogenoalkyl, each of which has 1 to 4 carbon atoms,

Ar represents in each case optionally substituted phenylene or naphthylene, or represents heteroarylene having 5 or 6 ring members of which at least one represents oxygen, sulphur or nitrogen and, if appropriate, one or two further ring members representing nitrogen, the substituents which are possible preferably being selected from the list below:
halogen, cyano, nitro, amino, hydroxyl, formyl, carboxyl, carbamoyl, thiocarbamoyl, in each case straight-chain or branched alkyl, alkoxy, alkylthio, alkylsulphinyl or alkylsulphonyl, each of which has 1 to 6 carbon atoms, in each case straight-chain or branched alkenyl, alkenyloxy or alkinyloxy, each of which has 2 to 6 carbon atoms, in each case straight-chain or branched halogenoalkyl, halogenoalkoxy, halogenoalkylthio, halogenoalkylsulphinyl or halogenoalkylsulphonyl, each of which has 1 to 6 carbon atoms and 1 to 13 identical or different halogen atoms, in each case straight-chain or branched halogenoalkenyl or halogenoalkenyloxy, each of which has 2 to 6 carbon atoms and 1 to 13 identical or different halogen atoms, in each case straight-chain or branched alkylamino, dialkylamino, alkylcarbonyl, alkylcarbonyloxy, alkoxycarbonyl, alkylsulphonyloxy, hydroximinoalkyl or alkoximinoalkyl, each of which has 1 to 6 carbon atoms in the individual alkyl moieties, or represents in each case divalent alkylene or dioxyalkylene, each of which has 1 to 6 carbon atoms and each of which is optionally monosubstituted or polysubstituted by identical or different substituents from

the series consisting of halogen and/or straight-chain or branched alkyl having 1 to 4 carbon atoms and/or straight-chain or branched halogenoalkyl having 1 to 4 carbon atoms and 1 to 9 identical or different halogen atoms,

E represents one of the groups below

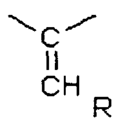 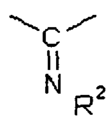 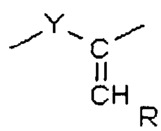 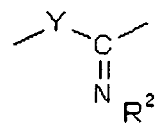 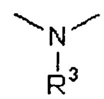

in which

Y represents oxygen, sulphur, methylene ($CH_2$) or alkylimino (N-R),

R represents alkyl having 1 to 6 carbon atoms,

$R^1$ represents hydrogen, halogen, cyano, or represents alkyl, alkoxy, alkylthio, alkylamino or dialkylamino, each of which has 1 to 6 carbon atoms in the alkyl radicals and each of which is optionally substituted by halogen, cyano or $C_1$-$C_4$-alkoxy,

$R^2$ represents hydrogen, amino, cyano, or represents alkyl, alkoxy, alkylamino or dialkylamino, each of which has 1 to 6 carbon atoms in the alkyl radicals and each of which is optionally substituted by halogen, cyano or $C_1$-$C_4$-alkoxy, and

$R^3$ represents hydrogen, cyano, or represents alkyl, alkenyl or alkinyl, each of which has up to 6 carbon atoms and each of which is optionally substituted by halogen, cyano or $C_1$-$C_4$-alkoxy, or represents cycloalkyl or cycloalkylalkyl having 3 to 6 carbon atoms in the cycloalkyl moieties and, if appropriate, 1 to 4 carbon atoms in the alkyl moiety, each of these cycloalkyl or cycloalkylalkyl radicals optionally being substituted by halogen, cyano, carboxyl, $C_1$-$C_4$-alkyl or $C_1$-$C_4$-alkoxy-carbonyl,

G represents a single bond, oxygen, or represents alkanediyl, alkenediyl, oxaalkenediyl, alkinediyl, each of which has up to 4 carbon atoms and each of which is optionally substituted by halogen, hydroxyl, $C_1$-$C_4$-alkyl, $C_1$-$C_4$-halogenoalkyl or $C_3$-$C_6$-cycloalkyl, or represents one of the groups below
-Q-CQ-, -CQ-Q-, $-CH_2$-Q-; -Q-$CH_2$-, -CQ-Q-$CH_2$-, -$CH_2$-Q-CQ-, -Q-CQ-$CH_2$-, -Q-CQ-Q-$CH_2$-, -N=N-, -$S(O)_n$-, -$CH_2$-$S(O)_n$-, -CQ-, -$S(O)_n$-$CH_2$-, -C($R^4$)=N-O-, -C($R^4$)=N-O-$CH_2$-, -N($R^5$)-, -CQ-N($R^5$)-, -N($R^5$)-CQ-, -Q-CQ-N($R^5$)-, -N=C ($R^4$)-Q-$CH_2$-, -$CH_2$-O-N=C($R^4$)-, -N($R^5$)-CQ-Q-, -CQ-N($R^5$)-CQ-Q-, -N($R^5$)-CQ-Q-$CH_2$-, -CQ-$CH_2$- or -N=N-C($R^4$)=N-O-,
where

n represents the numbers 0, 1 or 2,

Q represents oxygen or sulphur,

$R^4$ represents hydrogen, cyano, or represents alkyl, alkoxy, alkylthio, alkylamino or dialkylamino, each of which has 1 to 6 carbon atoms in the alkyl groups and each of which is optionally substituted by halogen, cyano or $C_1$-$C_4$-alkoxy, or represents cycloalkyl having 3 to 6 carbon atoms which is in each case optionally substituted by halogen, cyano, carboxyl, $C_1$-$C_4$-alkyl or $C_1$-$C_4$-alkoxy-carbonyl, and

$R^5$ represents hydrogen, hydroxyl, cyano, or represents alkyl having 1 to 6 carbon atoms which is optionally substituted by halogen, cyano or $C_1$-$C_4$-alkoxy, or represents cycloalkyl having 3 to 6 carbon atoms which is optionally substituted by halogen, cyano, carboxyl, $C_1$-$C_4$-alkyl or C1-C4-alkoxy-carbonyl, and

Z represents alkyl having 1 to 8 carbon atoms which is optionally substituted by halogen, cyano, hydroxyl, amino, $C_1$-$C_4$-alkoxy, $C_1$-$C_4$-alkylthio, $C_1$-$C_4$-alkylsulphinyl or $C_1$-$C_4$-alkylsulphonyl (each of which is optionally substituted by halogen), or represents alkenyl or alkinyl, each of which has up to 8 carbon atoms and

each of which is optionally substituted by halogen, or represents cycloalkyl having 3 to 6 carbon atoms which is in each case optionally substituted by halogen, cyano, carboxyl, phenyl (which is optionally substituted by halogen, cyano, $C_1$-$C_4$-alkyl, $C_1$-$C_4$-halogenoalkyl, $C_1$-$C_4$-alkoxy or $C_1$-$C_4$-halogenoalkoxy), $C_1$-$C_4$-alkyl or $C_1$-$C_4$-alkoxy-carbonyl, or represents in each case optionally substituted phenyl, naphthyl or (optionally benzo-fused) heterocyclyl having 5 or 6 ring members of which at least one represents oxygen, sulphur or nitrogen and, if appropriate, one or two further ring members represent nitrogen, other possible substituents preferably being selected from the list below:
oxygen (as a replacement for two geminal hydrogen atoms), halogen, cyano, nitro, amino, hydroxyl, formyl, carboxyl, carbamoyl, thiocarbamoyl, in each case straight-chain or branched alkyl, alkoxy, alkylthio, alkylsulphinyl or alkylsulphonyl, each of which has 1 to 6 carbon atoms, in each case straight-chain or branched alkenyl or alkenyloxy, each of which has 2 to 6 carbon atoms, in each case straight-chain or branched halogenoalkyl, halogenoalkoxy, halogenoalkylthio, halogenoalkylsulphinyl or halogenoalkylsulphonyl, each of which has 1 to 6 carbon atoms and 1 to 13 identical or different halogen atoms, in each case straight-chain or branched halogenoalkenyl or halogenoalkenyloxy, each of which has 2 to 6 carbon atoms and 1 to 13 identical or different halogen atoms, in each case straight-chain or branched alkylamino, dialkylamino, alkylcarbonyl, alkylcarbonyloxy, alkoxycarbonyl, alkylsulphonyloxy, hydroximinoalkyl or alkoximinoalkyl, each of which has 1 to 6 carbon atoms in the individual alkyl moieties, in each case divalent alkylene or dioxyalkylene, each of which has 1 to 6 carbon atoms and each of which is optionally monosubstituted or polysubstituted by identical or different substituents from the series consisting of halogen and/or straight-chain or branched alkyl having 1 to 4 carbon atoms and/or straight-chain or branched halogenoalkyl having 1 to 4 carbon atoms and 1 to 9 identical or different halogen atoms, or cycloalkyl having 3 to 6 carbon atoms, heterocyclyl or heterocyclylmethyl, each of which has 3 to 7 ring members, of which in each case 1 to 3 are identical or different hetero atoms - in particular nitrogen, oxygen and/or sulphur -, and phenyl, phenoxy, benzyl, benzyloxy, phenylethyl or phenylethyloxy, each of which is optionally monosubstituted or polysubstituted in the phenyl moiety by identical or different substituents from the series consisting of halogen, cyano, nitro, carboxyl, carbamoyl and/or straight-chain or branched alkyl having 1 to 4 carbon atoms and/or straight-chain or branched halogenoalkyl having 1 to 4 carbon atoms and 1 to 9 identical or different halogen atoms and/or straight-chain or branched alkoxy having 1 to 4 carbon atoms and/or straight-chain or branched halogenoalkoxy having 1 to 4 carbon atoms and 1 to 9 identical or different halogen atoms and/or alkylcarbonyl or alkoxycarbonyl, each of which has up to 5 carbon atoms.

**3.** Compounds of the formula (I) according to Claim 1, in which

A represents methylene or dimethylene (ethane-1,2-diyl) each of which is optionally substituted by fluorine, chlorine, methyl, ethyl or trifluoromethyl,

Ar represents in each case optionally substituted ortho-, meta- or para-phenylene, or represents furandiyl, thiophenediyl, pyrrolediyl, pyrazolediyl, triazolediyl, oxazolediyl, isoxazolediyl, thiazolediyl, isothiazolediyl, oxadiazolediyl, thiadiazolediyl, pyridinediyl, pyrimidinediyl, pyridazinediyl, pyrazinediyl, 1,3,4-triazinediyl or 1,2,3-triazinediyl, the possible substituents being selected, in particular, from the list below:
fluorine, chlorine, cyano, methyl, ethyl, trifluoromethyl, methoxy, ethoxy, methylthio, methylsulphinyl or methylsulphonyl,

E represents one of the groups below

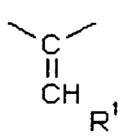 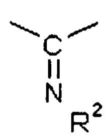 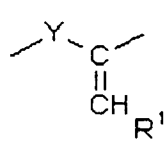 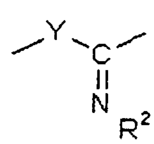 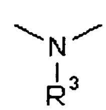

in which

Y represents oxygen, sulphur, methylene ($CH_2$) or alkylimino (N-R),

R represents methyl, ethyl, n- or i-propyl, or n-, i- or s-butyl,

$R^1$ represents hydrogen, fluorine, chlorine, bromine, cyano, or represents methyl, ethyl, propyl, methoxy, ethoxy, methylthio, ethylthio, methylamino, ethylamino or dimethylamino, each of which is optionally substituted by fluorine, chlorine, cyano, methoxy or ethoxy,

$R^2$ represents hydrogen, amino, cyano, or represents methyl, ethyl, methoxy, ethoxy, methylamino, ethylamino or dimethylamino, each of which is optionally substituted by fluorine, chlorine, cyano, methoxy or ethoxy, and

$R^3$ represents hydrogen, cyano, or represents methyl, ethyl, n- or i-propyl, or n-, i- or s-butyl, each of which is optionally substituted by fluorine, cyano, methoxy or ethoxy, represents allyl or propargyl, or represents cyclopropyl, cyclobutyl, cyclopentyl, cyclohexyl, cyclopropylmethyl, cyclobutylmethyl, cyclopentylmethyl or cyclohexylmethyl, each of which is optionally substituted by fluorine, chlorine, cyano, carboxyl, methyl, ethyl, n- or i-propyl, methoxy-carbonyl or ethoxy-carbonyl,

G represents a single bond, oxygen, or represents methylene, dimethylene (ethane-1,2-diyl), ethene-1,2-diyl, ethine-1,2-diyl, each of which is optionally substituted by fluorine, chlorine, hydroxyl, methyl, ethyl, n- or i-propyl, trifluoromethyl, cyclopropyl, cyclobutyl, cyclopentyl or cyclohexyl, or represents one of the groups below
$-Q-CQ-$, $-CQ-Q-$, $-CH_2-Q-$, $-Q-CH_2-$, $-CQ-Q-CH_2-$, $-CH_2-Q-CQ-$, $-Q-CQ-CH_2-$, $-Q-CQ-Q-CH_2-$, $-N=N-$, $-S(O)_n-$, $-CH_2-S(O)_n-$, $-CQ-$, $-S(O)_n-CH_2-$, $-C(R^4)=N-O-$, $-C(R^4)=N-O-CH_2-$, $-N(R^5)-$, $-CQ-N(R^5)-$, $-N(R^5)-CQ-$ , $-Q-CQ-N(R^5)-$, $-N=C(R^4)-Q-CH2-$, $CH_2-O-N=C(R^4)-$, $-N(R^5)-CQ-Q-$, $-CQ-N(R^5)-CQ-Q-$, $-N(R^5)-CQ-Q-CH_2-$, $-CQ-CH_2-$ or $-N=N-C(R^4)=N-O-$,
where

n represents the numbers 0, 1 or 2,

Q represents oxygen or sulphur,

$R^4$ represents hydrogen, cyano, or represents methyl, ethyl, n- or i-propyl, n-, i- or s-butyl, methoxy, ethoxy, propoxy, butoxy, methylthio, ethylthio, propylthio, butylthio, methylamino, ethylamino, propylamino, dimethylamino or diethylamino, each of which is optionally substituted by fluorine, chlorine, cyano, methoxy or ethoxy, or represents cyclopropyl, cyclobutyl, cyclopentyl or cyclohexyl, each of which is optionally substituted by fluorine, chlorine, cyano, carboxyl, methyl, ethyl, n- or i-propyl, methoxycarbonyl or ethoxycarbonyl, and

$R^5$ represents hydrogen, hydroxyl, cyano, or represents methyl, ethyl, n- or i-propyl or n-, i-, s- or t-butyl, each of which is optionally substituted by fluorine, chlorine, cyano, methoxy or ethoxy, or represents cyclopropyl, cyclobutyl, cyclopentyl or cyclohexyl, each of which is optionally substituted by fluorine, chlorine, cyano, carboxyl, methyl, ethyl, nor i-propyl, methoxy-carbonyl or ethoxycarbonyl, and

Z represents methyl, ethyl, n- or i-propyl or n-, i-, s- or t-butyl, each of which is optionally substituted by fluorine, chlorine, bromine, cyano, hydroxyl, amino, methoxy, ethoxy, methylthio, ethylthio, methylsulphinyl, ethylsulphinyl, methylsulphonyl or ethylsulphonyl (each of which is optionally substituted by fluorine and/or chlorine), or represents allyl, crotonyl, 1-methyl-allyl, propargyl or 1-methyl-propargyl, each of which is optionally substituted by fluorine, chlorine or bromine, or represents cyclopropyl, cyclobutyl, cyclopentyl or cyclohexyl, each of which is optionally substituted by fluorine, chlorine, bromine, cyano, carboxyl, phenyl (which is optionally substituted by fluorine, chlorine, bromine, cyano, methyl, ethyl, n- or i-propyl, n-, i-, s- or t-butyl, trifluoromethyl, methoxy, ethoxy, n- or i-propoxy, difluoromethoxy or trifluoromethoxy), methyl, ethyl, n- or i-propyl, methoxycarbonyl or ethoxy-carbonyl, or represents in each case optionally substituted phenyl, naphthyl, furyl, tetrahydrofuryl, benzofuryl, tetrahydropyranyl, thienyl, benzothienyl, pyrrolyl, dihydropyrrolyl, tetrahydropyrrolyl, benzopyrrolyl, benzodihydropyrrolyl, oxazolyl, benzoxazolyl, isoxazolyl, thiazolyl, benzothiazolyl, isothiazolyl, imidazolyl, benzimidazolyl, oxadiazolyl, thiadiazolyl, pyridinyl, pyrimidinyl, pyridazinyl, pyrazinyl, 1,2,3-triazinyl, 1,2,4-triazinyl or 1,3,5-triazinyl, the possible substituents in each case preferably being selected from the list below:
oxygen (as a replacement for two geminal hydrogen atoms), fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, difluoromethoxy, trifluoromethoxy, difluoromethylthio, trifluoro-methylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxy-carbonyl, methylsulphonyloxy, ethylsulphonyl-oxy, hydroximinomethyl, hydroximinoethyl, methoximino-methyl, ethoximinomethyl, methoximinoethyl or ethoximinoethyl; or trimethylene (propane-1,3-diyl), methylenedioxy or ethylenedioxy, each of which is optionally monosubstituted or polysubstituted by identical or different substituents from the series consisting of fluorine, chlorine, methyl, ethyl or n- or i-propyl, or cyclopropyl, cyclobutyl, cyclopentyl or cyclohexyl, and phenyl, phenoxy, benzyl or benzyloxy, each of which is optionally monosubstituted or polysubstituted in the phenyl moiety by identical or different substituents from the series consisting of fluorine, chlorine, bromine, cyano, nitro, carboxyl, carbamoyl, methyl, ethyl, n- or i-propyl, n-, i-, s- or t-butyl, trifluoromethyl, methoxy, ethoxy, n- or i-propoxy, difluoromethoxy, trifluoromethoxy, acetyl, methoxycarbonyl or ethoxycarbonyl.

**4.** Compounds of the formula (I) according to Claim 1, in which

A represents dimethylene (ethane-1,2-diyl),

Ar represents ortho-phenylene, pyridine-2,3-diyl or thiophene-2,3-diyl,

E represents one of the groups below

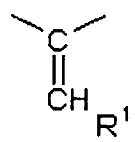

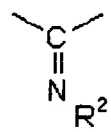

in which

$R^1$ and $R^2$ in each case represent methoxy,

G represents oxygen, methylene or one of the groups below
$-CH_2-O-$, $-O-CH_2-$, $-S(O)_n-$, $-CH_2-S(O)_n-$, $-S(O)_n-CH_2-$, $-C(R^4)=N-O-$, $O-N=C(R^4)-$, $-C(R^4)=N-O-CH_2-$, $-N(R^5)-$ or $-CH_2-O-N=C(R^4)-$,
where

n represents the numbers 0, 1 or 2,

$R^4$ represents hydrogen, methyl or ethyl and $R^5$ represents hydrogen, methyl or ethyl, and

Z represents in each case optionally substituted phenyl, pyridinyl, pyrimidinyl, pyridazinyl, pyrazinyl, 1,2,3-triazinyl, 1,2,4-triazinyl or 1,3,5-triazinyl, the possible substituents preferably being selected from the list below:
fluorine, chlorine, bromine, cyano, methyl, ethyl, n- or i-propyl, n-, i-, s- or t-butyl, methoxy, ethoxy, n- or i-propoxy, methylthio, ethylthio, n- or i- propylthio, methylsulphinyl, ethylsulphinyl, methylsulphonyl or ethylsulphonyl, trifluoromethyl, difluoromethoxy, trifluoromethoxy, difluoromethylthio, trifluoromethylthio, trifluoromethylsulphinyl or trifluoromethylsulphonyl, methoxycarbonyl, ethoxycarbonyl, methoximinomethyl, ethoximinomethyl, methoximinoethyl or ethoximinoethyl, or methylenedioxy or ethylenedioxy, each of which is optionally monosubstituted or polysubstituted by identical or different substituents from the series consisting of fluorine, chlorine, methyl or ethyl, and phenyl, phenoxy, benzyl or benzyloxy, each of which is optionally monosubstituted or polysubstituted in the phenyl moiety by identical or different substituents from the series consisting of fluorine, chlorine, bromine, cyano, methyl, ethyl, n- or i-propyl, n-, i-, s- or t-butyl, trifluoromethyl, methoxy, ethoxy, n- or i-propoxy, difluoromethoxy or trifluoromethoxy.

**5.** Fungicidal compositions, characterized in that they contain at least one compound of the formula (I) according to Claim 1.

**6.** Method of combating undesired fungi, characterized in that compounds of the formula (I) according to Claim 1 are allowed to act on fungi and/or their environment.

**7.** Process for the preparation of azadioxacycloalkenes of the general formula (I)

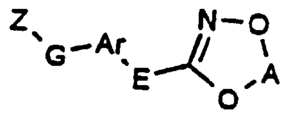

(I)

in which

A represents optionally substituted alkanediyl (alkylene),

Ar represents in each case optionally substituted arylene or heteroarylene,

E represents a 1-alkene-1,1-diyl group with a radical $R^1$ in the 2-position, or a 2-aza-1-alkene-1,1-diyl group with a radical $R^2$ in the 2-position, or a 3-oxa- or 3-thia-1-propene-2,3-diyl group with a radical $R^1$ in the 1-position, or represents a 3-aza-1-propene-2,3-diyl group with a radical R in the 3-position and a radical $R^1$ in the 1-position, or represents a 1-aza-1-propene-2,3-diyl group with a radical $R^2$ in the 1-position, or represents a 3-oxa- or 3-thia-l-aza-propene-2,3-diyl group with a radical $R^2$ in the 1-position, or represents a 1,3-diaza-1-propene-2,3-diyl group with a radical R in the 3-position and a radical $R^2$ in the 1-position, or represents an optionally substituted imino group ("azamethylene", N-$R^3$), where

R represents alkyl,

$R^1$ represents hydrogen, halogen, cyano or in each case optionally substituted alkyl, alkoxy, alkylthio, alkylamino or dialkylamino,

$R^2$ represents hydrogen, amino, cyano or in each case optionally substituted alkyl, alkoxy, alkylamino or dialkylamino, and

$R^3$ represents hydrogen, cyano or in each case optionally substituted alkyl, alkenyl, alkinyl, cycloalkyl or cycloalkylalkyl,

G represents a single bond, oxygen, or represents alkanediyl, alkenediyl, oxaalkenediyl, alkinediyl, each of which is optionally substituted by halogen, hydroxyl, alkyl, halogenoalkyl or cycloalkyl, or represents one of the groups below
-Q-CQ-, -CQ-Q-, -$CH_2$-Q-; -Q-$CH_2$-, -CQ-Q-$CH_2$-, -$CH_2$-Q-CQ-, -Q-CQ-$CH_2$-, -Q-CQ-Q-$CH_2$-, -N=N-, -$S(O)_n$-, -$CH_2$-$S(O)_n$-, -CQ-, -$S(O)_n$-$CH_2$-, -C($R^4$)=N-O-, -C($R^4$)=N-O-$CH_2$-, -N($R^5$)-, -CQ-N($R^5$)-, -N($R^5$)-CQ-, -Q-CQ-N($R^5$)-, -N=C($R^4$) -Q-$CH_2$-, -$CH_2$-O-N=C ($R^4$)-, -N($R^5$)-CQ-Q-, -CQ-N($R^5$)-CQ-Q-, -N($R^5$)-CQ-Q-$CH_2$-, -CQ-$CH_2$- or -N=N-C($R^4$)=N-O-,
where

n represents the numbers 0, 1 or 2,

Q represents oxygen or sulphur

$R^4$ represents hydrogen, cyano, or represents alkyl, alkoxy, alkylthio, alkylamino, dialkylamino or cycloalkyl, each of which is optionally substituted, and

$R^5$ represents hydrogen, hydroxyl, cyano, or represents alkyl, alkoxy or cycloalkyl, each of which is optionally substituted, and

Z represents in each case optionally substituted alkyl, alkenyl, alkinyl, cycloalkyl, aryl or heterocyclyl,

characterized in that

(a) carboxylic acid derivatives of the general formula (II)

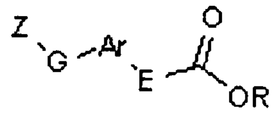

(II)

in which

Ar, E, G and Z have the abovementioned meaning and

R represents alkyl

are reacted, in a first step, with hydroxylamine or with a hydrohalide thereof, if appropriate in the presence of an acid acceptor and if appropriate in the presence of a diluent, and the product of the first step is reacted in situ, i.e. without intermediate isolation, in a second step with disubstituted alkanes of the general formula (III)

X-A-X (III)

in which

A has the abovementioned meaning and

X represents halogen, alkylsulphonyloxy or arylsulphonyloxy,

if appropriate in the presence of an acid acceptor and, if appropriate, in the presence of a diluent, or when,

(b) in the event that, in formula (I), G represents oxygen or the group $-CH_2-O-$ and A, Ar, E and Z have the meaning given in Claim 1,
hydroxyaryl compounds of the general formula (IV),

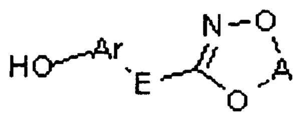

(IV)

in which

A, Ar and E have the abovementioned meaning

are reacted with compounds of the general formula (V),

$Z-(CH_2)_m-X$ (V)

in which

X and Z have the abovementioned meaning and

m represents the numbers 0 or 1,

if appropriate in the presence of an acid acceptor and, if appropriate, in the presence of a diluent, and, if appropriate, substitution reactions are subsequently carried out on the group Z by customary methods, or when,

(c) in the event that, in formula (I), G represents the group $-Q-CH_2-$ and A, Ar, E and Z have the abovementioned meaning,
halogenomethyl compounds of the general formula (VI)

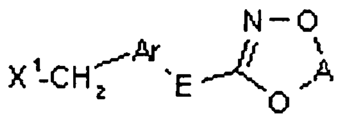

(VI)

in which

A, Ar and E have the abovementioned meaning and

$X^1$ represents halogen

are reacted with compounds of the general formula (VII)

Z-Q-H (VII)

in which

Q and Z have the abovementioned meaning,

if appropriate in the presence of an acid acceptor and, if appropriate, in the presence of a diluent, or when

(d) hydroxyalkoxyamides of the general formula (VIII)

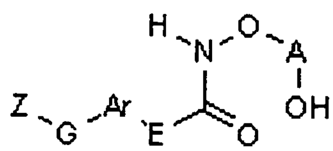

(VIII)

in which

A, Ar, E, G and Z have the abovementioned meaning

are subjected to a dehydrating cyclization reaction with a dehydrating agent, if appropriate in the presence of a diluent.

**8.** Use of compounds of the formula (I) according to Claim 1 as fungicides.

**9.** Process for the preparation of fungicidal compositions, characterized in that compounds of the formula (I) according to Claim 1 are mixed with extenders and/or surface-active substances.

**10.** Compounds of the formula (IV)

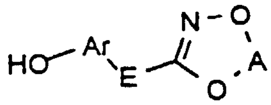

(IV)

in which A, Ar and E have the meanings given in Claim 1.

**11.** Process for the preparation of compounds of the formula (IV), characterized in that tetrahydropyranyloxy compounds of the general formula (IX)

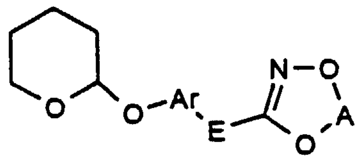

(IX)

in which

A, Ar and E have the meaning given in Claim 10

are reacted with an acid or an acidic ion exchanger at temperatures between 0°C and 100°C, if appropriate in the presence of a diluent.

**12.** Compounds of the formula (IX)

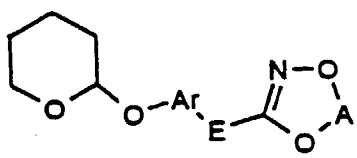

(IX)

in which A, Ar and E have the meanings given in Claim 1.

**13.** Process for the preparation of compounds of the formula (IX), characterized in that esters of the general formula (X)

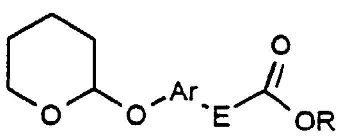

(X)

in which

Ar, E and R have the meaning given in Claim 12

are reacted with hydroxylamine - or, if appropriate, with its hydrochloride - if appropriate in the presence of an acid acceptor and if appropriate in the presence of diluents, and the intermediate formed in this process is reacted further in situ with dihalogenalkanes of the general formula (III) - as given in Claim 7 - at temperatures between 0°C and 100°C, if appropriate in the presence of an acid acceptor.

**14.** Compounds of the formula (VI)

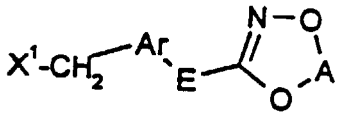

(VI)

in which

A, Ar and E have the meanings given in Claim 1 and
$X^1$ represents halogen.

**15.** Process for the preparation of compounds of the formula (VI), characterized in that methyl compounds of the general formula (XIII)

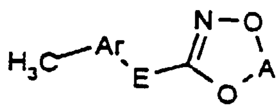

(XIII)

in which

A, Ar and E have the meaning given in Claim 14

are reacted with a halogenating agent at temperatures between 0°C and 150°C, if appropriate in the presence of a catalyst and if appropriate in the presence of a diluent such as, for example, tetrachloromethane.

**16.** Compounds of the formula (XIII)

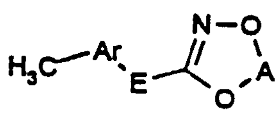

(XIII)

in which A, Ar and E have the meanings given in Claim 1.

**17.** Process for the preparation of compounds of the formula (XIII), characterized in that esters of the general formula (XIV)

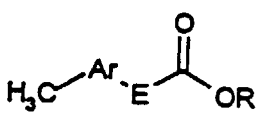

(XIV)

in which

Ar, E and R have the meaning given in Claim 16

are reacted with hydroxylamine or hydroxylamine hydrochloride, if appropriate in the presence of an acid acceptor and if appropriate in the presence of a diluent, and subsequently with a disubstituted alkane of the general formula (III) - as given in Claim 7 -, if appropriate in the presence of an acid acceptor, at temperatures between 0°C and 150°C in analogy to process (a) according to the invention, of Claim 7.

**18.** Compounds of the formula (VIII)

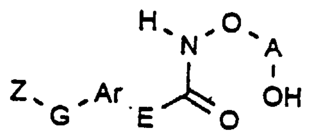

(VIII)

in which A, Ar, E, G and Z have the meanings given in Claim 1.

**19.** Process for the preparation of compounds of the formula (VIII), characterized in that carboxylic acid derivatives of the general formula (XV)

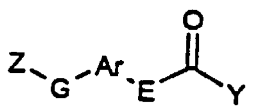

(XV)

in which

| | |
|---|---|
| Ar, E, G and Z | have the meaning given in Claim 18 and |
| Y | represents halogen, hydroxyl or alkoxy |

are reacted with hydroxylamines of the general formula (XVI)

$$H_2N\text{-}O\text{-}A\text{-}OH \qquad (XVI)$$

in which

A has the abovementioned meaning

at temperatures between 0°C and 150°C, if appropriate in the presence of an acid acceptor and if appropriate in the presence of a diluent.

## Revendications

**1.** Azadioxacycloalcènes de formule générale (I)

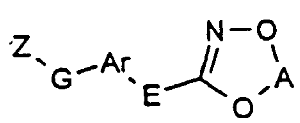

(I)

dans laquelle

A représente un groupe alcanediyle(alkylène) éventuellement substitué,
Ar représente un groupe arylène ou un groupe hétéroarylène chacun éventuellement substitué,
E représente un groupement 1-alcène-1,1-diyle, qui porte en position 2 un reste $R^1$, ou un groupement 2-aza-1-alcène-1,1-diyle, qui porte en position 2 un reste $R^2$, ou un groupement 3-oxa- ou 3-thia-1-propène-1,2-diyle, qui porte en position 1 un reste $R^1$, ou un groupement 3-aza-1-propène-2,3-diyle, qui porte en position 3 un reste R et en position 1 un reste $R^1$, ou un groupement 1-aza-1-propène-2,3-diyle, qui porte en position

1 un reste $R^2$, ou un groupement 3-oxa- ou 3-thia-1-aza-propène-2,3-diyle, qui porte en position 1 un reste $R^2$, ou un groupement 1,3-diaza-1-propène-2,3-diyle, qui porte en position 3 un reste R et en position 1 un reste $R^2$, ou un groupement imino ("azaméthylène", N-$R^3$) éventuellement substitué,

R est un groupe alkyle,
$R^1$ représente de l'hydrogène, un halogène, un groupe cyano ou un groupe alkyle, alkoxy, alkylthio, alkylamino ou dialkylamino, chacun éventuellement substitué,
$R^2$ est de l'hydrogène, un groupe amino, cyano, ou un groupe alkyle, alkoxy, alkylamino ou dialkylamino, chacun éventuellement substitué, et
$R^3$ est de l'hydrogène, un groupe cyano, ou un groupe alkyle, alcényle, alcynyle, cycloalkyle ou cycloalkylalkyle, chacun éventuellement substitué,

G désigne une liaison simple, un atome d'oxygène, un groupe alcanediyle, alcènediyle, oxa-alcènediyle, alcynediyle, chacun éventuellement substitué par un radical halogéno, hydroxy, alkyle, halogénalkyle ou cycloalkyle, ou l'un des groupements suivants :
-Q-CQ-, -CQ-Q-, $-CH_2$-Q-; -Q-$CH_2$-, -CQ-Q-$CH_2$-, $CH_2$-Q-CQ-, -Q-CQ-$CH_2$-, -Q-CQ-Q-$CH_2$-, -N=N-, $-S(O)_n-$, $-CH_2-S(O)_n-$, -CQ-, $-S(O)_n-CH_2-$, -C($R^4$)=N-O-, -C($R^4$)=N-O-$CH_2$-, -N($R^5$)-, -CQ-N($R^5$)-, -N($R^5$)-CQ-, -Q-CQ-N($R^5$)-, -N=C($R^4$)-Q-$CH_2$-, $-CH_2$-O-N=C($R^4$)-, -N($R^5$)-CQ-Q-, -CQ-N($R^5$)-CQ-Q-, -N($R^5$)-CQ-Q-$CH_2$-,-CQ-$CH_2$- ou -N=N-C($R^4$)=N-O-,
où

n représente les nombres 0, 1 ou 2,
Q est de l'oxygène ou du soufre,
$R^4$ est de l'hydrogène, un groupe cyano, ou bien un groupe alkyle, alkoxy, alkylthio, alkylamino, dialkylamino ou cycloalkyle, chacun éventuellement substitué, et
$R^5$ est de l'hydrogène, un groupe hydroxy, cyano, ou bien un groupe alkyle, alkoxy ou cycloalkyle, chacun éventuellement substitué, et

Z est un groupe alkyle, alcényle, alcynyle, cycloalkyle, aryle ou hétérocyclyle, chacun éventuellement substitué.

**2.** Composés de formule (I) suivant la revendication 1, dans laquelle

A représente un groupe alcanediyle ayant 1 à 3 atomes de carbone, éventuellement substitué par un halogène ou par un radical alkyle ou halogénalkyle ayant chacun 1 à 4 atomes de carbone,
Ar est un groupe phénylène ou naphtylène dont chacun est éventuellement substitué ou un groupe hétéroarylène à noyau de 5 ou 6 chaînons dont au moins l'un est de l'oxygène, du soufre ou de l'azote et, le cas échéant, un ou deux autres représentent de l'azote, les substituants possibles étant choisis de préférence parmi les substituants énumérés ci-après :

halogène, cyano, nitro, amino, hydroxy, formyle, carboxy, carbamoyle, thiocarbamoyle, groupe alkyle, alkoxy, alkylthio, alkylsulfinyle ou alkylsulfonyle dont chacun est linéaire ou ramifié et contient 1 à 6 atomes de carbone, groupe alcényle, alcényloxy ou alcynyloxy dont chacun est linéaire ou ramifié et contient 2 à 6 atomes de carbone,
groupe halogénalkyle, halogénalkoxy, halogénalkylthio, halogénalkylsulfinyle ou halogénalkylsulfonyle dont chacun est linéaire ou ramifié et contient 1 à 6 atomes de carbone et 1 à 13 atomes d'halogènes identiques ou différents, groupe halogénalcényle ou halogénalcényloxy dont chacun est linéaire ou ramifié et contient 2 à 6 atomes de carbone et 1 à 13 atomes d'halogènes identiques ou différents, groupe alkylamino, dialkylamino, alkylcarbonyle, alkylcarbonyloxy, alkoxycarbonyle, alkylsulfonyloxy, hydroximinoalkyle ou alkoximinoalkyle dont chacun est linéaire ou ramifié et contient 1 à 6 atomes de carbone dans les parties alkyle individuelles, groupe alkylène ou dioxyalkylène dont chacun est divalent, contient 1 à 6 atomes de carbone et porte le cas échéant un ou plusieurs substituants, identiques ou différents, halogène et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents,

E est l'un des groupements suivants :

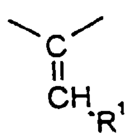 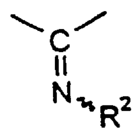 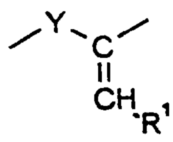 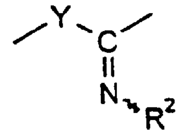 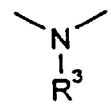

dans lesquels

- Y est de l'oxygène, du soufre, un groupe méthylène ($CH_2$) ou alkylimino(N-R),
- R est un groupe alkyle ayant 1 à 6 atomes de carbone,
- $R^1$ représente de l'hydrogène, un halogène, un groupe cyano, ou bien un groupe alkyle, alkoxy, alkylthio, alkylamino ou dialkylamino avec dans chaque cas 1 à 6 atomes de carbone dans les restes alkyle, et chacun étant éventuellement substitué par un radical halogéno, cyano ou alkoxy en $C_1$ à $C_4$,
- $R^2$ est de l'hydrogène, un groupe amino, cyano, ou bien un groupe alkyle, alkoxy, alkylamino ou dialkylamino ayant dans chaque cas 1 à 6 atomes de carbone dans les restes alkyle et chacun étant éventuellement substitué par un radical halogéno, cyano ou alkoxy en $C_1$ à $C_4$, et
- $R^3$ est de l'hydrogène, un groupe cyano, ou bien un groupe alkyle, alcényle ou alcynyle ayant chacun jusqu'à 6 atomes de carbone et dont chacun est éventuellement substitué par un radical halogéno, cyano ou alkoxy en $C_1$ à $C_4$, ou un groupe cycloalkyle ou cycloalkylalkyle ayant 3 à 6 atomes de carbone dans les parties cycloalkyle et le cas échéant 1 à 4 atomes de carbone dans la partie alkyle et chacun étant éventuellement substitué par un radical halogéno, cyano, carboxy, alkyle en $C_1$ à $C_4$ ou (alkoxy en $C_1$ à $C_4$)-carbonyle,

G désigne une liaison simple, de l'oxygène, un groupe alcanediyle, alcènediyle, oxa-alcènediyle, alcynediyle ayant dans chaque cas jusqu'à 4 atomes de carbone et dont chacun est éventuellement substitué par un radical halogéno, hydroxy, alkyle en $C_1$ à $C_4$, halogénalkyle en $C_1$ à $C_4$ ou cycloalkyle en $C_3$ à $C_6$, ou l'un des groupements suivants :
-Q-CQ-, -CQ-Q-, $-CH_2$-Q-; -Q-$CH_2$-, -CQ-Q-$CH_2$-, $CH_2$-Q-CQ-, -Q-CQ-$CH_2$-, -Q-CQ-Q-$CH_2$-, -N=N-, $-S(O)_n-$, $-CH_2-S(O)_n-$, -CQ-, $-S(O)_n-CH_2-$, -C($R^4$)=N-O-, -C($R^4$)=N-O-$CH_2$-, -N($R^5$)-, -CQ-N($R^5$)-, -N($R^5$)-CQ-, -Q-CQ-N($R^5$)-, -N=C($R^4$)-Q-$CH_2$-, $-CH_2$-O-N=C($R^4$)-, -N($R^5$)-CQ-Q-, -CQ-N($R^5$)-CQ-Q-, -N($R^5$)-CQ-Q-$CH_2$-, -CQ-$CH_2$- ou -N=N-C($R^4$)=N-O-,
dans lesquels

- n représente les nombres 0, 1 ou 2,
- Q est de l'oxygène ou du soufre,
- $R^4$ est de l'hydrogène, un groupe cyano, un groupe alkyle, alkoxy, alkylthio, alkylamino ou dialkylamino ayant dans chaque cas 1 à 6 atomes de carbone dans les groupes alkyle et chacun étant éventuellement substitué par un radical halogéno, cyano, alkoxy en $C_1$ à $C_4$, ou bien un groupe cycloalkyle de 3 à 6 atomes de carbone éventuellement substitué par un radical halogéno, cyano, carboxy, alkyle en $C_1$ à $C_4$ ou (alkoxy en $C_1$ à $C_4$)-carbonyle, et
- $R^5$ représente de l'hydrogène, un groupe hydroxy, cyano, ou bien un groupe alkyle ayant 1 à 6 atomes de carbone éventuellement substitué par un radical halogéno, cyano ou alkoxy en $C_1$ à $C_4$, ou bien un groupe cycloalkyle de 3 à 6 atomes de carbone éventuellement substitué par un radical halogéno, cyano, carboxy, alkyle en $C_1$ à $C_4$, ou (alkoxy en $C_1$ à $C_4$)-carbonyle, et

Z est un groupe alkyle ayant 1 à 8 atomes de carbone éventuellement substitué par un halogène, un radical cyano, hydroxy, amino, ou des radicaux alkoxy en $C_1$ à $C_4$, alkylthio en $C_1$ à $C_4$, alkylsulfinyle en $C_1$ à $C_4$ ou alkylsulfonyle en $C_1$ à $C_4$ (qui sont substitués chacun le cas échéant par un halogène), un groupe alcényle ou alcynyle ayant chacun jusqu'à 8 atomes de carbone et dont chacun est éventuellement substitué par un halogène, un groupe cycloalkyle de 3 à 6 atomes de carbone substitué le cas échéant par un radical halogéno, cyano, carboxy, phényle (qui est substitué le cas échéant par un radical halogéno, cyano, alkyle en $C_1$ à $C_4$, halogénalkyle en $C_1$ à $C_4$, alkoxy en $C_1$ à $C_4$ ou halogénalkoxy en $C_1$ à $C_4$), alkyle en $C_1$ à $C_4$ ou (alkoxy en $C_1$ à $C_4$)-carbonyle, un groupe phényle, naphtyle ou hétérocyclyle (éventuellement condensé au benzène) à 5 ou 6 chaînons dont l'un au moins est de l'oxygène, du soufre ou de l'azote et, le cas échéant, un ou deux autres sont de l'azote, chacun d'eux étant éventuellement substitué, les substituants possibles étant de préférence choisis parmi ceux qui sont énumérés ci-après :

oxygène (en remplacement de deux atomes géminés d'hydrogène), halogène, cyano, nitro, amino, hydroxy, formyle, carboxy, carbamoyle, thiocarbamoyle, groupe alkyle, alkoxy, alkylthio, alkylsulfinyle ou alkylsulfonyle dont chacun est linéaire ou ramifié et contient 1 à 6 atomes de carbone, groupe alcényle ou alcényloxy dont chacun est linéaire ou ramifié et contient 2 à 6 atomes de carbone, groupe halogénalkyle, halogénalkoxy, halogénalkylthio, halogénalkylsulfinyle ou halogénalkylsulfonyle dont chacun est linéaire ou ramifié et contient 1 à 6 atomes de carbone et 1 à 13 atomes d'halogènes identiques ou différents, groupe halogénalcényle ou halogénalcényloxy dont chacun est linéaire ou ramifié et contient 2 à 6 atomes de carbone et 1 à 13 atomes d'halogènes identiques ou différents, groupe alkylamino, dialkylamino, alkylcarbonyle, alkylcarbonyloxy, alkoxycarbonyle, alkylsulfonyloxy, hydroximinoalkyle ou alkoximinoalkyle dont chacun est linéaire ou ramifié et contient 1 à 6 atomes de carbone dans les parties alkyle individuelles, groupe alkylène ou dioxyalkylène dont chacun est divalent, contient 1 à 6 atomes de carbone et est substitué le cas échéant une ou plusieurs fois identiques ou différentes par un halogène et/ou un radical alkyle linéaire ou ramifié ayant 1 à 4 atomes de carbone et/ou un radical halogénalkyle linéaire ou ramifié ayant 1 à 4 atomes de carbone et 1 à 9 atomes d'halogènes identiques ou différents, groupe cycloalkyle ayant 3 à 6 atomes de carbone, groupe hétérocyclyle ou hétérocyclylméthyle ayant chacun 3 à 7 chaînons, dont 1 à 3 dans chaque cas sont des hétéroatomes identiques ou différents - notamment azote, oxygène et/ou soufre -, ainsi que groupe phényle, phénoxy, benzyle, benzyloxy, phényléthyle ou phényléthyloxy, chacun étant éventuellement substitué dans la partie phényle une ou plusieurs fois identiques ou différentes par un radical halogéno, cyano, nitro, carboxy, carbamoyle et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents et/ou alkoxy linéaire ou ramifié ayant 1 à 4 atomes de carbone et/ou halogénalkoxy linéaire ou ramifié ayant 1 à 4 atomes de carbone et 1 à 9 atomes d'halogènes identiques ou différents et/ou alkylcarbonyle ou alkoxycarbonyle ayant chacun jusqu'à 5 atomes de carbone.

**3.** Composés de formule (I) suivant la revendication 1, dans laquelle

A représente un groupe méthylène ou diméthylène (éthane-1,2-diyle) dont chacun est éventuellement substitué par du fluor, du chlore, un radical méthyle, éthyle ou trifluorométhyle,

Ar représente un groupe ortho-, méta- ou paraphénylène, furannediyle, thiophènediyle, pyrrolediyle, pyrazolediyle, triazolediyle, oxazolediyle, isoxazolediyle, thiazolediyle, isothiazolediyle, oxadiazolediyle, thiadiazolediyle, pyridinediyle, pyrimidinediyle, pyridazinediyle, pyrazinediyle, 1,3,4-triazinediyle ou 1,2,3-triazinediyle dont chacun est éventuellement substitué, les substituants possibles étant choisis en particulier parmi ceux qui sont énumérés ci-après :
fluor, chlore, cyano, méthyle, éthyle, trifluorométhyle, méthoxy, éthoxy, méthylthio, méthylsulfinyle ou méthylsulfonyle,

E représente l'un des groupements ci-après :

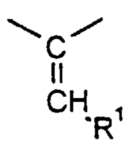 . 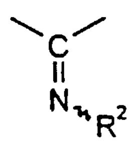 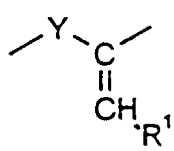 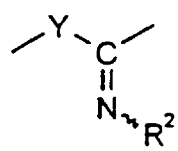 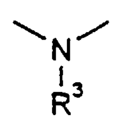

dans lesquels

Y est de l'oxygène, du soufre, un groupe méthylène ($CH_2$) ou alkylimino(N-R),

R est un groupe méthyle, éthyle, n-propyle, iso-propyle, n-butyle, isobutyle ou sec.-butyle,

$R^1$ est de l'hydrogène, du fluor, du chlore, du brome, un groupe cyano, ou bien un groupe méthyle, éthyle, propyle, méthoxy, éthoxy, méthylthio, éthylthio, méthylamino, éthylamino ou diméthylamino, dont chacun est substitué le cas échéant par du fluor, du chlore, un radical cyano, méthoxy ou éthoxy,

$R^2$ représente de l'hydrogène, un groupe amino, cyano, ou bien un groupe méthyle, éthyle, méthoxy, éthoxy, méthylamino, éthylamino ou diméthylamino dont chacun est substitué le cas échéant par du fluor, du chlore, un radical cyano, méthoxy ou éthoxy, et

$R^3$ est de l'hydrogène, un groupe cyano, ou bien un groupe méthyle, éthyle, n-propyle, iso-propyle, n-butyle, isobutyle ou sec.-butyle dont chacun est substitué le cas échéant par du fluor, un radical cyano, méthoxy ou éthoxy, un groupe allyle ou un groupe propargyle, ou bien un groupe cyclopropyle, cyclobutyle, cyclopentyle, cyclohexyle, cyclopropylméthyle, cyclobutylméthyle, cyclopentylméthyle ou cyclohexylméthyle dont chacun est substitué le cas échéant par du fluor, du chlore, un radical cyano, carboxy, méthyle, éthyle, n-propyle, isopropyle, méthoxycarbonyle ou éthoxycarbonyle,

G représente une liaison simple, de l'oxygène, un groupe méthylène, diméthylène (éthane-1,2-diyle), éthène-1,2-diyle, éthyne-1,2-diyle dont chacun est substitué le cas échéant par du fluor, du chlore, un radical hydroxy, méthyle, éthyle, n-propyle, isopropyle, trifluorométhyle, cyclopropyle, cyclobutyle, cyclopentyle ou cyclohexyle, ou l'un des groupements indiqués ci-après :
-Q-CQ-, -CQ-Q-, $-CH_2-Q-$; $-Q-CH_2-$, $-CQ-Q-CH_2-$, $CH_2-Q-CQ-$, $-Q-CQ-CH_2-$, $-Q-CQ-Q-CH_2-$, -N=N-, $S(O)_n$, $-CH_2-S(O)_n-$, -CQ-, $-S(O)_n-CH_2-$, $-C(R^4)=N-O-$, $-C(R^4)=N-O-CH_2-$, $-N(R^5)-$, $-CQ-N(R^5)-$, $-N(R^5)-CQ-$, $-Q-CQ-N(R^5)-$, $-N=C(R^4)-Q-CH_2-$, $-CH_2-O-N=C(R^4)-$, $-N(R^5)-CQ-Q-$, $-CQ-N(R^5)-CQ-Q-$, $-N(R^5)-CQ-Q-CH_2-$, $-CQ-CH_2-$ ou $-N=N-C(R^4)=N-O-$,
où

n représente les nombres 0, 1 ou 2,
Q est de l'oxygène ou du soufre,
$R^4$ est de l'hydrogène, un groupe cyano, un groupe méthyle, éthyle, n-propyle, iso-propyle, n-butyle, isobutyle, sec.-butyle, méthoxy, éthoxy, propoxy, butoxy, méthylthio, éthylthio, propylthio, butylthio, méthylamino, éthylamino, propylamino, diméthylamino ou diéthylamino dont chacun est substitué le cas échéant par du fluor, du chlore, un radical cyano, méthoxy ou éthoxy, ou un groupe cyclopropyle, cyclobutyle, cyclopentyle ou cyclohexyle dont chacun est substitué le cas échéant par du fluor, du chlore, un radical cyano, carboxy, méthyle, éthyle, n-propyle, isopropyle, méthoxycarbonyle ou éthoxycarbonyle, et
$R^5$ est de l'hydrogène, un groupe hydroxy, cyano, ou bien un groupe méthyle, éthyle, n-propyle, isopropyle, n-butyle, isobutyle, sec.-butyle ou tertio-butyle dont chacun est substitué le cas échéant par du fluor, du chlore, un radical cyano, méthoxy ou éthoxy, ou bien un groupe cyclopropyle, cyclobutyle, cyclopentyle ou cyclohexyle dont chacun est substitué le cas échéant par du fluor, du chlore, un radical cyano, carboxy, méthyle, éthyle, n-propyle, isopropyle, méthoxycarbonyle ou éthoxycarbonyle, et

Z représente un groupe méthyle, éthyle, n-propyle, isopropyle, n-butyle, isobutyle, sec.-butyle ou tertio-butyle substitué le cas échéant par du fluor, du chlore, du brome, un radical cyano, hydroxy, amino, des radicaux méthoxy, éthoxy, méthylthio, éthylthio, méthylsulfinyle, éthylsulfinyle, méthylsulfonyle, éthylsulfonyle (dont chacun est substitué le cas échéant par du fluor et/ou du chlore), un groupe allyle, crotonyle, 1-méthylallyle, propargyle ou 1-méthylpropargyle, dont chacun est substitué le cas échéant par du fluor, du chlore ou du brome, un groupe cyclopropyle, cyclobutyle, cyclopentyle ou cyclohexyle substitué le cas échéant par du fluor, du chlore, du brome, un radical cyano, carboxy, phényle (qui est substitué le cas échéant par du fluor, du chlore, du brome, un radical cyano, méthyle, éthyle, n-propyle, iso-propyle, n-butyle, isobutyle, sec.-butyle, tertio-butyle, trifluorométhyle, méthoxy, éthoxy, n-propoxy, isopropoxy, difluorométhoxy ou trifluorométhoxy), méthyle, éthyle, n-propyle, isopropyle, méthoxycarbonyle ou éthoxycarbonyle, un groupe phényle, naphtyle, furyle, tétrahydrofuryle, benzofuryle, tétrahydropyrannyle, thiényle, benzothiényle, pyrrolyle, dihydropyrrolyle, tétrahydropyrrolyle, benzopyrrolyle, benzodihydropyrrolyle, oxazolyle, benzoxazolyle, isoxazolyle, thiazolyle, benzothiazolyle, isothiazolyle, imidazolyle, benzimidazolyle, oxadiazolyle, thiadiazolyle, pyridinyle, pyrimidinyle, pyridazinyle, pyrazinyle, 1,2,3-triazinyle, 1,2,4-triazinyle ou 1,3,5-triazinyle dont chacun est substitué le cas échéant, les substituants possibles étant choisis de préférence parmi ceux qui sont énumérés ci-après : oxygène (en remplacement de deux atomes géminés d'hydrogène) fluor, chlore, brome, cyano, nitro, amino, hydroxy, formyle, carboxy, carbamoyle, thiocarbamoyle, méthyle, éthyle, n-propyle, iso-propyle, n-butyle, isobutyle, sec.-butyle, tertio-butyle, méthoxy, éthoxy, n-propoxy, iso-propoxy, méthylthio, éthylthio, n-propylthio, iso-propylthio, méthylsulfinyle, éthylsulfinyle, méthylsulfonyle, éthylsulfonyle, trifluorométhyle, difluorométhoxy, trifluorométhoxy, difluorométhylthio, trifluorométhylthio, trifluorométhylsulfinyle, trifluorométhylsulfonyle, méthylamino, éthylamino, n-propylamino, iso-propylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximinoéthyle, méthoximinométhyle, éthoximinométhyle, méthoximinoéthyle ou éthoximinoéthyle ; groupe triméthylène (propane-1,3-diyle), méthylènedioxy ou éthylènedioxy, cyclopropyle, cyclobutyle, cyclopentyle ou cyclohexyle dont chacun porte le cas échéant un ou plusieurs substituants identiques ou différents, fluoro, chloro, méthyle, éthyle, n-propyle ou isopropyle, ainsi que groupe phényle, phénoxy, benzyle ou benzyloxy dont chacun est substitué le cas échéant dans la partie phényle, une ou plusieurs fois identiques ou différentes, par du fluor, du chlore, du brome, un radical cyano, nitro, carboxy, carbamoyle, méthyle, éthyle, n-propyle, isopropyle, n-butyle, isobutyle, sec.-butyle, tertio-butyle, trifluorométhyle, méthoxy, éthoxy, n-propoxy, isopropoxy, difluorométhoxy, trifluorométhoxy, acétyle, méthoxycarbonyle ou éthoxycarbonyle.

**4.** Composés de formule (I) suivant la revendication 1, dans laquelle

A est un groupe diméthylène (éthane-1,2-diyle),
Ar est un groupe orthophénylène, pyridine-2,3-diyle ou thiophène-2,3-diyle,
E est l'un des groupements suivants :

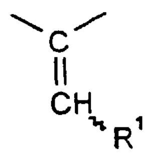

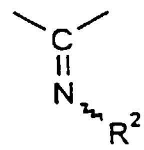

dans lesquels

$R^1$ et $R^2$ représentent chacun un groupe méthoxy,

G est de l'oxygène, un groupe méthylène ou l'un des groupements suivants : $-CH_2-O-$, $-O-CH_2-$, $-S(O)_n-$, $-CH_2-S(O)_n-$, $-S(O)_n-CH_2-$, $-C(R^4)=N-O-$, $-O-N=C(R^4)-$, $-C(R^4)=N-O-CH_2-$, $-N(R^5)-$ ou $-CH_2-O-N=C(R^4)-$
dans lesquels

n représente les nombres 0, 1 ou 2,
$R^4$ est de l'hydrogène, un groupe méthyle ou éthyle et
$R^5$ est de l'hydrogène, un groupe méthyle ou éthyle, et

Z représente un groupe phényle, pyridinyle, pyrimidinyle, pyridazinyle, pyrazinyle, 1,2,3-triazinyle, 1,2,4-triazinyle ou 1,3,5-triazinyle dont chacun est éventuellement substitué, les substituants possibles étant choisis de préférence parmi ceux qui sont énumérés ci-après : fluor, chlore, brome, cyano, méthyle, éthyle, n-propyle, isopropyle, n-butyle, isobutyle, sec.-butyle, ou tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, difluorométhoxy, trifluorométhoxy, difluoro-méthylthio, trifluorométhylthio, trifluorométhylsulfinyle, trifluorométhylsulfonyle, méthoxycarbonyle, éthoxycarbonyle, méthoximinométhyle, éthoximinométhyle, méthoximinoéthyle, éthoximinoéthyle, méthylènedioxy ou éthylènedioxy dont chacun porte le cas échéant un ou plusieurs substituants fluoro, chloro, méthyle ou éthyle identiques ou différents, ainsi que phényle, phénoxy, benzyle ou benzyloxy dont chacun porte le cas échéant dans la partie phényle un ou plusieurs substituants, identiques ou différents, fluoro, chloro, bromo, cyano, méthyle, éthyle, n-propyle, isopropyle, n-butyle, isobutyle, sec.-butyle, tertio-butyle, trifluorométhyle, méthoxy, éthoxy, n-propoxy, isopropoxy, difluorométhoxy ou trifluorométhoxy.

**5.** Compositions fongicides, caractérisées par une teneur en au moins un composé de formule (I) suivant la revendication 1.

**6.** Procédé pour combattre des champignons indésirables, caractérisé en ce qu'on fait agir des composés de formule (I) suivant la revendication 1 sur les champignons et/ou sur leur milieu.

**7.** Procédé de production d'azadioxacycloalcènes de formule générale (I)

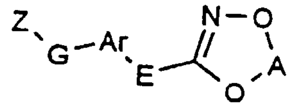

**(I)**

dans laquelle

A représente un groupe alcanediyle(alkylène) éventuellement substitué,
Ar représente un groupe arylène ou un groupe hétéroarylène chacun éventuellement substitué,
E représente un groupe 1-alcène-1,1-diyle, qui porte en position 2 un reste $R^1$, ou un groupement 2-aza-1-alcène-1,1-diyle, qui porte en position 2 un reste $R^2$, ou un groupement 3-oxa- ou 3-thia-1-propène-2,3-diyle, qui porte en position 1 un reste $R^1$, ou un groupement 3-aza-1-propène-2,3-diyle, qui porte en position 3 un reste R et en position 1 un reste $R^1$, ou un groupement 1-aza-1-propène-2,3-diyle, qui porte en position 1 un reste $R^2$, ou un groupement 3-oxa- ou 3-thia-1-aza-propène-2,3-diyle, qui porte en position 1 un reste $R^2$, ou un groupement 1,3-diaza-1-propène-2,3-diyle, qui porte en position 3 un reste R et en position 1 un reste $R^2$, ou un groupement imino ("azaméthylène", N-$R^3$, éventuellement substitué,

R est un groupe alkyle,
$R^1$ représente de l'hydrogène, un halogène, un groupe cyano ou un groupe alkyle, alkoxy, alkylthio, alkylamino ou dialkylamino, chacun éventuellement substitué,
$R^2$ est de l'hydrogène, un groupe amino, cyano, ou un groupe alkyle, alkoxy, alkylamino ou dialkylamino, chacun éventuellement substitué, et
$R^3$ est de l'hydrogène, un groupe cyano, ou un groupe alkyle, alcényle, alcynyle, cycloalkyle ou cycloalkylalkyle, chacun éventuellement substitué,

G désigne une liaison simple, un atome d'oxygène, un groupe alcanediyle, alcènediyle, oxa-alcènediyle, alcynediyle, chacun éventuellement substitué par un radical halogéno, hydroxy, alkyle, halogénalkyle ou cycloalkyle, ou l'un des groupements suivants :
$-Q-CQ-$, $-CQ-Q-$,$-CH_2-Q-$; $-Q-CH_2-$, $-CQ-Q-CH_2-$, $CH_2-Q-CQ-$, $-Q-CQ-CH_2-$, $-Q-CQ-Q-CH_2-$, $-N=N-$, $-S(O)_n-$, $-CH_2-S(O)_n-$, $-CQ-$, $-S(O)_n-CH_2-$, $-C(R^4)=N-O-$, $-C(R^4)=N-O-CH_2-$, $-N(R^5)-$, $-CQ-N(R^5)-$, $-N(R^5)-CQ-$, $-Q-CQ-N(R^5)-$, $-N=C(R^4)-Q-CH_2-$, $-CH_2-O-N=C(R^4)-$, $-N(R^5)-CQ-Q$, $-CQ-N(R^5)-CQ-Q$, $-N(R^5)-CQ-Q-CH_2-$, $-CQ-CH_2-$ ou $-N=N-C(R^4)=N-O-$,
où

n représente les nombres 0, 1 ou 2,
Q est de l'oxygène ou du soufre,
$R^4$ est de l'hydrogène, un groupe cyano, ou bien un groupe alkyle, alkoxy, alkylthio, alkylamino, dialkylamino ou cycloalkyle, chacun éventuellement substitué, et
$R^5$ est de l'hydrogène, un groupe hydroxy, cyano, ou bien un groupe alkyle, alkoxy ou cycloalkyle, chacun éventuellement substitué, et

Z est un groupe alkyle, alcényle, alcynyle, cycloalkyle, aryle ou hétérocyclyle, chacun éventuellement substitué,

caractérisé en ce que

(a) dans une première étape, on fait réagir des dérivés d'acides carboxyliques de formule générale (II)

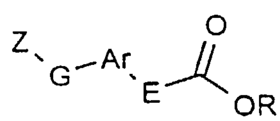

(II)

dans laquelle

Ar, E, G et Z ont la définition indiquée ci-dessus et
R est un groupe alkyle,

avec l'hydroxylamine ou avec un halogénhydrate d'hydroxylamine, le cas échéant en présence d'un accepteur d'acide et en la présence éventuelle d'un diluant et dans une seconde étape, on fait réagir le produit de la première étape in situ, c'est-à-dire sans isolement intermédiaire, avec des alcanes disubstitués de formule générale (III)

$$X\text{-}A\text{-}X \qquad (III)$$

dans laquelle

A  a la définition indiquée ci-dessus et
X  représente un halogène, un groupe alkylsulfonyloxy ou arylsulfonyloxy,

le cas échéant en présence d'un accepteur d'acide et en la présence éventuelle d'un diluant, ou bien
(b) au cas où, dans la formule (I), G représente de l'oxygène et le groupement $-CH_2-O-$ et A, Ar, E et Z ont la définition indiquée dans la revendication 1,
on fait réagir des composés hydroxyaryliques de formule générale (IV)

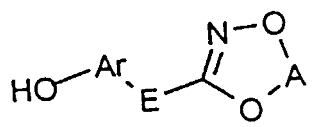
(IV)

dans laquelle
A, Ar et E ont la définition indiquée ci-dessus, avec des composés de formule générale (V)

$$Z\text{-}(CH_2)_m\text{-}X \qquad (V)$$

dans laquelle

X et Z  ont la définition indiquée ci-dessus et
m  représente le nombre 0 ou 1,

le cas échéant en présence d'un accepteur d'acide et en la présence éventuelle d'un diluant et on conduit ensuite éventuellement des réactions de substitution selon des opérations classiques sur le groupement Z, ou bien
(c) au cas où, dans la formule (I), G représente le groupement $-Q-CH_2-$ et A, Ar, E et Z ont la définition indiquée ci-dessus,
on fait réagir des composés halogénométhyliques de formule générale (VI)

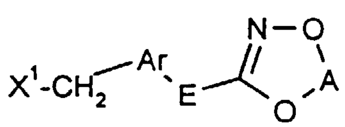
(VI)

dans laquelle

A, Ar et E  ont la définition indiquée ci-dessus et
$X^1$  représente un halogène,

avec des composés de formule générale (VII)

$$Z\text{-}Q\text{-}H \qquad (VII)$$

dans laquelle

Q et Z ont la définition indiquée ci-dessus,

le cas échéant en présence d'un accepteur d'acide et en la présence éventuelle d'un diluant, ou bien
(d) on effectue la cyclisation déshydratante d'hydroxyalkoxyamides de formule générale (VIII)

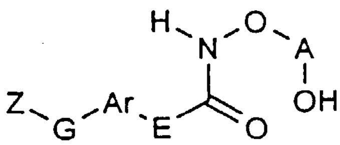

(VIII)

dans laquelle

A, Ar, E, G et Z ont la définition indiquée ci-dessus,

avec un agent déshydratant, le cas échéant en présence d'un diluant.

**8.** Utilisation de composés de formule (I) suivant la revendication 1 comme fongicides.

**9.** Procédé de préparation de compositions fongicides, caractérisé en ce qu'on mélange des composés de formule (I) suivant la revendication 1 avec des diluants et/ou des agents tensio-actifs.

**10.** Composés de formule (IV)

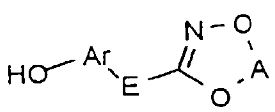

(IV)

dans laquelle A, Ar et E ont les définitions indiquées dans la revendication 1.

**11.** Procédé de production de composés de formule (IV), caractérisé en ce qu'on fait réagir des composés tétrahydropyrannyloxy de formule générale (IX)

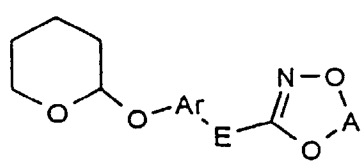

(IX)

dans laquelle
A, Ar et E ont la définition indiquée dans la revendication 10, avec un acide ou un échangeur ionique acide, le cas échéant en présence d'un diluant, à des températures comprises entre 0°C et 100°C.

**12.** Composés de formule (IX)

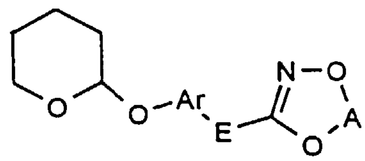

(IX)

dans laquelle A, Ar et E ont les définitions indiquées dans la revendication 1.

**13.** Procédé de production de composés de formule (IX), caractérisé en ce qu'on fait réagir des esters de formule générale (X)

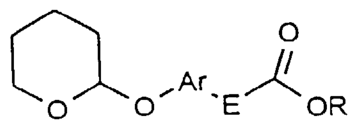

(X)

dans laquelle
Ar, E et R ont la définition indiquée dans la revendication 12,
avec l'hydroxylamine - ou le cas échéant avec son chlorhydrate -, le cas échéant en présence d'un accepteur d'acide et en la présence éventuelle de diluants et on poursuit la réaction in situ du produit intermédiaire ainsi formé, avec des dihalogénalcanes de formule générale (III) - comme indiqué dans la revendication 7 -, le cas échéant en présence d'un accepteur d'acide, à des températures comprises entre 0°C et 100°C.

**14.** Composés de formule (VI)

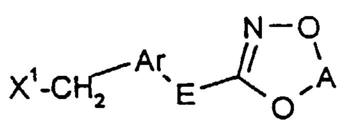

(VI)

dans laquelle
A, Ar et E ont les définitions indiquées dans la revendication 1 et $X^1$ représente un halogène.

**15.** Procédé de production de composés de formule (VI), caractérisé en ce qu'on fait réagir des composés méthyliques de formule générale (XIII)

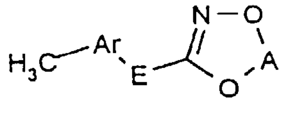

(XIII)

dans laquelle
A, Ar et E ont la définition indiquée dans la revendication 14,
avec un agent d'halogénation, le cas échéant en présence d'un catalyseur et en la présence éventuelle d'un diluant tel que, par exemple, le tétrachlorométhane, à des températures comprises entre 0°C et 150°C.

**16.** Composés de formule (XIII)

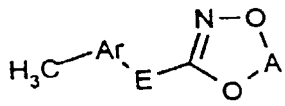 (XIII)

dans laquelle A, Ar et E ont les définitions indiquées dans la revendication 1.

**17.** Procédé de production de composés de formule (XIII), caractérisé en ce qu'on fait réagir des esters de formule générale (XIV)

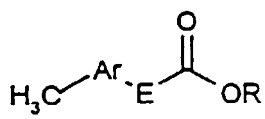 (XIV)

dans laquelle
Ar, E et R ont la définition indiquée dans la revendication 16,
avec l'hydroxylamine ou le chlorhydrate d'hydroxylamine, éventuellement en présence d'un accepteur d'acide et, le cas échéant, en présence d'un diluant, puis avec un alcane disubstitué de formule générale (III) - comme indiqué dans la revendication 7 -, le cas échéant en présence d'un accepteur d'acide, par un mode opératoire analogue au procédé de l'invention (a) suivant la revendication 7, à des températures comprises entre 0°C et 150°C.

**18.** Composés de formule (VIII)

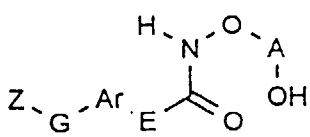 (VIII)

dans laquelle A, Ar, E, G et Z ont les définitions indiquées dans la revendication 1.

**19.** Procédé de production de composés de formule (VIII), caractérisé en ce qu'on fait réagir des dérivés d'acides carboxyliques de formule générale (XV)

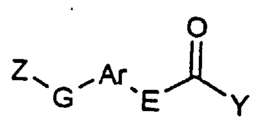 (XV)

dans laquelle
Ar, E, G et Z ont la définition indiquée dans la revendication 18, et

Y représente un halogène, un groupe hydroxy ou alkoxy,

avec des hydroxylamines de formule générale (XVI)

$$H_2N\text{-}O\text{-}A\text{-}OH \qquad (XVI)$$

dans laquelle

A a la définition indiquée ci-dessus,

le cas échéant en présence d'un accepteur d'acide et en la présence éventuelle d'un diluant, à des températures comprises entre 0°C et 150°C.